# EUROPÄISCHE PATENTSCHRIFT

(11) **EP 2 753 310 B1**
(45) Veröffentlichungstag und Bekanntmachung des Hinweises auf die Patenterteilung: **24.03.2021**
(21) Anmeldenummer: 12829553.2
(22) Anmeldetag: 07.09.2012
(51) Int. Cl.: A61K 9/22, A61K 9/28, A61K 9/16, A61K 9/48, A61K 9/20, A61K 9/50

(54) **FORMULIERUNG ZUR KONTROLLIERTEN FREISETZUNG EINES ODER MEHREREN STOFFE IM VERDAUUNGSTRAKT EINES SÄUGETIERS**
FORMULATION FOR THE CONTROLLED RELEASE OF ONE OR SEVERAL SUBSTANCES IN THE DIGESTIVE TRACT OF A MAMMAL
FORMULATION OPTIMALE POUR LA LIBÉRATION D'UN PRINCIPE ACTIF DANS LE GROS INTESTIN

(30) Priorität: 07.09.2011 DE 102011112501; 11.09.2011 DE 102011112761; 19.12.2011 DE 102011056646; 03.04.2012 EP 12163060; 17.04.2012 EP 12164414; 30.04.2012 EP 12166248; 22.05.2012 EP 12168985; 01.06.2012 EP 12170634; 14.08.2012 EP 12180485
(43) Veröffentlichungstag der Anmeldung: 16.07.2014
(73) Patentinhaber: Saur-Brosch, Roland, 69126 Heidelberg (DE)
(72) Erfinder: Saur-Brosch, Roland, 69126 Heidelberg (DE)
(86) Internationale Anmeldenummer: PCT/IB2012/054655
(87) Internationale Veröffentlichungsnummer: WO 2013/035081

(56) Entgegenhaltungen:
- EP-A1- 0 759 303
- EP-A1- 1 607 087
- WO-A2-2006/102446
- RU-C1- 2 130 311
- RU-C2- 2 336 865
- US-A1- 2005 037 077
- US-A1- 2010 209 520

## Beschreibung

Zur Erzielung bestimmter gewünschter Effekte ist es vorteilhaft, einen oder mehrere Stoffe im Dickdarm eines Säugetieres freizusetzen.

Die gewünschten Effekte können beispielsweise die gezielte Verabreichung eines oder mehrerer Stoffe sein, welche eine besondere therapeutische Wirksamkeit aufweisen, wenn sie im Dickdarm freigesetzt werden, zum Beispiel dadurch, dass sie dort gezielt auf die Dickdarmschleimhaut einwirken, auf Bakterien der Dickdarmflora einwirken oder durch besondere Absorptionsfähigkeit der Dickdarmschleimhaut oder durch Umgehung von im Dünndarm vorhandenen Verdauungssäften eine bessere systemische Wirksamkeit entfalten können. Weitere gewünschte Effekte der Freisetzung eines oder mehrerer Stoffe im Dickdarm eines Säugetiers sind dem Fachmann bekannt.

Es ist bekannt, dass der pH-Wert des Darminhaltes, der im Prinzip eine Art wässrige Lösung oder ein wässriges Medium darstellt, nach dem Passieren des Magens ansteigt, und kurz vor dem Erreichen des Dickdarms einen typischen Wert von etwa 7 erreicht. Es wird versucht, diesen Anstieg zu nutzen, um die Freisetzung von Wirkstoffen einzuleiten. Hierzu werden wirkstoffhaltige Kerne mit anionischen Polymeren beschichtet, welche in wässrigen Lösungen bei einem pH Wert über 7 löslich sind (Zeichnung 1 E). Steigt der pH Wert kurz vor Erreichen des Dickdarms über 7, so löst sich die Beschichtung auf und die Formulierung setzt die Wirkstoffe kurz vor dem Eintritt in den Dickdarm frei. Damit die Freisetzung erst im Dickdarm stattfindet, wird teilweise eine sich langsam auflösende Schicht unter der enterischen Schicht aufgebracht, welche die Freisetzung kurze Zeit verzögert, damit die Freisetzung möglichst nicht bereits im Dünndarm beginnt. Da der im Dünndarm maximal erreichte pH Wert einer interindividuellen Variabilität von deutlich über einer pH Stufe unterliegt, wird ein pH Wert von 7 nicht bei allen Individuen überschritten, so dass es teilweise nicht zur beabsichtigten Freisetzung kommt. Senkt man den Löslichkeitsschwellwert des anionischen Polymers, beispielsweise auf pH 6, um auch in diesen Fällen eine Freisetzung zu erreichen, so erfolgt bei Individuen, bei denen kurz vor dem Dickdarm pH 6 überschritten wird, die Freisetzung wunschgemäß, bei Individuen bei denen kurz vor dem Dickdarm pH 7 überschritten wird, die Freisetzung jedoch möglicherweise zu früh, weil bei diesen Individuen pH 6 bereits im Jejunum überschritten werden kann. Solche Formulierungen, welche die Freisetzung abhängig von der Überschreitung eines bestimmten pH Wertes auslösen, sind also abhängig von dem maximal im Dünndarm erreichten pH Wert. Sie funktionieren nur dann sicher, wenn in der Zielgruppe von Individuen, bei denen eine Freisetzung im Dickdarm erwünscht ist, der unter Berücksichtigung der interindividuellen Variabilität maximal im Dünndarm erreichte pH Wert innerhalb gewisser Grenzen liegt. Der Bereich innerhalb dieser Grenzen wird im weiteren Verlauf auch als Arbeitsbereich bezeichnet. Damit es nicht zum Ausbleiben der Freisetzung kommt, muss der Schwellwert des anionischen Polymers kleiner oder gleich der unteren Grenze des gewünschten Arbeitsbereichs der Formulierung sein. Allerdings kann eine Freisetzung bereits dann erfolgen, wenn der pH Wert die untere Grenze des Arbeitsbereichs überschreitet. Da die Anstiegsgeschwindigkeit des pH Wertes von Mitte bis Ende des Dünndarms sehr gering ist, und der Schwellwert des anionischen Polymers nicht bereits in der Mitte des Dünndarms überschritten werden soll, ist der Arbeitsbereich solch einer Technologie sehr schmal, typischerweise etwa 0,5 pH Stufen, was für die meisten Anwendungen nicht ausreichend ist.

Es ist bekannt, dass sich unabhängig von interindividuellen Variabilitäten des intestinalen pH-Wertes der pH-Wert des Darminhaltes nach dem Passieren der Ileozäkalklappe um typisch 1 bis 1,5 Einheiten senkt, und es wird versucht, diese Senkung zu nutzen, um die Freisetzung von Wirkstoffen einzuleiten. Die Patentschrift EP 1607087 offenbart Formulierungen zur dickdarmgezielten Freisetzung.

US20100209520 beschreibt ein dreischichtiges System, bei dem zwei Schichten die Freisetzung kontrollieren. Es wird ein wirkstofffreier Kern zuerst mit einer wirkstoffhaltigen, dort als innere Schicht bezeichnete Schicht beschichtet. Danach wird der beschichtete Kern mit einer dort als intermediär bezeichneten Schicht aus einem Material beschichtet, das unterhalb eines pH Wertes von 6,6 löslich wird. Danach erfolgt eine weitere, als äußere Schicht bezeichnete Beschichtung mit einem Material, das oberhalb eines pH Wertes von 7,0 löslich wird. Der Wirkstoff wird freigesetzt, wenn der pH Wert beim Eintritt in das Zäkum unter 6,6 sinkt, nachdem er zuvor nach dem Austritt aus dem Magen über 7 gestiegen war.

Da die vorliegende Erfindung ähnliche Schichten aufweist, diese aber anders bezeichnet, werden im weiteren Verlauf Schichten ähnlich der Schicht, die in US20100209520 innere Schicht genannt ist, nicht als innere Schichten bezeichnet, sondern als Bestandteile wirkstoffhaltiger Kerne angesehen. Schichten ähnlich der intermediären Schicht werden als innerste Schichten bezeichnet oder als "Caecal Coating" (Zeichnung 2, C), da diese sich nicht vor dem Eintritt ins Zäkum auflösen. Schichten ähnlich der äußeren Schicht von US20100209520 oder ähnlich der Schicht aus anionischen Polymeren, welche zum Auslösen der Freisetzung nach Überschreitung eines bestimmten pH Wertes benutzt werden, werden als innere Schichten oder als "Enteric Coating" (Zeichnung 2, E) bezeichnet. Als äußere oder äußerste Schichten werden nur solche Schichten bezeichnet, die über einer inneren Schicht liegen und vorzugsweise Schutz vor Feuchtigkeit oder neutralem bis basischem Speichel bieten, also sogenannte "Protective Coatings" (Zeichnung 2, P).

Das in US20100209520 offenbarte System hat den Nachteil, dass der pH Wert mindestens über 7 steigen muss, und danach mindestens unter 6,6 abfallen muss, damit die Freisetzung erfolgt.

Somit ist das System ungeeignet, wenn der pH Wert im Dünndarm bei der Zielgruppe einen Wert von 7 nicht sicher übersteigt.

Bei Individuen mit einem niedrigeren maximalen pH Wert im Dünndarm erfolgt keine Freisetzung. Es ist ebenso ungeeignet, wenn der pH Wert beim Eintritt in den Dickdarm nicht unter 6,6 sinkt. Bei Individuen mit einem hohen maximalen pH Wert im Dünndarm und/oder bei Individuen mit einem nicht sehr ausgeprägten Abfall des pH Wertes beim Eintritt in den Dickdarm, erfolgt ebenfalls keine Freisetzung. Der Bereich an pH Werten, in dem ein solches System zuverlässig Wirkstoffe im Dickdarm freisetzen kann (der Arbeitsbereich), wird also nach unten hin durch den Schwellwert der inneren Schicht begrenzt, und nach oben hin durch den Schwellwert der innersten Schicht, erhöht um das Maß, um das der pH Wert beim Eintritt in den Dickdarm abfällt. Die maximal zur Freisetzung erforderliche Verringerung des pH-Wertes beim Eintritt in den Dickdarm berechnet sich folgendermaßen: Maximalwert des bei der Zielgruppe zu erwartenden pH-Wertes im Dünndarm abzüglich des Schwellwertes, unter dem die innerste Schicht löslich wird. Da die Höhe des maximalen pH Wertes im Dünndarm und die Höhe des Abfalls des pH Wertes beim Eintritt in den Dickdarm weder proportional sind, noch anderweitig korrelieren, muss die maximal zur Freisetzung erforderliche Verringerung des pH-Wertes eines Systems für eine sichere Freisetzung geringer sein, als der geringste in der Zielgruppe vorkommende Abfall des pH Wertes beim Erreichen des Dickdarms.

Ist der Abfall in der Zielgruppe nicht sicher höher als 1,4 pH Stufen, setzt das in US20100209520 offenbarte System nur in einem Bereich der maximal im Dünndarm vorkommenden pH Werte von über 7 bis unter 8 den oder die Wirkstoffe sicher im Dickdarm frei. Wird bei Individuen der Zielgruppe ein maximaler pH Wert im Dünndarm von pH 8,5 erreicht, kann ein Abfall des pH Wertes beim Erreichen des Dickdarms von über 1,9 pH Stufen erforderlich sein. Der Schwellwert der innersten Schicht, unterhalb dem diese löslich oder permeabel wird (im weiteren Verlauf auch bestimmter erster pH Wert genannt), darf auch nicht über den Schwellwert der inneren Schicht, oberhalb dem diese löslich oder permeabel wird (im weiteren Verlauf auch bestimmter letzter pH Wert genannt), angehoben werden, da ansonsten bei sehr langsamer Steigung des pH Wertes die innerste Beschichtung gleich nach der inneren Beschichtung löslich werden würde ohne dass ein Abfall des pH-Wertes erforderlich wäre. Auch eine nur leichte Anhebung des Schwellwertes der innersten Schicht kann zu fehlerhafter Freisetzung führen, da der pH Wert im Verlauf des Dünndarms leichte Schwankungen aufweisen kann.

Ebenso darf der Schwellwert der inneren Schicht nicht unter den der innersten Schicht abgesenkt werden. Es ist bei diesem System also nicht möglich, den eingeschränkten Arbeitsbereich zu vergrößern, den zur Freisetzung maximal erforderlichen Abfall des pH Wertes beim Erreichen des Dickdarms zu verringern, oder die Unempfindlichkeit gegenüber Schwankungen des pH Wertes innerhalb des Dünndarms zu vergrößern, ohne jeweils mindestens einen der beiden anderen Parameter zu verschlechtern. Bei einer Zielgruppe von Individuen, bei welcher der geringste vorkommende Abfall des pH Wertes beim Erreichen des Dickdarms nicht größer ist, als der Bereich, innerhalb dem die individuellen Maximalwerte des pH Wertes im Dünndarm liegen, kann mit dieser bekannten Technik keine sichere Freisetzung im Dickdarm erreicht werden. Mit dieser Technik ist es nur dann möglich, Wirkstoffe sicher im Dickdarm freizusetzen, wenn der Abfall des pH-Wertes nach Passage der Ileozäkalklappe größer ist, als die interindividuelle Variabilität des maximalen pH-Wertes im Dünndarm. Die maximal zur Freisetzung erforderliche Verringerung (Abfall) des pH-Wertes innerhalb des Verdauungstraktes ist also größer als die interindividuelle Variabilität des maximal zu erwartenden pH Wertes innerhalb des Verdauungstraktes vor Erreichen der Ileozäkalklappe in der Gruppe von Individuen, für welche der freizusetzende Stoff, bzw. die freizusetzenden Stoffe bestimmt sind, also größer als der erforderliche Arbeitsbereich. Arbeitsbereich (AB), Toleranz gegenüber Schwankungen des pH Wertes im Dünndarm (ST) und erforderlicher Abfall des pH Wertes beim Eintritt in den Dickdarm (EA) sind bei Systemen im Stand der Technik folgendermaßen verknüpft: EA=AB+ST

### Aufgabe:

Um die Sicherheit der Freisetzung im Dickdarm zu verbessern und sowohl das Risiko für eine zu frühe Freisetzung, als auch für eine zu späte oder eine überhaupt nicht erfolgende Freisetzung zu verringern, besteht Bedarf an einer Formulierung zur dickdarmgezielten Freisetzung eines oder mehrerer Wirkstoffe oder eines oder mehrerer wirkstoffhaltiger Kerne, bei welcher die Freisetzung nicht bereits dann ausgelöst wird (auch nicht verzögert), wenn die untere Grenze des Arbeitsbereiches überschritten wird, und bei welcher der erforderliche Abfall des pH Wertes beim Eintritt in den Dickdarm (EA) kleiner ist, als die Summe aus der Breite des Arbeitsbereiches und der erforderlichen Schwankungstoleranz (AB+ST) und vorzugsweise auch kleiner ist, als die Breite des Arbeitsbereiches an sich, wenn ST nicht größer als 20% der Breite des Arbeitsbereiches ist.

### Beschreibung der Erfindung:

Eine in falscher Reihenfolge beschichtete Formulierung, welche zur Freisetzung durch bakterielle Enzyme der Dickdarmflora gedacht war, setzte die Wirkstoffe unbeabsichtigterweise und daher überraschend auch in einem simulierten Zäkum-Medium frei, das zur Kontrolle keine entsprechenden bakteriellen Enzyme beinhaltete.

Untersuchungen ergaben, dass eine Vertauschung der Reihenfolge passiert war, so dass das als protective Coating gedachte Kollicoat Smartseal 30D als erste Schicht statt als letzte Schicht aufgebracht worden war, und dass eine solche Schichtfolge überraschenderweise für eine dickdarmgezielte Verabreichung geeignet ist.

Ursprünglich war beabsichtigt gewesen, das Kollicoat Smartseal 30D als äußerste Schicht aufzubringen, um einen Schutz vor neutralem bis leicht basischem Speichel zu bewirken, als darunter liegende Schicht Eudragit L 100 um einen Schutz gegen die Magensäure zu bilden und als unterste Schicht ein mit Chlorogensäure gepfropftes Chitosan, um einerseits eine Auflösung der Schicht durch bakterielle Enzyme der dickdarmständigen Bakterien zu erreichen und andererseits auch dann eine Freisetzung zu ermöglichen, wenn aufgrund der Zusammensetzung der Dickdarmflora keine oder nicht ausreichend viel entsprechende saccharolytische Enzyme vorhanden sind, aber der pH-Wert im Zäkum durch proteolytische Bakterien höher als üblich ist.

Durch die Vertauschung der Reihenfolge wurde überraschenderweise festgestellt, dass mit einer solchen Schichtfolge auch ohne das Vorhandensein spezieller bakterieller Enzyme, welche das verwendete Polymer abbauen können, eine dickdarmgezielte Verabreichungsform realisiert werden kann.

Durch weitere Untersuchungen konnte überraschenderweise festgestellt werden, dass auch ohne die innerste Schicht eine dickdarmgezielte Verabreichung realisiert werden konnte, bei welcher der Arbeitsbereich größer ist, als bei Anwendung von im Stand der Technik beschriebenen Formulierungen.

Die Erfindung löst die Aufgabe, indem sie eine Formulierung bereitstellt, die eine Beschichtung oder sonstige Umhüllung aufweist, welche oberhalb eines bestimmten letzten pH Wertes löslich oder permeabel ist.

Unter dieser Beschichtung, beziehungsweise von dieser Umhüllung umhüllt, weist die erfindungsgemäße Formulierung eine oder mehrere untereinander liegende beziehungsweise voneinander eingehüllte weitere Beschichtungen oder weitere Umhüllungen auf, welche jeweils oberhalb eines individuellen bestimmten oberen pH Wertes und unterhalb eines individuellen bestimmten unteren pH Wertes löslich oder permeabel sind.

Unterhalb dieser weiteren Schicht oder Schichten, beziehungsweise von dieser oder diesen weiteren Umhüllungen umhüllt, weist sie einen oder mehrere Wirkstoffe oder einen oder mehrere wirkstoffhaltige Kerne auf.

In einer bevorzugten Ausführungsform der Erfindung ist zwischen der oder den weiteren Schichten oder Umhüllungen und dem oder den Wirkstoffen oder wirkstoffhaltigen Kernen eine zusätzliche, innerste Schicht oder Umhüllung angeordnet, welche unterhalb eines bestimmten ersten pH Wertes löslich oder permeabel ist. In einer weiter bevorzugten Ausführungsform weist diese innerste Beschichtung oder auf sonstige Art ausgeführte Umhüllung einen bestimmten zweiten pH Wert auf, über dem sie ebenfalls löslich oder permeabel ist.

Die Erfindung stellt sozusagen eine Formulierung bereitstellt, die eine Art innere Schicht aufweist (Schicht E, Zeichnungen 1 bis 3), optional eine Art innerste Schicht aufweist (Schicht C in Zeichnung 2, Schicht C1 in Zeichnung 3), und einen oder mehrere Wirkstoffe oder einen oder mehrere wirkstoffhaltige Kerne (W in Zeichnungen 1-3), wobei sie zwischen die vorstehend genannte innerste Schicht und die vorstehend genannte innere Schicht oder zwischen den oder die Wirkstoffe oder den oder die wirkstoffhaltigen Kerne und die vorstehend genannte innere Schicht eine oder mehrere weitere Schichten anordnet, wobei diese Schicht oder Schichtenfolge beispielsweise durch Beschichtungs-, als auch Verpressungs-, Emulgierungs- oder Dispersionsschritte realisiert werden kann (Zeichnung 3, Schichten C2 bis Cn). Solche zwischen der inneren und der innersten Schicht oder zwischen der inneren Schicht und dem oder den Wirkstoffen oder dem oder den wirkstoffhaltigen Kernen liegende Schichten werden auch nachfolgend weitere Schichten genannt.

Diese eine oder mehrere weiteren Schichten weisen ähnlich wie die vorgenannte innere oder innerste Schicht ebenfalls pHabhängige Löslichkeits- oder Permeabilitätseigenschaften auf, allerdings dergestalt, dass sie jeweils unter einem bestimmten unteren pH-Wert löslich oder permeabel sind oder werden und zusätzlich oberhalb eines bestimmten oberen pH-Wertes löslich oder permeabel sind oder werden. In dem Bereich zwischen deren individuellen bestimmten unteren und bestimmten oberen pH-Wert ist diese Schicht, bzw. sind diese Schichten jeweils nicht löslich oder permeabel, zumindest nicht innerhalb eines Zeitraumes, dem sie im Gastrointestinaltrakt vor der Ileozäkalklappe dem Inhalt desselben ausgesetzt sind.

Damit die einzelnen weiteren Schichten bei monoton steigenden pH-Werten nur nacheinander, und dies auch nur bei immer weiter steigenden pH-Werten, löslich oder permeabel werden, ist der bestimmte obere pH-Wert jeder zuvor aufgebrachten, bzw. jeder nachfolgend dem Darminhalt ausgesetzten weiteren Schicht, um einen bestimmten, nicht gezwungenermaßen immer gleichen, aber immer positiven Wert höher, als der bestimmte obere pH-Wert der danach aufgebrachten, bzw. der vorhergehend dem Darminhalt ausgesetzten Schicht.

Damit bei fallendem pH-Wert (ausgenommen beim Erreichen des Magens) mit einem Abfall von mindestens der gewünschten Höhe, bzw. auf jeden Fall bei einem Abfall mindestens in Höhe der Differenz zwischen dem bestimmten oberen pH Wert und dem bestimmten unteren pH Wert der aktuell der wässrigen Lösung ausgesetzten Schicht, alle weiteren noch vorhandenen Schichten löslich oder permeabel werden, und somit der oder die Wirkstoffe oder ein oder mehrere Wirkstoffkerne freigesetzt werden, ist der bestimmte untere pH-Wert jeder zuvor aufgebrachten, bzw. jeder nachfolgend dem Darminhalt ausgesetzten Schicht vorzugsweise höher als oder genau so hoch wie der bestimmte untere pH-Wert der danach aufgebrachten, bzw. der vorhergehend dem Darminhalt ausgesetzten Schicht.

Der bestimmte obere pH-Wert jeder weiteren Schicht ist um ein bestimmtes Maß "Delta 1" höher als der bestimmte obere pH-Wert der darüber liegenden Schicht. Der bestimmte untere pH-Wert jeder weiteren Schicht ist um ein bestimmtes Maß "Delta 2" niedriger als der bestimmte obere pH-Wert der darüber liegenden Schicht, aber vorzugsweise nicht niedriger als deren bestimmter unterer pH-Wert.

Dadurch wird folgendes sichergestellt: Bei monotoner Erhöhung des pH-Wertes löst sich jeweils immer nur eine Schicht auf oder wird permeabel, um die nächste Schicht freizusetzen.

Bei Ausführungsformen ohne innerste Schicht werden nach der Auflösung oder dem permeabel Werden der am weitesten innen liegenden weiteren Schicht der oder die Wirkstoffe freigesetzt.

Bei Ausführungen mit innerster Schicht wird bei monotoner Erhöhung des pH Wertes keine Freisetzung ausgelöst, es sei denn, der bestimmte zweite pH Wert der innersten Schicht wird überschritten.

Bei einer nach der erfolgten Magenpassage erneuten Erniedrigung des pH-Wertes um mindestens das bestimmte Maß "Delta 2" und maximal um die Summe der bestimmten Maße "Delta 1" und "Delta 2" lösen sich aber alle Beschichtungen auf oder werden permeabel, so dass der oder die Wirkstoffe freigesetzt werden.

Die innere Schicht weist einen bestimmten oberen pH-Wert "letzter pH Wert" auf, der üblicherweise über dem im Magen zu erwartenden pH-Wert liegt, und über dem diese Schicht löslich oder permeabel ist. Sind die darunter liegenden Schichten ausreichend lange beständig in dem pH-Bereich, in dem sie nicht löslich oder permeabel werden sollen, so kann der bestimmte obere pH Wert der inneren Schicht auch unterhalb des maximal im Magen zu erwartenden pH Wertes liegen. Dies ist besonders vorteilhaft bei Verabreichung an Individuen, welche aufgrund von inneren oder äußeren Umständen, wie beispielsweise Krankheiten oder Medikamenten, über relativ hohe Magen-pH-Werte verfügen, sowie auch für Individuen, welche aufgrund von inneren oder äußeren Umständen, wie beispielsweise Krankheiten oder Medikamenten, über relativ niedrige Dünndarm-pH-Werte verfügen, sowie für Individuen, welche beiden Gruppen angehören, weshalb eine weitere bevorzugte Ausgestaltung der Erfindung dies vorsieht.

Um sicher zu stellen, dass die innere Schicht vor dem Erreichen der Ileozäkalklappe löslich oder permeabel wird, sieht die Erfindung in einer weiteren bevorzugten Ausführungsform vor, dass der pH-Wert, über dem die innere Schicht löslich wird vorzugsweise nicht höher ist, als der niedrigste zu erwartende Maximalwert des pH-Wertes, der bei der Zielgruppe (Menschen oder Tiere, denen der oder die Wirkstoffe verabreicht werden sollen) im Dünndarm vorkommt. Dieser Wert ist beispielsweise bei Menschen typisch 6,5, kann aber bei bestimmten Personengruppen, beispielsweise mit gastrointestinalen Krankheiten, auch höher, vor allem aber auch niedriger sein, beispielsweise 6 oder teilweise auch darunter (minimale Werte von 5,5 oder 5 kommen teilweise vor).

Interindividuelle Variabilität auch bei ansonsten darmgesunden Individuen kann bewirken, dass im Dünndarm ein pH Wert von 7 nicht zuverlässig überschritten wird. Bei manchen Krankheiten, wie z.B. Morbus Crohn oder bei Individuen, deren Dünndarm zum Teil entfernt wurde, können die maximal im Dünndarm erreichten pH Werte beispielsweise unter pH 6 liegen, je nach Schwere der Erkrankung auch unter 5, z.B. wenn beide Erkrankungen vorkommen, oder bei Kombinationen von weiteren Ursachen für niedrige pH Werte sogar maximale pH Werte von unter 4,5. Die Erfindung sieht deshalb in einer weiteren bevorzugten Ausführungsform vor, dass der bestimmte obere pH Wert der inneren Schicht niedriger ist, als pH 7, bevorzugt niedriger als pH 6, mehr bevorzugt niedriger als pH 5 und besonders bevorzugt niedriger als pH 4,5.

Die innere Schicht kann auch unterhalb von bestimmten pH-Werten löslich oder permeabel sein, allerdings erst unterhalb eines pH-Wertes, der unterhalb des Wertes liegt, der bei der Zielgruppe im Gastrointestinaltrakt mindestens zu erwarten ist. Ebenso kann die innerste Schicht löslich oder permeabel sein oberhalb eines bestimmten pH Wertes (des bestimmten zweiten pH Wertes). Dieser bereich oberhalb des bestimmten zweiten pH Wertes kann sich erstrecken über den gesamten Bereich oberhalb des maximalen Wertes, der bei der Zielgruppe maximal zu erwarten ist, ohne dass dadurch das Freisetzungsverhalten maßgeblich verändert wird, weshalb dies in einer weiteren bevorzugten Ausführungsform der Erfindung vorgesehen ist. Für besondere Anwendungen kann es vorteilhaft sein, dass der bestimmte zweite pH Wert unterhalb des bei der Zielgruppe maximal zu erwartenden pH Wertes im Verlauf des Dünndarms liegt. Dies kann beispielsweise dazu genutzt werden, eine Freisetzung einzuleiten, wenn in einem Teil der Zielgruppe ein bestimmter pH Wert kurz vor Erreichen des Dickdarms überschritten wird. Eine weitere bevorzugte Ausführungsform der Erfindung sieht vor, dass der bestimmte zweite pH Wert unterhalb des bei der Zielgruppe maximal zu erwartenden pH Wertes im Verlauf des Dünndarms liegt, und zwar bevorzugt um 0,2 bis 1,2 pH Stufen, mehr bevorzugt um 0,4 bis 0,8 pH Stufen, besonders bevorzugt um 0,5 bis 0,65 pH Stufen, beispielsweise um 0,55 pH Stufen.

Durch die Möglichkeit, den bestimmten oberen pH Wert der inneren Schicht niedrig halten zu können, da eine Freisetzung erst nach ebenfalls erfolgter Auflösung oder permeabel Werdung der nächst inneren Schicht erfolgt, kann der bestimmte untere pH Wert der nächst inneren Schicht ebenfalls niedrig gehalten werden, was eben besonders der Realisierbarkeit von entsprechend freisetzungskontrollierten Verabreichungsformen für Individuen, welche aufgrund von inneren oder äußeren Umständen, über relativ niedrige Dünndarm-pH-Werte verfügen, zugute kommt.

Der bestimmte untere pH Wert der äußersten weiteren Schicht kann niedrig gehalten werden, beispielsweise um den oben genannten Krankheiten wie Morbus Crohn, Kurzdarmsyndrom oder ähnlichem Rechnung zu tragen, weshalb die Erfindung in einer weiteren bevorzugten Ausführungsform vorsieht, dass der bestimmte untere pH Wert der äußersten weiteren Schicht niedriger ist, als pH 7, bevorzugt niedriger als pH 6, mehr bevorzugt niedriger als pH 5 und besonders bevorzugt niedriger als pH 4.

Die innere Schicht ist entweder im gesamten pH Bereich unterhalb ihres bestimmten oberen pH Wertes oder mindestens bis unterhalb des minimal im Magen zu erwartenden pH Wertes beständig.

Bei der Bestimmung der Parameter "Delta 1" und "Delta 2" der einzelnen Schichten können diese Parameter bei allen weiteren Schichten gleich sein, aber auch unterschiedliche Werte annehmen, um gewünschte Freisetzungseigenschaften zu erhalten.

Der Wert "letzter pH Wert" weist bevorzugt einen Wert von 2 bis 9 auf, mehr bevorzugt einen Wert von 4 bis 8 und besonders bevorzugt einen Wert von 4,5 bis 7,5 auf, da maximale Magen-pH-Werte fast nie pH 7,5 erreichen, und der maximal im Dünndarm zu erwartende pH Wert meist nicht unterhalb von 4,5 liegt.

Der Wert "erster pH Wert" weist bevorzugt einen Wert von 2 bis 9 auf, mehr bevorzugt einen Wert von 3,5 bis 8 und besonders bevorzugt einen Wert von 4,5 bis 7,5 auf, da maximale Zäkum-pH-Werte weniger oft pH 8 erreichen, und der maximal im Dünndarm zu erwartende pH Wert meist nicht unterhalb von 4,5 liegt.

Die Parameter "Delta 1" und "Delta 2" der einzelnen Schichten weisen vorzugsweise Werte von 0,1 bis 2, mehr bevorzugt Werte von 0,25 bis 1, und besonders bevorzugt Werte von 0,4 bis 0,7 auf. Somit kann eine kontrollierte Freisetzung auch dann erreicht werden, wenn die interindividuelle Variabilität des maximal zu erwartenden Dünndarm-pH-Wertes kleiner 2 pH Stufen ist, wie bei sehr heterogen zusammengesetzten Zielgruppen, wenn die Variabilität kleiner 1 ist, wie bei normal zusammengesetzten Zielgruppen, und es kann bei entsprechend homogenen Zielgruppen oder bei Verwendung von mehreren weiteren Schichten durch Wahl kleiner "Delta 1" und "Delta 2" Maße auch Nutzen aus relativ kleinen pH Absenkungen beim Passieren der Ileozäkalklappe gezogen werden. Insbesondere bei Verwendung von mehreren weiteren Schichten kann eine kontrollierte Freisetzung auch dann erreicht werden, wenn die interindividuelle Variabilität des maximal zu erwartenden pH-Wertes im Dünndarm größer ist als 1 pH Stufe, vorzugsweise größer als 2 pH Stufen, mehr bevorzugt größer als 3 pH Stufen, und besonders bevorzugt größer als 4 pH Stufen.

Der pH Wert unterhalb dem sich die innerste Schicht oder eine weitere Schicht löst, liegt vorzugsweise nicht oberhalb des bestimmten pH Wertes, über dem sich die darüber liegende schicht löst, da ansonsten bei zu langsamer Steigung des pH Wertes die innerste oder weitere Schicht gleich nach der darüber liegenden Schicht löslich oder permeabel werden würde, ohne dass ein Abfall des pH-Wertes erforderlich wäre, weshalb eine weitere bevorzugte Ausführungsform der Erfindung eine solche bevorzugte Ausführung vorsieht.

Delta 2 kann aber auch 0 sein. Bei relativ niedriger Auflösungsgeschwindigkeit der Schichten und relativ schnell steigenden pH Werten im Dünndarm, kann Delta 2 auch negativ sein, ohne dass dadurch eine zu frühe Freisetzung erfolgt. Dadurch kann der Arbeitsbereich weiter vergrößert, oder der erforderliche Abfall des pH Wertes beim Eintritt in den Dickdarm weiter verringert werden. In einer weiteren bevorzugten Ausführungsform der Erfindung ist deshalb vorgesehen, dass Delta 2 nicht positiv sein muss, jedoch bevorzugt nicht negativer ist als -0,5, besonders bevorzugt nicht negativer als -0,25.

Damit sich die nächst innere Schicht nicht schon dann auflöst oder permeabel wird, wenn der Anstieg des pH-Wertes sehr langsam erfolgt, und Schwankungen des pH-Wertes auftreten, beispielsweise durch ungleichmäßige Vermischung des Darminhaltes mit Verdauungssäften, sieht eine weitere Ausgestaltung der Erfindung vor, dass der pH-Schwellwert, unterhalb dem die nächst innere Schicht löslich oder Permeabel wird (der bestimmte erste pH Wert der innersten Schicht oder der bestimmte untere pH Wert einer weiteren Schicht), um ein bestimmtes Maß tiefer liegt, als der pH-Schwellwert, über dem die über ihr angeordnete Schicht löslich oder permeabel wird (der bestimmte letzte pH Wert der inneren Schicht oder der bestimmte obere pH Wert einer weiteren Schicht).

Dieses Maß sollte mindestens 0,1 pH Stufen betragen, da Schwankungen unter diesem Wert sehr häufig vorkommen, bevorzugt 0,2 pH Stufen, da Schwankungen um diesen Wert öfters vorkommen, und besonders bevorzugt mindestens 0,4 Stufen, da Schwankungen um 0,2 bis 0,4 Stufen nicht ausgeschlossen sind. Das Maß sollte allerdings nicht größer als 2,5 pH Stufen sein, da der Abfall des pH Wertes nach Passage der Ileozäkalklappe üblicherweise nicht mehr als 2,5 pH Stufen beträgt, bevorzugt nicht höher als 1,5 Stufen, da er oft nicht über 1,5 Stufen beträgt, und besonders bevorzugt nicht mehr als 1 pH Stufe, da der Abfall nicht bei allen Säugetieren über 1 pH-Stufe beträgt.

Es wurde ebenfalls überraschend festgestellt, dass es bei der untersuchten Schichtfolge (Schichtenfolge) möglich war, sogar noch größere Schwankungen des pH Wertes innerhalb des Dünndarms zu tolerieren, ohne die gezielte Freisetzung im Dickdarm zu beeinträchtigen, und dass eine solche Schichtfolge vorteilhaft war.

Eine weitere bevorzugte Ausführungsform der Erfindung sieht deshalb vor, dass die Formulierung auch für Individuen geeignet ist, bei denen die Schwankungen des pH Wertes nach Verlassen des Magens und vor Erreichen der Ileozäkalklappe, bzw. die Abweichungen von der Monotonität des pH Verlaufes in diesem Bereich größer sind als 0,1 pH Stufe, bevorzugt größer als 0,3 pH Stufen, besonders bevorzugt größer als 0,6 pH Stufen.

Insbesondere bei wiederkäuenden Säugetieren sind größere und unter Umständen wiederholte Schwankungen des pH Wertes möglich, weshalb die Erfindung in einer weiteren bevorzugten Ausführungsform vorsieht, dass die beschriebenen Formulierungen nicht zur Verwendung bei wiederkäuenden Säugetieren bestimmt sind, bevorzugt nicht zur Verwendung bei wiederkäuenden Säugetieren, bei denen die Schwankungen des pH Wertes nach dem Verlassen des Magens größer sind als 1,0 pH Stufen, besonders bevorzugt nicht zur Verwendung bei wiederkäuenden Säugetieren, bei denen diese Schwankungen größer sind als 2,0 pH Stufen.

Besonders vorteilhaft ist die Verwendung der beschriebenen Formulierungen bei monogastrischen Säugetieren, weshalb die Erfindung dies in einer weiteren bevorzugten Ausführungsform vorsieht.

Eine weitere bevorzugte Ausführungsform der Erfindung sieht vor, dass das Säugetier, für das die Formulierung bestimmt ist, ein Mensch ist.

Eine weitere bevorzugte Ausführungsform sieht vor, dass die Beschichtung oder Umhüllung des oder der Wirkstoffe oder des oder der wirkstoffhaltigen Kerne sich erst dann vollständig auflöst oder permeabel wird, wenn der pH-Wert des die Beschichtung umgebenden Speisebreis um einen Betrag von 0,2 bis 4, mehr bevorzugt um einen Betrag von 0,4 bis 1,5, besonders bevorzugt um einen Betrag von 0,5 bis 0,8 sinkt, nachdem er zuvor über einen bestimmten Wert angestiegen war. Durch das Erfordernis, dass ein bestimmter Mindestbetrag an pH-Senkung für die Auflösung erforderlich ist, wird erreicht, dass der oder die Wirkstoffe nicht bereits bei kleineren Schwankungen des pH-Wertes innerhalb des Dünndarms freigesetzt werden.

Durch die eine oder mehreren weiteren Schichten ist es möglich, den bestimmten oberen pH Wert der inneren Schicht niedriger zu wählen, als den bestimmten unteren pH Wert der innersten Schicht oder der am weitesten innen gelegenen weiteren Schicht, ohne dass es zu einer unbeabsichtigten Freisetzung kommt, selbst wenn der pH Wert im Verlauf des Dünndarms nur langsam oder über längere Zeit gar nicht ansteigt. Eine weitere bevorzugte Ausführungsform der Erfindung sieht deshalb vor, dass der bestimmte obere pH Wert der inneren Schicht kleiner ist, als der bestimmte untere pH Wert der innersten Schicht. Eine weitere bevorzugten Ausführungsform der Erfindung sieht vor, dass der bestimmte obere pH Wert der inneren Schicht kleiner ist, als der bestimmte untere pH Wert der am weitesten innen gelegenen weiteren Schicht, sofern zwischen diesen beiden Schichten mindestens eine zusätzliche weitere Schicht angeordnet ist. Das Maß, um das der bestimmte obere pH Wert der inneren Schicht kleiner ist, beträgt jeweils bevorzugt von 0,2 bis 1,2 pH Stufen, mehr bevorzugt 0,4 bis 0,8 pH Stufen, besonders bevorzugt 0,5 bis 0,65 pH Stufen, beispielsweise 0,55 pH Stufen.

Damit die innere Schicht nicht schon in der Mundhöhle aufgelöst wird, sind dem Fachmann mehrere Möglichkeiten bekannt. Unter anderem die Verabreichung der wie beschrieben beschichteten Wirkstoffe innerhalb einer Kapsel aus Hartgelatine oder Hydroxypropylmethylcellulose oder auch das Aufbringen einer zusätzlichen, äußeren Beschichtung, welche sich erst im Magen auflöst.

Die verschiedenen Ausführungsformen der Erfindung ermöglichen es, dass die maximal zur Freisetzung erforderliche Verringerung des pH-Wertes innerhalb des Verdauungstraktes kleiner ist, als die interindividuelle Variabilität des maximal zu erwartenden pH Wertes innerhalb des Verdauungstraktes vor Erreichen der Ileozäkalklappe in der Gruppe von Individuen, für welche der freizusetzende Stoff, bzw. die freizusetzenden Stoffe bestimmt sind.

Die maximal zur Freisetzung erforderliche Verringerung des pH-Wertes innerhalb des Verdauungstraktes berechnet sich aus den folgenden Gleichungen:
Wert A: Maximalwert des bei der Zielgruppe zu erwartenden pH-Wertes im Dünndarm abzüglich des bestimmten pH-Wertes, unter dem sich die innerste Beschichtung auflöst oder permeabel wird.
Wert B: Abstand zwischen bestimmten oberen und bestimmten unteren pH-Wert oberhalb dem, bzw. unterhalb dem diejenige weitere Schicht löslich oder permeabel wird, bei der dieser Abstand am größten ist.

Der größte der zwei Werte A bis B stellt die maximal zur Freisetzung erforderliche Verringerung des pH-Wertes innerhalb des Verdauungstraktes dar.

Bei Ausführungsformen der Erfindung ohne innerste Schicht kommt nur Wert B zur Anwendung. Bei Ausführungsformen, bei denen der bestimmte zweite pH Wert der innersten Schicht kleiner ist, als der bei der Zielgruppe maximal zu erwartende pH-Wert im Dünndarm, berechnet sich Wert A aus der Differenz zwischen bestimmtem zweiten pH Wert und bestimmten ersten pH Wert der innersten Schicht. Das Verhältnis von erforderlicher Verringerung des pH Wertes zum Arbeitsbereich verbessert sich also um die Differenz zwischen dem bei der Zielgruppe maximal zu erwartende pH-Wert im Dünndarm und dem bestimmten zweiten pH Wert der innersten Schicht, bzw. dem bestimmten oberen pH Wert der am weitesten innen gelegenen weiteren Schicht. Diese Differenz bestimmt bei solchen Ausführungsformen auch das Maß, um das der Arbeitsbereich bei gleich bleibendem erforderlichem Abfall des pH Wertes nach oben erweitert werden kann.

Diese Differenz sollte nicht zu groß gewählt werden, da ansonsten bei Individuen, deren maximal im Dünndarm erreichter pH Wert am oberen Rand des Arbeitsbereiches liegt, der bestimmte obere pH Wert der am weitesten innen gelegenen weiteren Schicht, oder der bestimmte zweiten pH Wert der innersten Schicht, eventuell zu lange vor dem Erreichen des Dickdarms überschritten wird, und das Risiko einer Freisetzung bereits vor dem Erreichen des Dickdarms steigt. Die Differenz beträgt deshalb bevorzugt zwischen 0,1 und 1,2 pH Stufen, mehr bevorzugt zwischen 0,4 und 0,8 pH Stufen, besonders bevorzugt zwischen 0,5 und 0,65 pH Stufen, beispielsweise um 0,55 pH Stufen.

Die Ausnutzung der Veränderung des pH Wertes zur Freisetzung des oder der Wirkstoffe wird realisiert durch vorzugsweise mehrfach nacheinander erfolgende Beschichtung, Vermischung, Dispersion, Emulsion, Verpressung, Granulierung oder anderweitig ausgeführte Umhüllung des oder der Wirkstoffe, die ggf. vermischt, verpresst oder anderweitig mit oder ohne weiteren Zusatzstoffen verarbeitet sind, mit Materialien oder Materialmischungen, welche definierte Beständigkeiten gegenüber wässrigen Lösungen mit bestimmten pH Werten aufweisen. Die Materialien oder Materialmischungen sind in wässrigen Lösungen bestimmter Bereiche des pH Wertes löslich oder permeabel, und in wässrigen Lösungen anderer Bereiche des pH Wertes unlöslich oder nicht permeabel.

Die Materialien sind beispielsweise ausgewählt aus der Gruppe Polymere, Copolymere, Monomere, Gele, Polysaccharide, ggf. vermischt oder anderweitig verarbeitet mit pharmazeutischen Hilfsstoffen.

Durch die Abfolge verschiedener Beschichtungen wird erreicht, dass erst dann alle Schichten beseitigt, aufgelöst oder permeabel geworden sind, wenn nach einem Anstieg des pH Wertes über ein bestimmtes Maß ein Abfall des pH-Wertes um ein weiteres bestimmtes Maß, bzw. unter ein bestimmtes Maß erfolgt ist, oder bei besonderen Ausführungsformen wenn der Anstieg über ein bestimmtes höheres Maß erfolgt ist. Dies wird dadurch erreicht, dass sich jede Schicht erst dann auflöst oder permeabel wird, wenn ein bestimmter oberer pH-Wert überschritten, oder ein anderer bestimmter unterer pH-Wert unterschritten wird.

Zur Ausbildung der weiteren Schichten kommen beispielsweise Materialien zum Einsatz, die nur in bestimmten Bereichen des pH-Wertes beständig sind gegenüber wässrigen Lösungen, oder Mischungen verschiedener Materialien, von denen mindestens eines oberhalb bestimmter pH-Werte löslich oder zumindest permeabel in wässrigen Lösungen ist, und mindestens ein anderes unterhalb bestimmter pH-Werte löslich oder permeabel ist in wässrigen Medien, um eine Schicht zu erhalten, welche nur in dem Bereich beständig gegen wässrige Lösungen ist, in der mindestens diese beiden verwendeten Materialien beständig sind.

Beispielsweise, aber die Erfindung nicht auf diese beschränkend, seien hier folgende Materialien, welche vorzugsweise als Komponenten solcher Mischungen dienen können, genannt: Eudragit L 100, Eudragit FS 30D, Eudragit E 100, Kollicoat Smartseal 30D, Polyvinylacetal-Diethylaminoacetat, Polyvinylacetal-Dimethylaminoacetat, Poly(N-acryloyl-N'-ethyl piperazin-co-methyl methacrylat), poly(Diethylaminoethylmethacrylat-HCl)("Potentiometric Titrations of Polyelectrolytes with Separation of Phases", Shatkay et al.).

Der Aufbau, bzw. die Herstellung solcher Schichten beruht beispielsweise, die Erfindung aber nicht limitierend, auf den gleichen oder ähnlichen Schritten und Materialien wie bei den bereits erwähnten Schichten, die nur unterhalb oder nur oberhalb eines bestimmten pH-Wertes löslich oder permeabel sind, wobei die Bestandteile welche für die pH-abhängigen Eigenschaften hauptverantwortlich sind, wie z.B. die kationischen oder anionischen Polymere oder Copolymere, ausgetauscht sind gegen Polymere oder Copolymere oder andere filmbildende pH-abhängig löslich- oder permeable Materialien, die vorzugsweise sowohl kationische als auch anionische Eigenschaften aufweisen. Dies können beispielsweise kationische Polymere sein, deren Molekülstruktur mit anionischen Untereinheiten gepfropft ist, oder anionische Polymere mit kationischen Untereinheiten. Es können auch Polymere verwendet werden, bei denen einzelne, bzw. bestimmte Anteile ionischer Molekülteile, auch als funktionale Gruppen bezeichnet, gegen andere ionische Molekülteile ausgetauscht sind, welche gegensätzliche ionische Eigenschaften haben, beispielsweise Carboxylgruppen gegen Aminogruppen. Ebenso können andere Materialien verwendet werden, die löslich oder permeabel unterhalb und oberhalb eines bestimmten Bereiches des umgebenden pH Wertes sind, und beständig innerhalb des bestimmten Bereiches.

Ein die Erfindung nicht limitierendes Beispiel ist ein Chitosanpolymer, das kationische Eigenschaften aufweist, und somit löslich ist bei sauren pH-Werten aber unlöslich bei neutralen und basischen pH-Werten der umgebenden Lösung. Durch enzymatische Pfropfung mit Chlorogensäure werden Untereinheiten mit anionischen Eigenschaften eingefügt, wodurch das Polymer auch in basischen Lösungen löslich wird, und somit nur in einem begrenzten Bereich um den neutralen pH-Wert unlöslich ist.

Durch den prozentualen Anteil der Pfropfung kann die Löslichkeit des Polymers im basischen und sauren Bereich eingestellt werden, womit hauptsächlich der obere, aber auch der untere pH-Wert, über bzw. unter dem eine mit Hilfe dieses Polymers hergestellte Schicht löslich ist, bestimmt werden kann. Siehe "Enzymatic Grafting of a Natural Product onto Chitosan to Confer Water Solubility Under Basic Conditions" (Guneet Kumar et al, BIOTECHNOLOGY AND BIOENGINEERING, VOL. 63, NO. 2, APRIL 20, 1999).

Auch EP0466566B1 beschreibt, dass der pH Wert, unter dem Chitosan löslich ist, abhängig ist von dem Grad der Deacetylierung und dem Grad der Polymerisation, so dass dem Fachmann entsprechende Möglichkeiten zur Einstellung der gewünschten Parameter bekannt sind.

Unter anderem deshalb, weil Chitosan ein modifiziertes Naturprodukt ist, hängen die Löslichkeitsparameter auch von dem verwendeten Herstellungsprozess ab, so dass je nach Hersteller, Polymerisierungs- und Deacetylisierungsgrad unterschiedliche Pfropungsgrade erforderlich sein können, sowie die Schichtdicken unter Umständen angepasst werden müssen.

Andere Polymere, wie z.B. Amylose können auf ähnliche Weise modifiziert werden, um entsprechende Löslichkeitsprofile zu erreichen, wie beispielsweise die in vorgenannter Publikation erwähnten Veröffentlichungen. Hierzu ist es nicht zwingend erforderlich, dass enzymatische Verfahren verwendet werden.

Ein weiteres Beispiel für ein Polymer, das zur Herstellung einer oder mehrerer weiterer Schichten verwendet werden kann, ist ein Chitosanpolymer, das mit Bernsteinsäureanhydrid modifiziert wird, wie beispielsweise in "Zwitterionic chitosan derivatives for pH-sensitive stealth coating", Peisheng Xu et al., Biomacromolecules, 2010 September 13; 11(9): 2352-2358, beschrieben. Weitere Beschreibungen, wie funktionelle Molekülgruppen modifiziert werden können, sind in der chemischen Fachliteratur beschrieben, beispielsweise die Modifizierung von Aminogruppen in "Modification of Amino Groups" in "Current Protocols in Protein Science", Supplement 4, 1996, Wiley, hier insbesondere die Succinylierung, Amidierung und Methylierung, sowie Hilfestellungen bei möglicherweise auftretenden Problemen, sowie auch in Klotz, I.M. 1967. "Succinylation". Methods Enzymol. 11:576-580. Polyaspartamid-Polymere, entsprechende Copolymere und/oder gepfropfte Varianten wie z.B. PHEA-g-C₁₈10-IM50,PHEA-g-C₁₈10-IM90,PHEA-g-C₁₈10-PY45, PHEA-g-C₁₈10-PY70 in "Tunable phase transition behaviors of pH-sensitive polyaspartamides having various cationic pendant groups", Han Woong Park, Colloid Polym Sci (2009) 287:919-926, beschrieben, sind ebenso verwendbar. Die Gewichtsanteile der einzelnen funktionalen Gruppen können wie beschrieben variiert werden, auch in Verhältnissen, die dort nicht explizit genannt sind.

Dem Fachmann sind einige Arten der Realisierung von Formulierungen bekannt, die er anwenden kann, um die Freigabe von Wirkstoffen oder von wirkstoffhaltigen Kernen so lange zu verhindern, bis ein bestimmter pH Wert überschritten wurde.

Ihm stehen hierfür beispielsweise von der Industrie bereitgestellte Mittel zur Verfügung, die üblicherweise als "Enteric Coating" bezeichnet werden. Auch in der vorliegenden Erfindung werden solche Beschichtungen und ggf. die dafür vorgesehenen Materialien unter anderem als "Enteric Coating" (E) bezeichnet. Die innere Schicht der vorliegenden Erfindung wird üblicherweise unter Verwendung solcher oder ähnlicher Materialien hergestellt. Beispiele für hierfür verwendbare Produkte sind unter anderem, aber die Erfindung nicht beschränkend, Eudragit FS 30D, Eudragit S 100, Eudragit L 100, Eudragit L 100-55, Eudragit L 30 D-55. In mehreren Broschüren der Firma Evonik, Darmstadt, ist die Realisierung entsprechender Beschichtungen beschrieben. Weitere Beispiele sind Hydroxypropyl-Methylcellulose-Acetat-Succinat (HPMCAS; Shin-Etsu AQOAT®, Typen AS-LF, AS-MF, AS-HF; Shin-Etsu Chemical Co., Ltd., Niigata, Japan), cellulose acetate phthalate (CAP), hydroxypropyl methylcellulose phthalate (HPMCP), polyvinyl acetate phthalate (PVAP), cellulose acetate trimellitate, hydroxypropyl methylcellulose succinate, cellulose acetate succinate, cellulose acetate hexahydrophthalate, cellulose propionate phthalate, cellulose acetate maleate, cellulose acetate butyrate, cellulose acetate propionate, copolymer of methylmethacrylic acid and methyl methacrylate, copolymer of methyl acrylate, methylmethacrylate and methacrylic acid, copolymer of methylvinyl ether and maleic anhydride (Gantrez ES series), ethyl methyacrylate-methylmethacrylate-chlorotrimethylammonium ethyl acrylate copolymer, natural resins such as zein, shellac and copal collophorium, and several commercially available enteric dispersion systems (e.g., Eudragit L30 D-55), Kollicoat EMM30D, Estacryl 30D, Coateric, and Aquateric).

Ebenso sind dem Fachmann einige Arten der Realisierung von Formulierungen bekannt, die er anwenden kann, um die Freigabe von Wirkstoffen oder von wirkstoffhaltigen Kernen so lange zu verhindern, bis ein bestimmter pH Wert unterschritten wurde. Ihm stehen hierfür beispielsweise von der Industrie bereitgestellte Mittel zur Verfügung, die üblicherweise als "Gastric Coating" oder "Protective Coating" bezeichnet werden. Auch in der vorliegenden Erfindung werden solche Beschichtungen und die dafür vorgesehenen Materialien unter anderem als "Protective Coating" (P) bezeichnet, können aber auch als "Caecal Coating" (C) dienen. Die innerste Schicht bestimmter Ausführungsformen der vorliegenden Erfindung wird üblicherweise unter Verwendung solcher oder ähnlicher Materialien hergestellt. Beispiele für hierfür verwendbare Produkte sind unter anderem, aber die Erfindung nicht beschränkend, Eudragit EPO, Eudragit E 100, Kollicoat Smartseal 30D, Poly(Methylmethacrylat-Diethylaminoethylmethacrylat)-Copolymere (Poly(MMA-DEAEMA)), Poly(N-acryloyl-N'-ethyl piperazin-co-methyl methacrylat)-Copolymere, Poly(Methylmethacrylat-Dimethylaminoethylmethacrylat)-Copolymere (Poly(MMA-DEAMMA)). In mehreren Broschüren der Firma Evonik, Darmstadt, bzw. der Fa. BASF, ist die Realisierung entsprechender Beschichtungen beschrieben.

Eine Einstellung des pH-Wertes, ober- bzw. unterhalb dessen eine Schicht löslich oder permeabel wird, kann auch durch Copolymerisation verschiedener Monomere erfolgen, die unterschiedliche hydrophobe, hydrophile oder ionische Eigenschaften aufweisen.

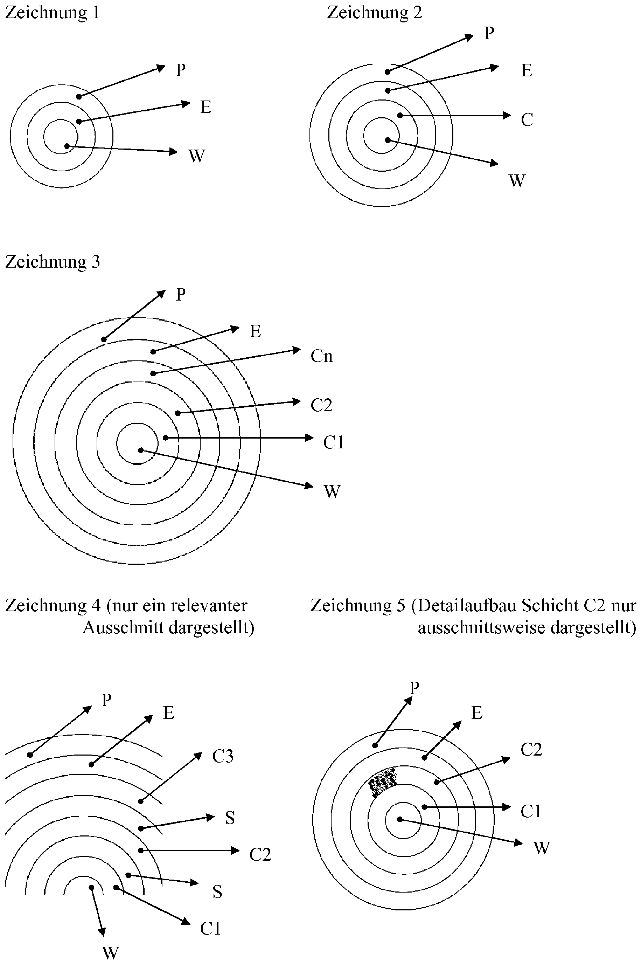

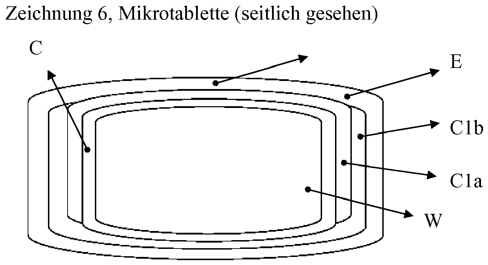

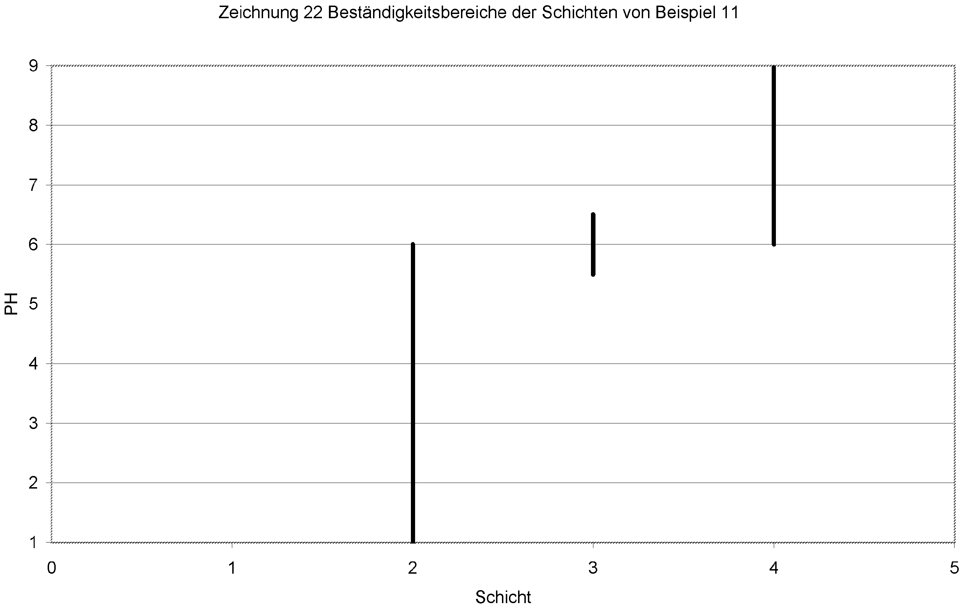

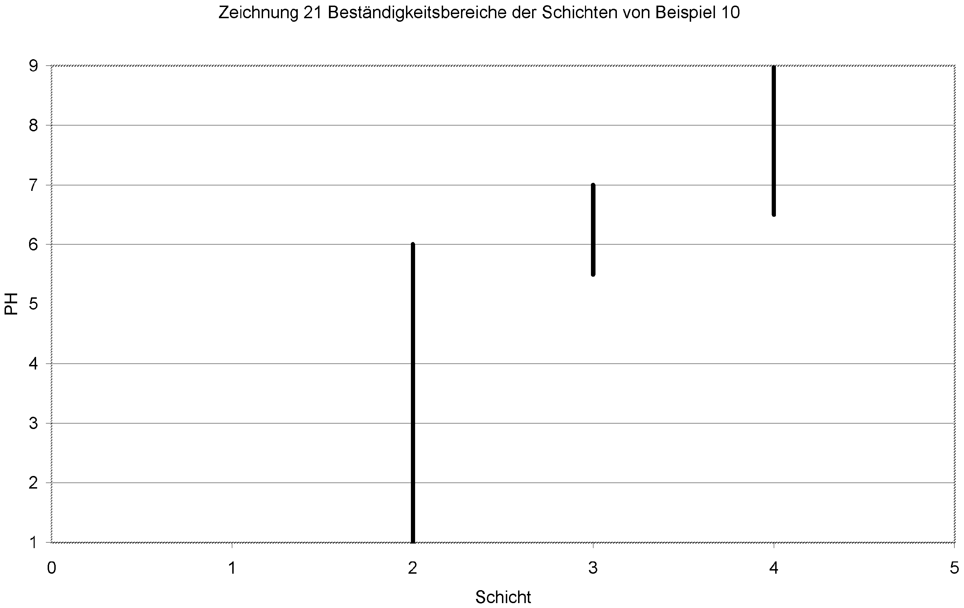

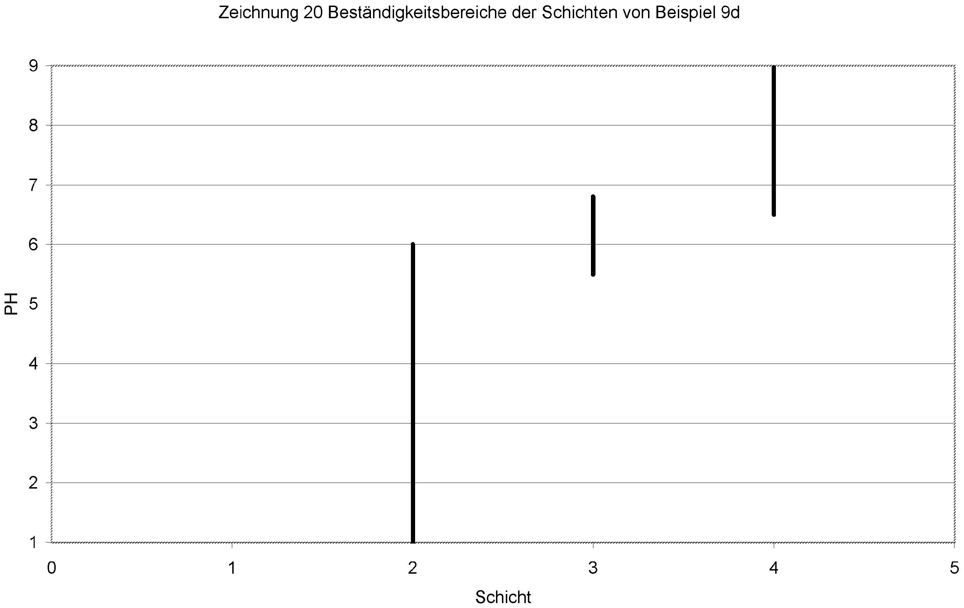

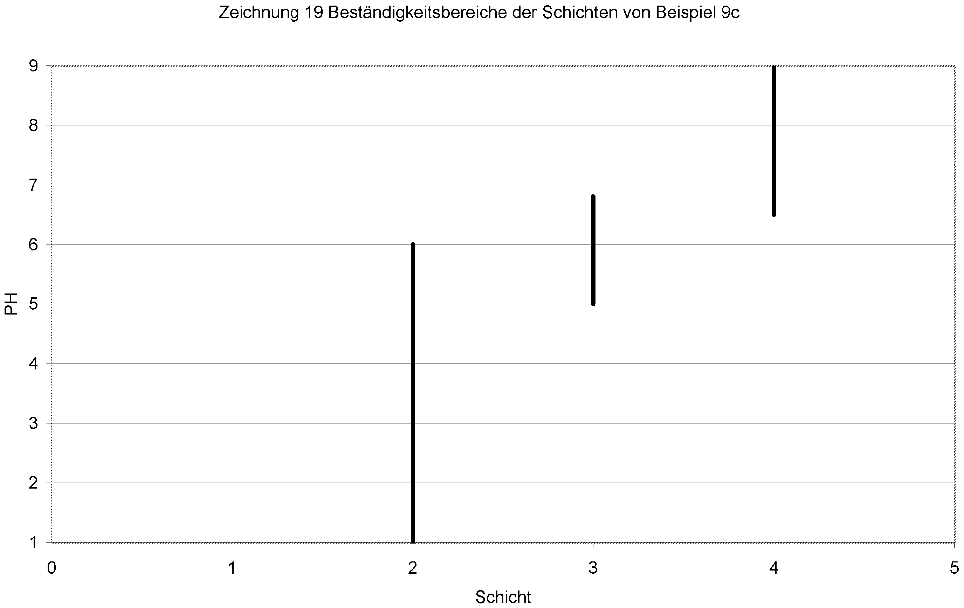

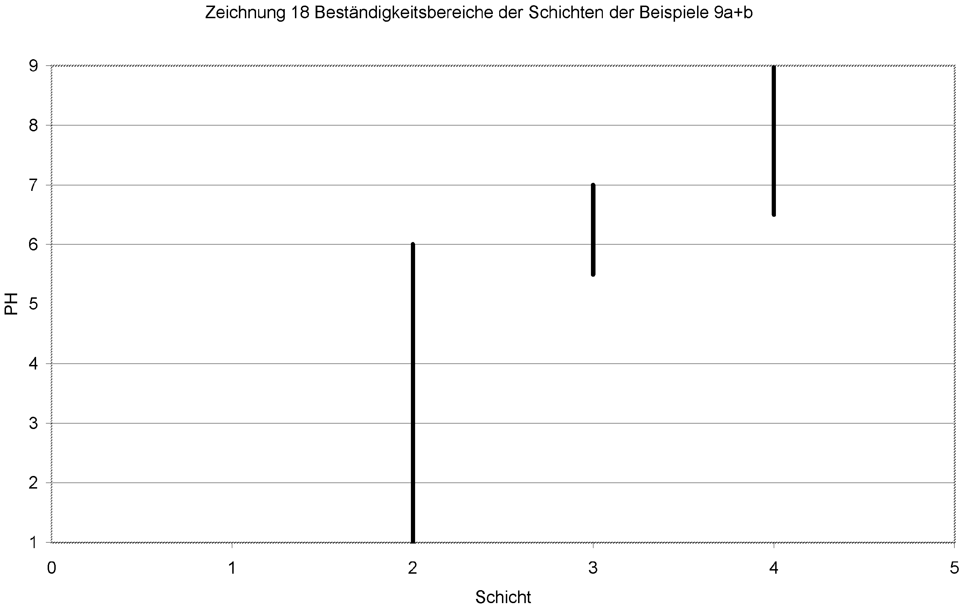

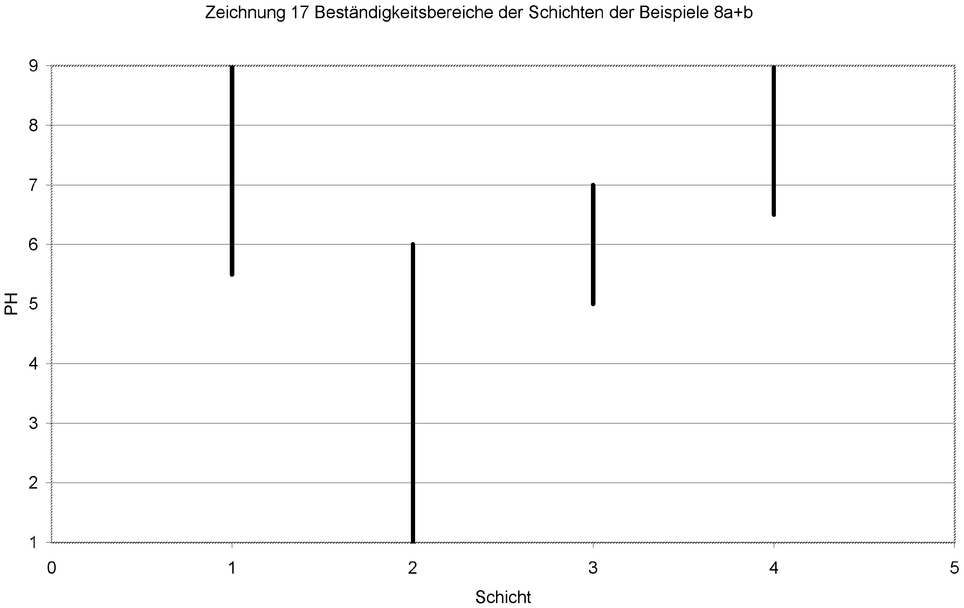

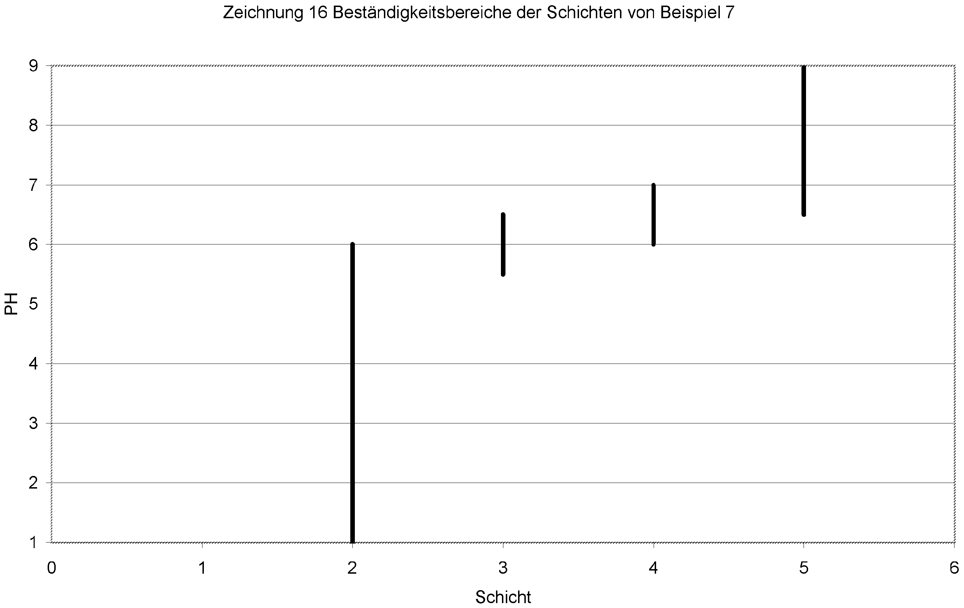

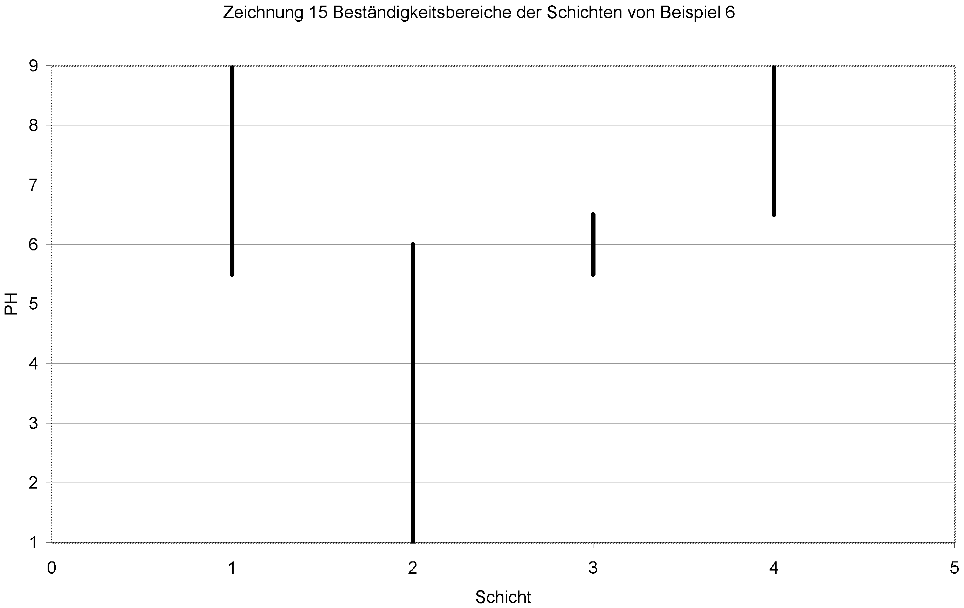

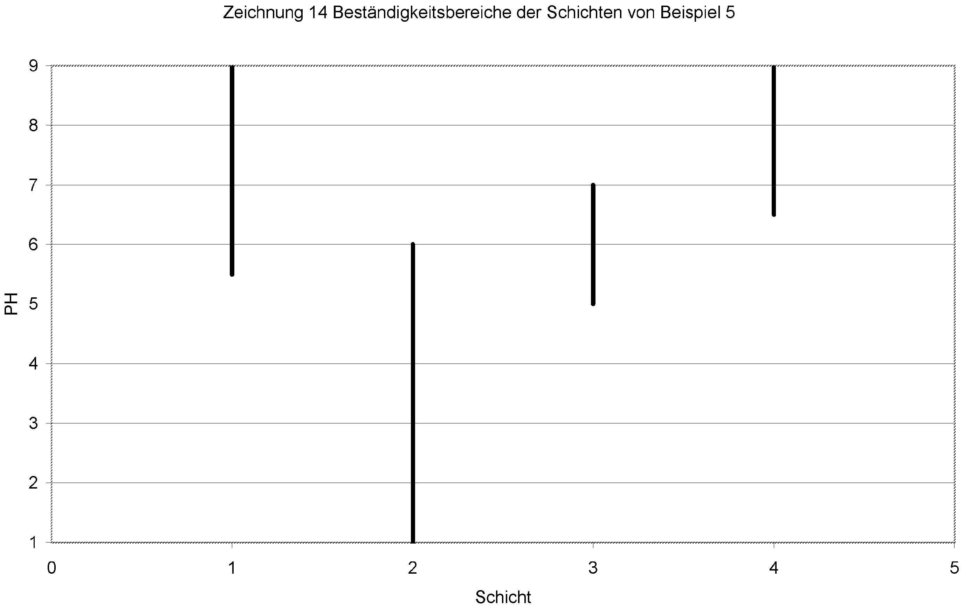

Beispielhaft sei hier verwiesen auf "Tunable phase transition behaviors of pH-sensitive polyaspartamides having various cationic pendant groups", Han Woong Park, Colloid Polym Sci (2009) 287:919-926. Dort werden verschiedene Polymere synthetisiert, die in begrenzten pH Bereichen unlöslich sind, darüber und darunter allerdings löslich. Die Einstellung der pH-Werte, ober- bzw. unterhalb derer eine Schicht löslich oder permeabel wird, kann einerseits durch die Art der verwendeten Monomere erfolgen, insbesondere durch Verwendung von Monomeren mit unterschiedlichen pKs Werten, oder über deren Anteile am Copolymer, beispielsweise über variable Mengenverhältnisse, siehe "Enteric microparticles coated with smart polymers for controlled drug delivery applications" (Annalisa Dalormo), insbesondere Kapitel 3, sowie speziell Figure 29. Beispielsweise können die Schwellwerte von Poly(MMA-AA)-Copolymeren zwischen pH 2,7 und pH 8,3 eingestellt werden, wodurch sie bevorzugt zwischen pH 2 und pH 9 zum Einsatz kommen können.

Analog zu der Einstellung des pH Wertes, oberhalb dessen z.B. ein Poly(MMA-AA)-Copolymer löslich wird, durch Änderung des prozentualen Gehalts an Acrylsäure, kann beispielsweise auch der pH Wert, unterhalb dessen z.B. ein Poly(MMA-DEAEMA)-Copolymer, wie es in Kollicoat Smartseal 30D verwendet wird, löslich wird, durch Änderung des prozentualen Gehalts an Diethylaminoethyl-Methacrylat eingestellt werden. Dem Fachmann sind hierzu verschiedene Möglichkeiten bekannt. Auch Polyvinylacetal-Diethylaminoacetat kann durch das Verhältnis der Monomere entsprechend angepasst werden. Der pH Wert, unterhalb dessen ein Poly(N-acryloyl-N'-ethyl piperazin-co-methyl methacrylat)-Copolymer in wässrigen Lösungen bei 37°C löslich wird, kann beispielsweise durch den prozentualen Gehalt an N-acryloyl-N'-ethyl piperazin eingestellt werden, beispielsweise zwischen pH 7 und pH 9 bei 52 bis 62mol% AcrNEP wie beschrieben in "Solution Properties of Water-Soluble "Smart" Poly(N-acryloyl-N'-ethyl piperazine-co-methyl methacrylate)", G. Roshan Deen, Polymers 2012, 4, 32-45; doi:10.3390/polym4010032. Mit den unterhalb eines bestimmten pH Schwellwertes löslichen Poly(MMA-DEAEMA)-, Poly(MMA-DEAMMA)- und Poly(MMA-AcrNEP)-Copolymeren kann ein Anwendungsbereich zwischen pH 2 und pH 9 abgedeckt werden. Insbesondere bei Schwellwerten von pH 7,0 und darüber kann es vorteilhaft sein, entsprechende Poly(MMA-DEAEMA)-Copolymere durch Poly(MMA-AcrNEP)-Copolymere mit vergleichbaren Schwellwerten zu ersetzen, z.B. um höhere Glasübergangstemperaturen zu erreichen. Hierdurch kann ein geringeres Risiko für ein Verkleben der Partikel beim Beschichten erreicht werden. Die Ausführungsbeispiele können entsprechend abgewandelt werden.

Um feine Abstufungen der Löslichkeits-Grenzwerte einstellen zu können, müssen nicht unbedingt jeweils für jede Beschichtung spezielle Polymere oder Copolymere mit eigenen Monomerverhältnissen synthetisiert werden. In gewissen Bereichen ist eine Einstellung der Löslichkeits-Grenzwerte auch durch Mischung von Copolymeren mit verschiedenen Monomeranteilen möglich, wie beispielsweise durch Mischung von organischen Sprühlösungen von Eudragit L und Eudragit S in unterschiedlichen Gewichtsanteilen

Bevorzugt enthalten die verwendeten Polymere oder Copolymere bis zu 95% hydrophobe Bestandteile, mehr bevorzugt bis zu 70%, noch mehr bevorzugt bis zu 50% und besonders bevorzugt bis zu 30%.

Bei Verwendung von gepfropften Polymeren sind diese bevorzugt zu 15% bis 85% gepfropft, mehr bevorzugt zu 30% bis 70% und besonders bevorzugt zu 42% bis 60%.

Die Molekülgewichte (in Dalton oder g/mol) der verwendeten Polymere liegen bevorzugt über 10000, mehr bevorzugt über 35000, besonders bevorzugt über 100000 und speziell bevorzugt zwischen 150000 und 375000.

Es kann auch vorteilhaft sein, wenn die verwendeten Polymere eher niedrige Molekülgewichte haben. Beispielsweise kann dies vorteilhaft sein, wenn die Schichten nach Überschreitung oder Unterschreitung bestimmter pH Werte möglichst schnell aufgelöst werden sollen. Vorzugsweise liegen die Molekülgewichte (in Dalton oder g/mol) der verwendeten Polymere unter 220000, mehr bevorzugt unter 100000, besonders bevorzugt unter 40000, noch mehr bevorzugt unter 23000 und speziell bevorzugt zwischen 15000 und 45000.

Die erzeugten Schichten weisen vorzugsweise einen Polymeranteil von mehr als 30% auf, mehr bevorzugt von mehr als 50%, besonders bevorzugt mehr als 70%, und besonders bevorzugt von mehr als 90%.

Der Anteil an pH-abhängig löslich- oder permeablen Polymeren am Polymeranteil einer oder mehrerer Schichten beträgt vorzugsweise mehr als 5%, mehr bevorzugt mehr als 10%, noch mehr bevorzugt mehr als 25%, besonders bevorzugt mehr als 50% und speziell bevorzugt mehr als 80%.

Der Anteil an Weichmacher im Verhältnis zum Polymeranteil beträgt bevorzugt weniger als 60 Gewichtsprozente, mehr bevorzugt weniger als 25 Gewichtsprozente, noch mehr bevorzugt weniger als 15 Gewichtsprozente und besonders bevorzugt zwischen 2 und 7 Gewichtsprozenten oder zwischen 8 und 13,5 Gewichtsprozenten.

Bevorzugt in den Polymeren der innersten Schicht und/oder der einen oder mehreren weiteren Schichten enthaltene Monomere sind Acrylsäure, Methacrylsäure, Diethylaminoethylmethacrylsäure, Dimethylaminoethylmethacrylsäure, Diethylaminomethylmethacrylsäure, Dimethylaminomethylmethacrylsäure, Vinylacetat, Diethylaminoacetat, Dimethylaminoacetat,Glucosamin, wobei bevorzugt mindestens eines der Monomere einen Anteil an den gesamten Monomeren hat von mehr als 15% (Gewicht), mehr bevorzugt mehr als 25%, noch mehr bevorzugt mehr als 50%, besonders bevorzugt mehr als 65% und speziell bevorzugt zwischen 45% und 75%.

Bevorzugt in den Polymeren der inneren Schicht und/oder der einen oder mehreren weiteren Schichten enthaltene Monomere sind Acrylsäure, Methacrylsäure, Methylmethacrylsäure, Ethylacrylsäure, Vinylpyrrolidon, Vinylacetat, Glucosamin.

Bevorzugt hat mindestens eines der verwendeten Monomere, besonders bevorzugt das niedermolekularste verwendete Monomer, einen Anteil an den gesamten Monomeren von mehr als 15% (Gewicht), mehr bevorzugt mehr als 25%, noch mehr bevorzugt mehr als 50%, besonders bevorzugt mehr als 65% und speziell bevorzugt zwischen 45% und 75%.

Damit die pH Werte, oberhalb oder unterhalb dessen die verwendeten Materialien löslich oder permeabel werden, gut eingestellt werden können, kann es vorteilhaft sein, Materialien zu verwenden, deren isoelektrischer Punkt nicht zu weit von dem angestrebten Grenzwert zwischen beiden Zuständen (löslich und unlöslich, bzw. permeabel und unpermeabel) entfernt ist. Die Erfindung sieht deshalb in einer weiteren bevorzugten Ausführungsform vor, dass die verwendeten Materialien, wie beispielsweise, aber nicht die Erfindung limitierend, Polymere, Copolymere Polysaccharide, Monomere, etc. einen Abstand zwischen ihrem isoelektrischen Punkt und mindestens einem beabsichtigten Grenzwert zwischen löslichem und unlöslichen Zustand, bzw. permeablem und unpermeablen Zustand, aufweisen, der nicht größer ist als 6 pH Stufen, bevorzugt nicht größer als 3 pH Stufen, und besonders bevorzugt nicht größer als 1,5 pH Stufen.

Die genannten Copolymere und Monomere stellen nur Beispiele dar und sollen die Erfindung nicht auf die Benutzung genau dieser begrenzen, sondern nur als Beispiele dienen.

Beispielhafte Realisierungen stellten die Ausführungsbeispiele 5 und 6 dar. Die zugehörigen Diagramme in den Zeichnungen 14 und 15 zeigen die Beständigkeit der einzelnen Schichten gegenüber Lösungen mit bestimmten pH Werten.

Da die einzelnen Schichten im Gastrointestinaltrakt dem sie umgebenen Darminhalt jeweils nur eine begrenzte Zeit widerstehen müssen, da beispielsweise die Dünndarmpassagezeit zwar gewisse interindividuelle Variationen aufweist, aber bis auf wenige Ausnahmen sechs Stunden nicht überschreitet, muss eine Schicht oder eine Matrix bei einem bestimmten pH-Wert nicht für unbegrenzte Zeit beständig sein, um im Sinne dieser Erfindung als nicht löslich, bzw. nicht permeabel zu gelten. Es reicht aus, wenn die Schicht oder Matrix die darunter liegende Schicht oder den oder die darunter liegenden Wirkstoffe so lange vor der umgebenden Lösung schützt, wie es im Verlauf der Darmpassage notwendig ist.

Bevorzugt sind die Schichten in dem als beständig, unlöslich oder nicht permeabel bezeichneten pH Bereich zu nicht mehr als 80% ihrer Stärke löslich, mehr bevorzugt zu nicht mehr als 65%, noch mehr bevorzugt zu nicht mehr als 50%, besonders bevorzugt zu nicht mehr als 35%, bzw. für nicht mehr als 50% des oder der Wirkstoffe permeabel, bevorzugt nicht mehr als 30%, mehr bevorzugt nicht mehr als 10%, besonders bevorzugt nicht mehr als 5%, und zwar bevorzugt innerhalb einer Zeitspanne von mehr als einer Stunde, mehr bevorzugt länger als 2 Stunden, noch mehr bevorzugt länger als 3 Stunden, besonders bevorzugt länger als 4 Stunden.

Dem Fachmann stehen verschiedene Methoden zur Verfügung, durch Variation der Anteile der Bestandteile einer Schicht, wie z.B. Weichmacher, Porenbildner, weitere Polymere oder Copolymere, weiteren pharmazeutischen Hilfsstoffen (Excipients), deren Schichtstärke, Partikelgröße etc. die Löslichkeit bzw. Permeabilität einer Schicht, insbesondere auch die Dauer bis zu einer ausreichenden Auflösung oder der Erreichung einer ausreichenden Permeabilität oder die Dauer einer entsprechenden Beständigkeit einzustellen.

Ebenso sind dem Fachmann Möglichkeiten bekannt, die Auflösung einer löslich oder permeabel werdenden Schicht zu beschleunigen, beispielsweise durch eine unter dieser Schicht angeordneten zusätzlichen Schicht mit einem Sprengmittel, welches schon bei geringer Permeabilität der darüber angeordneten Schicht und dadurch ausgelösten Kontakt mit der umgebenden Lösung aufquillt und die permeabel gewordene Schicht absprengt.

Eine beispielhafte Schichtfolge zeigt Zeichnung 4.

Eine Beschleunigung der Auflösung kann auch dadurch erreicht werden, dass ein Sprengmittel direkt in die funktionale Schicht eingearbeitet wird, weshalb dies in einer weiteren bevorzugten Ausführungsform der Erfindung vorgesehen ist. Beispielhafte Realisierungen solcher Schichten finden sich in der dem Fachmann bekannten Literatur, beispielsweise in "Pulsatile drug delivery to ileo-colonic segments by structured incorporation of disintegrants in pH-responsive polymer coatings.", Schellekens et al., Journal of Controlled Release, 2008.

Dem Fachmann sind auch verschiedene Möglichkeiten bekannt, um das Quellverhalten der verwendeten Materialien oder Materialmischungen zu beeinflussen. Beispielsweise können hydrophile, hydrophobe oder amphiphile Stoffe zugemischt werden. Es können auch weitere Polymere, Copolymere, Filmbildner, Füllstoffe oder andere entsprechend geeignete Hilfsstoffe verwendet werden. Beispielhaft seien Fettsäureester, z.B. Dekaglycerin-Monopalmitinsäureester genannt, aber auch Ethylcellulose, Mikrokristalline Cellulose, Polymethylmethacrylate.

Welche Stoffe hierfür unter anderem verwendet werden können, sowie Anleitungen zum einfachen Ermitteln der zielführenden Mengenanteile, kann der Fachmann aus der entsprechend einschlägigen Literatur entnehmen. Beispiele hierfür wären US20050220861 (z.B. Verwendung von Ethylcellulose), US 2010/0047323 (z.B. Verwendung von Palmitinsäureester) und die dort genannten Dokumente.

Eine bevorzugte Ausführungsform zeigt Ausführungsbeispiel 5c.

Eine weitere Möglichkeit, Quellverhalten und Auflösungsverhalten einer Schicht zu beeinflussen, besteht in der Reprotonierung oder Deprotonierung der Schicht, oder zumindest deren oberflächlicher Anteile, weshalb die Erfindung dies in einer weiteren bevorzugten Ausführungsform vorsieht.

Beispielsweise kann eine Schicht aus einem kationischen Polymer, welches mit Hilfe einer Säure gelöst wurde, auch nach dem Abschluss der Filmbildung und Trocknung noch geringe Anteile der entsprechenden Säure enthalten, welche dazu führen können, dass die Schicht auch dann quillt oder angelöst wird, wenn sie von einer wässrigen Lösung umgeben wird, deren pH Wert noch oberhalb des pH Wertes liegt, unterhalb dem sie löslich oder permeabel werden soll.

Durch eine Nachbehandlung der Schicht mit einer alkalischen Pufferlösung können die verbliebenen Reste der Säure herausgelöst oder zu Salzen umgewandelt werden, so dass sie keine maßgebliche Verschiebung des inneren pH Wertes bewirken können.

Eine beispielhafte Ausführung wäre die Nachbehandlung der aus einer essigsäurehaltigen Lösung hergestellten Schichten aus verschiedenen aufgeführten Ausführungsbeispielen mit einem 0,1-molaren Phosphatpuffer mit einem pH Wert von einer pH Stufe oberhalb des pH Wertes, unterhalb dessen die entsprechende Schicht löslich werden soll. Die nachzubehandelnden Partikel (Kapseln, Tabletten, Pellets etc.) werden für 5 bis 20 Sekunden in das Nachbehandlungsmedium eingetaucht oder mit diesem besprüht. Danach wird die Nachbehandlungslösung durch Eintauchen der Partikel in (oder Besprühen mit) deionisiertem Wasser entfernt, und die Partikel werden getrocknet..

Der pH Wert einer solchen Nachbehandlungslösung kann auch zwischen 0,5 und 3 pH Stufen höher sein. Vorzugsweise ist er zwischen 0,8 und 2 pH Stufen höher. Teilweise sind auch höhere Unterschiede als 3 pH Stufen sinnvoll. Der optimale pH Wert, die optimale molare Konzentration der Nachbehandlungslösung und die Nachbehandlungsdauer für die jeweilige Anwendung kann durch einfache Auflösungsversuche der nachbehandelten Schichten ermittelt werden. Je nach Schichtdicke und Verfahren kann eine Nachbehandlungszeit von bis zu 10 Minuten oder auch darüber notwendig sein, um das gewünschte Auflösungsverhalten der Schicht zu erreichen.

Nach Anwendung der Nachbehandlungslösung wird diese mit deionisiertem Wasser abgespült und die nachbehandelte Schicht getrocknet.

Entsprechende Realisierungsmöglichkeiten zeigen auch die Ausführungsbeispiele 20 und 21.

Ähnlich wie bei kationischen Polymeren, die in Säuren gelöst zur Anwendung bei der Schichtbildung kommen können, und bei denen dann zur Nachbehandlung der hergestellten Schichten vorwiegend alkalische Lösungen zum Einsatz kommen, können bei anionischen Polymeren, welche in alkalischen Lösungen gelöst zur Anwendung kommen können, vorwiegend saure Lösungen zur Nachbehandlung eingesetzt werden.

Beispielsweise können Schichten, welche aus mit Hilfe von Ammoniumsalzen (z.B. Ammoniumhydroxid) in Wasser gelösten Polymeren hergestellt wurden, mit Pufferlösungen nachbehandelt werden, welche einen pH Wert aufweisen, der 0,5 bis 3 pH Stufen, bevorzugt 1 bis 2 pH Stufen, besonders bevorzugt 1,5 pH Stufen unterhalb dem pH Wert liegt, oberhalb dem sich die nachzubehandelnden Schichten auflösen oder permeabel werden sollen. Die Ermittlung der optimalen pH Werte, der molaren Konzentrationen und der Nachbehandlungszeiten gestaltet sich ähnlich wie oben beschrieben.

Auch Schichten, die unter Verwendung von Materialien hergestellt sind, die sowohl anionische, als auch kationische Eigenschaften haben, kann je nach verwendeter Lösung eine Reprotonierung und/oder Deprotonierung durchgeführt werden. Die dafür verwendeten Lösungen oder Puffer weisen pH Werte auf, die vorzugsweise innerhalb des Bereiches liegen, innerhalb dem die entsprechende Schicht beständig sein soll. Eine weitere Möglichkeit, das Quell- und Auflösungsverhalten besonders von chitosanhaltigen Schichten zu verbessern, besteht in der Verwendung von Kohlensäure als Lösungsmittel für das Chitosan. Aufgrund der erhöhten Flüchtigkeit von Kohlensäure gegenüber vielen anderen Säuren, ist es möglich, chitosanhaltige Schichten herzustellen, die geringere Restgehalte an Lösungsmittel aufweisen, und dadurch bei Kontakt mit wässrigen Lösungen oberhalb ihres pH Grenzwert, unterhalb dessen sie löslich werden, weniger Quellneigung zeigen. Die in den Ausführungsbeispielen gezeigten Verfahren zur Herstellung chitosanhaltiger Schichten können mit geringen Abwandlungen auch mit Kohlensäure anstelle von Essigsäure durchgeführt werden. Die verwendeten Lösungen sollten möglichst bis zur Verwendung und vorzugsweise auch während dem Sprühprozess unter CO2-Begasung stehen (im Druckbehälter mit CO2 Vorspannung oder durch ständiges Einblasen von CO2 in den Lösungsbehälter). Bei Beschichtungsprozessen im Granulator und anderen Powder-Layering-Verfahren, bei denen die Filmbildung langsamer vonstatten geht, ist die Anlage ggf. einer kontrollierten CO2 Atmosphäre auszusetzen, um eine gleichmäßige Verdunstung des Wassers und des darin gelösten CO2 zu erreichen, so dass das gelöste Chitosan nicht vor Eintritt der Filmbildung ausfällt.

Gegebenenfalls sind die Konzentrationen der verwendeten Lösungen anzupassen, um Lösungen mit gewünschten Viskositäten zu erhalten. Entsprechend sind die Auftragsmengen der Lösungen anzupassen, um die gewünschte Auftragsmenge an Polymer zu erreichen.

Die Herstellung entsprechender Chitosan-Lösungen kann beispielsweise wie in "A Novel Method of Dissolving Chitosan in Water for Industrial Application", Yasuo SAKAI et al., Polymer Journal, Vol. 33, No. 8, pp 640-642 (2001), oder "Chitosan-Coating of Cellulosic Materials Using an Aqueous Chitosan-CO2 Solution", Yasuo SAKAI et al., Polymer Journal, Vol. 34, No. 3, pp 144-148 (2002), beschrieben erfolgen. Der Einsatz von Kohlensäure als Lösungsmittel kann auch bei anderen Materialien mit kationischen Eigenschaften angewendet werden.

Eine Beschleunigung der Auflösung kann auch erreicht werden, indem beispielsweise unter der pH-abhängig löslichen oder permeabel werdenden Schicht eine zusätzliche Schicht angeordnet wird, welche in wässrigen Lösungen löslich oder quellbar ist, und welche durch Lösung oder Quellung Stoffe freisetzt, die den pH-Wert der Lösung in der näheren Umgebung verändern, und somit zu einer schnelleren Auflösung der darüber liegenden Schicht beitragen können. Eine weitere bevorzugte Ausführungsform der Erfindung verwendet diese Möglichkeit. Beispielsweise wird unterhalb einer Schicht, die sich unterhalb eines bestimmten pH-Wertes möglichst schnell auflösen soll (beispielsweise hergestellt durch Verwendung von Kollicoat Smartseal 30D oder Chitosan), eine wasserlösliche Polymerschicht angeordnet (z.B. basierend auf Hydroxypropylmethylcellulose), in welcher ein Stoff eingebettet ist, der geeignet ist, den pH-Wert der umgebenden Lösung zu senken, beispielsweise Zitronensäure, Essigsäure, Salzsäure etc.

Eine beispielhafte Realisierung stellt Ausführungsbeispiel 7 dar. Das zugehörige Diagramm in Zeichnung 16 zeigt die Beständigkeit der einzelnen Schichten gegenüber Lösungen mit bestimmten pH Werten.

Eine weitere bevorzugte Ausführungsform der Erfindung sieht vor, dass die Schicht, unter der solch eine beschleunigende Schicht angeordnet ist, erst dann ausreichend permeabel wird, um die beschleunigende Schicht aktiv werden zu lassen, wenn der pH Wert der umgebenden Lösung entweder den bestimmten unteren, bzw. ersten pH Wert unterschritten oder den bestimmten oberen, bzw. letzten pH Wert überschritten hat, bzw. sich den genannten bestimmten pH Werten stark angenähert hat, so dass die Aktivierung der beschleunigenden Schicht nicht durch zeitabhängige Diffusion von Wassermolekülen durch die überlagerte Schicht hindurch erfolgt, zumindest nicht dann, wenn sich der umgebende pH Wert in dem Bereich befindet, in dem die überlagerte Schicht beständig sein soll, und bevorzugt nicht während der Zeitspanne, in der die überlagernde Schicht beständig sein soll.

Da der Anstieg des pH-Wertes innerhalb des Dünndarms langsamer erfolgt, als der Abfall beim Transit der Ileozäkalklappe, und somit bei Überschreitung des bestimmten oberen pH-Wertes einer Schicht, die Schicht der Lösung mit diesem pH-Wert länger ausgesetzt ist, als einer Lösung, welche den unteren pH-Wert unterschreitet, ist es nicht kritisch, wenn die Auflösung der Schicht nur bei Unterschreitung des bestimmten unteren pH-Wertes beschleunigt wird, also aus der darunter liegenden Schicht nur den pH-Wert senkende Substanzen freigesetzt werden. Deshalb sieht die Erfindung dies in einer weiteren bevorzugten Ausführungsform vor.

Bekannt sind auch Stoffe, die durch Kontakt mit der umgebenden Lösung mit dieser eine chemische Reaktion eingehen, und auf diesem Wege, beispielsweise durch eine weitere Veränderung des pH-Wertes zum schnelleren Auflösen oder permeabel werden der pH-sensitiven Schicht beitragen.

Beispielsweise werden solche Zwischenschichten in US2010/0129446A1 beschrieben.

Eine weitere Möglichkeit, die Auflösung von pH-sensitiven Schichten zu beschleunigen, ist die zumindest partielle Neutralisation der Polymerdispersionen, wie sie unter anderem in EP0978275A1, EP1906939A2 und EP1848751B1 beschrieben ist. Diese Technik kann auch bei der vorliegenden Erfindung benutzt werden, um entsprechende weitere bevorzugte Ausführungsformen zu realisieren. Ebenso können die in EP0978275A1 und EP1848751B1 beschriebenen Beschichtungsprozesse zur Realisierung von Schichten benutzt werden, mit denen bestimmte Ausführungsformen der Erfindung ausgeführt werden können, jeweils gegebenenfalls unter Austausch der Polymere oder Copolymere gegen solche, die die gewünschten pH-abhängigen Löslichkeits- oder Quellbarkeitseigenschaften aufweisen.

Eine weitere bevorzugte Ausführungsform der Erfindung sieht vor, dass die Formulierung eine oder mehrere zusätzliche Zwischenschichten enthält, welche dazu geeignet sind, Interaktionen und/ oder Inkompatibilitäten zwischen den Schichten, zwischen denen sie angeordnet sind, oder zwischen der innersten Schicht und dem oder den Wirkstoffen oder dem oder den wirkstoffhaltigen Kernen zu verringern oder zu vermeiden. Zu diesem Zweck verwendet diese Ausführungsform der Erfindung beispielsweise geeignete Polymere und ggf. andere pharmazeutische Hilfsstoffe wie beispielsweise Weichmacher, Antiklebemittel, etc. Die Polymere können beispielsweise wasserlösliche Polymere wie HPMC oder PVP sein.

Diese Schicht oder Schichten können ähnlich realisiert sein, wie die bereits genannten Schichten zur beschleunigten Freisetzung, allerdings ohne die entsprechend aktiven Substanzen, welche beispielsweise den pH Wert der umgebenden Lösung modulieren oder besondere Quelleigenschaften aufweisen.

Eine weitere bevorzugte Ausführungsform der Erfindung sieht vor, diese Zwischenschichten, welche Inkompatibilitäten oder Interaktionen vermeiden sollen, zu kombinieren mit den freisetzungsbeschleunigenden Eigenschaften, indem sie ebenfalls entsprechend aktive Substanzen, welche beispielsweise den pH Wert der umgebenden Lösung modulieren oder besondere Quelleigenschaften aufweisen, enthalten.

Um Interaktionen oder Inkompatibilitäten zwischen dem oder den Wirkstoffen oder dem oder den wirkstoffhaltigen Kernen und den funktionalen Schichten zu vermeiden oder zu minimieren kann eine Zwischenschicht, oder gegebenenfalls auch mehrere davon, mit oder ohne einen oder mehrere freisetzungsbeschleunigende Inhaltsstoffe, auch direkt auf dem oder den Wirkstoffen oder dem oder den wirkstoffhaltigen Kernen aufgebracht werden, was eine weitere bevorzugte Ausführungsform der Erfindung vorsieht. Eine entsprechende Schicht kann z.B. auch verwendet werden, um bei Verwendung von Kapseln den Spalt zwischen den beiden Kapselhälften zu versiegeln.

Zusätzliche Zwischenschichten können auch Eigenschaften haben, die vom pH Wert des sie umgebenden Darminhaltes abhängig sind, beispielsweise zur Kompensation von pH-abhängigen Löslichkeitseigenschaften eines oder mehrerer Wirkstoffe. Vorzugsweise weisen solche zusätzlichen Zwischenschichten keine solchen pH-abhängigen Eigenschaften auf, wie sie für die eine oder mehreren weiteren Schichten beschrieben sind.

Die Materialien, bzw. Materialmischungen, die für die Beschichtungen, Dispersionen, Emulgierungen oder Verpressungen verwendet werden, können auch weitere Hilfsstoffe enthalten, beispielsweise die in einschlägigen Arzneibüchern (z.B. Ph. Eur., USP, JP) erwähnten Hilfsstoffe.

Geeignet sind unter anderem Weichmacher, Trennmittel, Aromastoffe, Netzmittel, Farbstoffe, Neutralisierungsmittel, Konservierungsstoffe, Porenbildner, Antioxidantien, Gleitmittel, Antiklebemittel, Süßungsmittel, etc. Beschreibungen dieser Stoffe können beispielsweise aus Lexikon der Hilfsstoffe für Pharmazie, Kosmetik und angrenzende Gebiete, Fiedler, H. P., entnommen werden.

Durch Verwendung solcher Techniken, bewirkt eine weitere bevorzugte Ausführungsform der Erfindung eine besonders schnelle Auflösung der Schichten, und so eine besonders schnelle Freisetzung des oder der Wirkstoffe nach der Passage der Ileozäkalklappe, vorzugsweise bereits zu mehr als 30% vor der ersten Flexur, bei entsprechender Einstellung der Schichtdicken bevorzugt mehr als 50% vor dieser. Besonders lösliche Wirkstoffe, oder Wirkstoffe, die auf oder in sprengmittelhaltigen Kernen auf- oder eingebracht sind, werden sogar zu mehr als 75% vor der ersten Flexur freigesetzt.

In einer weiteren bevorzugten Ausführungsform sieht die Erfindung vor, dass die weitere Schicht oder eine oder mehrere weitere Schichten realisiert werden, indem für deren Herstellung eine Mischung aus mehreren Materialien verwendet wird, von denen mindestens eines unterhalb des bestimmten unteren pH-Wertes der jeweiligen Schicht löslich oder permeabel ist, und mindestens ein anderes oberhalb des bestimmten oberen pH-Wertes.

Bei der Herstellung der Schicht werden diese Materialien effektiv parallel angeordnet, so dass ein löslich oder permeabel werden eines der Materialien eine ausreichende Löslichkeit oder Permeabilität der Schicht bewirkt.

Eine weitere bevorzugte Ausführungsform der Erfindung realisiert dies durch eine Mischung mehrerer Dispersionen, bei der die einzelnen Dispersionen jeweils Dispersionspartikel enthalten, die bestimmte Beständigkeiten gegenüber wässrigen Lösungen mit speziellen pH Werten aufweisen.

Beispielsweise werden 2 Dispersionen gemischt, bei denen die erste Dispersion Polymerpartikel enthält, die oberhalb eines bestimmten pH Wertes löslich sind (z.B. ein Poly(MMA-AA)-Copolymer), während die zweite Dispersion Polymerpartikel enthält, die unterhalb eines bestimmten pH Wertes löslich sind (z.B. ein Poly(MMA-DEAEMA)-Copolymer).

Eine mittels der gemischten Dispersion hergestellte Schicht ist sowohl oberhalb eines bestimmten oberen pH Wertes, als auch unterhalb eines bestimmten unteren pH-Wertes löslich, wobei sie im Bereich zwischen den beiden bestimmten pH Werten beständig ist (Einen schematischen Ausschnitt einer solchen Schicht zeigt Zeichnung 5, Schicht C2).

Beispielhafte Realisierungen stellten die Ausführungsbeispiele 9a und 9b dar. Die zugehörigen Diagramme in Zeichnung 18 zeigen die Beständigkeit der einzelnen Schichten gegenüber Lösungen mit bestimmten pH Werten.

In einer weiteren bevorzugten Ausführungsform der Erfindung kann dies beispielsweise durch Verwendung von Dispersion mit entsprechend großen dispergierten Teilchen wie z.B. Flocken erfolgen, welche statistisch so verteilt sind, dass sie sich zwar über die gesamte zu beschichtende Oberfläche mindestens einfach überlappen, so dass eine geschlossene Schicht entsteht, dass aber nicht über die gesamte Oberfläche eine Überlappung beider Materialien zustande kommt, sondern es mindestens an einem Teil der Schicht möglich ist, dass eine wässrige Lösung, deren pH-Wert es ihr ermöglicht, zumindest eines der Materialien zu lösen oder permeabel werden zu lassen, durch diese Schicht hindurch dringen kann.

Zur Herstellung dieser Dispersionspartikel können beispielsweise die gleichen oder ähnliche Polymere und Copolymere verwendet werden, wie sie auch zur Herstellung von im Stand der Technik bekannten Protective Coatings oder Enteric Coatings benutzt werden. Auch ungepfropfte oder mit anionischen Molekülgruppen gepfropfte Chitosan-Polymere können verwendet werden. Es ist also möglich, die Erfindung auszuführen, ohne dass Acrylate benötigt werden. Auch ohne die Verwendung von Phtalaten ist die Erfindung realisierbar.

Einige Beispiele zur Herstellung entsprechender Polymere und darauf basierender Dispersionen, sowie Möglichkeiten zur Veränderung einzelner Parameter können z.B. aus WO 2009/016258A1 entnommen werden.

Auch in "Entwicklung eines lösungsmittelfreien Befilmungsverfahrens für feste Arzneiformen", Engelmann S., Dissertation, Freiburg im Breisgau, Albert-Ludwigs-Universität, 2004, werden entsprechende Verfahren zur Polymerherstellung und Modifizierung ihrer Eigenschaften beschrieben. Ebenso in WO/2005/115352.

Weitere Möglichkeiten zur Herstellung solcher Polymere werden auch beschrieben in EP1496870B1, EP0704207A2, EP0704207A2.

Bei der Herstellung von Beschichtungslösungen mit verschiedenen Polymerpartikeln, z.B. beim Mischen von 2 oder mehr Dispersionen mit Polymerpartikeln unterschiedlicher Löslichkeitseigenschaften, bei denen sowohl Polymere mit anionischen, als auch Polymere mit kationischen Eigenschaften verwendet werden, kann es unter Umständen zu verfrühter Verklumpung der Polymerpartikel oder zur partiellen oder vollständigen Quellung oder Lösung von Polymerpartikeln kommen.

Dies verhindert oder minimiert eine weitere bevorzugte Ausführungsform der Erfindung, bei der die Dispersionen vor dem Vermischen durch Zugabe von Substanzen, welche geeignet sind, den pH Wert der Dispersionen zu verändern, beispielsweise Säuren oder Basen, wie z.B. Zitronensäure, Salzsäure, Ammoniak, NaOH, KOH, Ammoniumhydroxyd oder organische Basen, wie z. B. Triethanolamin etc., auf einen annähernd gleichen pH-Wert gebracht werden.

Dieses ist bereits in den Beispielen 9a und 9b realisiert.

Vorzugsweise wird der pH-Wert der einzelnen Dispersionen möglichst nicht so weit verändert, dass die Dispersionspartikel löslich werden, bzw. nur so weit, dass keine oder nur eine akzeptable Quellung erfolgt. Dies kann auch in einem gewissen Abstand der pH Werte der zu vermischenden Dispersionen resultieren, wobei es durch simple Löslichkeitsversuche möglich ist, die optimalen pH Werte zu finden.

Die zu mischenden Dispersionen werden in einer weiteren bevorzugten Ausführungsform der Erfindung in ihrem pH-Wert, sowie in ihrer Pufferkapazität so eingestellt, dass der sich nach der Mischung ergebende pH Wert möglichst zwischen den isoelektrischen Punkten der verwendeten Polymere befindet, vorzugsweise möglichst mittig dazwischen. Weisen einzelne verwendete Polymere bei diesem pH Wert eine signifikant höhere Löslichkeit oder Quellbarkeit auf, als andere, so kann der pH Wert der Mischung auch so eingestellt werden, dass alle verwendeten Polymere möglichst gleich beständig sind.

Die Einstellung des pH Wertes der Mischung kann anstatt oder zusätzlich zur Einstellung der zu mischenden Dispersionen auch durch Zugabe entsprechender Substanzen bei oder nach der Mischung erfolgen.

Sind nicht alle verwendeten Polymere bei dem in der Mischung realisierten pH Wert beständig, kann es vorteilhaft sein, die einzelnen Dispersionen erst kurz vor der Verwendung zur Herstellung der Beschichtung zu mischen, so dass möglichst keine vollständige Lösung der Partikel, bzw. nur eine akzeptable Quellung erfolgt. Dies stellt eine weitere bevorzugte Ausführungsform der Erfindung dar.

Sind alle zu verwendenden Polymere in einer Lösung dispergierbar, in der keines davon löslich oder über einen akzeptablen Wert hinaus quellbar ist, kann eine entsprechende Dispersion auch direkt aus vorgetrockneten Pulvern redispergierbarer Polymerpartikel hergestellt werden, ohne vorher zwei oder mehr unterschiedliche Dispersionen herzustellen und dann zu mischen.

Je nach den verwendeten Polymeren kann es schwierig bis unmöglich sein, eine gemischte Dispersion herzustellen, z.B. wenn kein pH-Wert gefunden werden kann, bei dem alle verwendeten Polymere zumindest für die Zeit zwischen Mischung der Dispersionen und Antrocknung auf dem zu beschichtenden Substrat ausreichend beständig sind.

Eine weitere bevorzugte Ausführungsform der Erfindung sieht deshalb vor, dass zwei Dispersionen bereitgestellt und aus verschiedenen Düsen gleichzeitig auf die zu beschichtenden Substrate aufgesprüht werden.

In einer weiteren bevorzugten Ausführungsform der Erfindung werden die verschiedenen Dispersionen aus separaten Düsen versprüht, so dass sie erst in der Beschichtungsanlage, vorzugsweise erst kurz vor dem Kontakt und besonders bevorzugt erst beim Kontakt mit den zu beschichteten Partikeln, wie z.B. Tabletten, Pellets, Kapseln oder ähnlichem, aufeinander treffen. Dies verhindert eine vorzeitige Auflösung oder Verklumpung der Dispersionsteilchen. Besonders gut eignen sich für solche Verarbeitungsvorgänge Dragierpfannen oder Wirbelschichtanlagen, wie sie auch in den Ausführungsbeispielen verwendet werden. Die Ausrüstung von Beschichtungsanlagen mit separaten Sprühvorrichtungen ist im Stand der Technik mehrfach beschrieben.

Eine weitere bevorzugte Ausführungsform der Erfindung realisiert die geometrisch parallele Anordnung der verschiedenen Materialien zur Herstellung von Schichten, welche nur in einem Bereich zwischen einem bestimmten unteren pH Wert und einem bestimmten oberen pH Wert beständig sind, dadurch, dass die zu beschichtenden Partikel, wie z.B. Kapseln, Pellets, Tabletten oder ähnliches, für eine bestimmte Zeit nicht vollständig eingetaucht werden in Lösungen, Dispersionen, Emulsionen oder sonstige flüssige oder fluidisierte Medien, welche die Materialien beinhalten, bzw. zumindest teilweise aus ihnen bestehen. Während des Eintauchens bleibt ein Teil des Mediums an den Partikeln haften und bildet nach dem Entnehmen des oder der Partikel aus dem Medium eine entsprechende Schicht, vorzugsweise durch Trocknung, Polymerisation, Gelierung, Aushärtung oder andere Arten der Filmbildung. Nach Abschluss der Filmbildung des ersten zu verwendenden Materials werden nacheinander alle zu verwendenden Materialien benutzt. Das teilweise Eintauchen erfolgt bei verschiedenen Materialien jeweils mit veränderter geometrischer Orientierung der Partikel und erst nachdem die Schichtbildung nach dem jeweils vorhergehenden Eintauchvorgang zumindest weitgehend abgeschlossen ist, so dass die einzelnen Materialien nicht wechselwirken, bzw. zumindest nicht so weit wechselwirken, dass die Löslichkeits- oder Quellbarkeitseigenschaften signifikant verändert werden würden.

Vorzugsweise stoßen die Bereiche, in denen verschiedene Materialien oder Materialmischungen zur Anwendung kommen, direkt aneinander. Mehr bevorzugt überlappen sich die entsprechenden Bereiche, besonders bevorzugt um mehr als 10% ihrer Fläche, speziell bevorzugt um mehr als 25% ihrer Fläche. Eine mögliche Ausführungsform zeigt Ausführungsbeispiel 14, eine weitere zeigt Ausführungsbeispiel 21.

Eine weitere bevorzugte Ausführungsform der Erfindung realisiert die geometrisch parallele Anordnung der verschiedenen Materialien zur Herstellung von Schichten, welche nur in einem Bereich zwischen einem bestimmten unteren pH Wert und einem bestimmten oberen pH Wert beständig sind, durch die Verwendung von unterschiedlich beschichteten Kapselhälften, oder von Kapselhälften, welche aus unterschiedlichen Materialien hergestellt sind. Beispielsweise wird der Kapselboden (teilweise auch als Kapselkörper bezeichnet) mit einem Material beschichtet, oder aus einem solchen Material hergestellt, welches bei Werten unterhalb eines bestimmten unteren pH Wertes löslich oder permeabel ist. Der Kapseldeckel wird mit einem Material beschichtet, oder aus einem solchen Material hergestellt, welches bei Werten oberhalb eines bestimmten oberen pH Wertes löslich oder permeabel ist. Vorzugsweise wird nach dem Befüllen und Zusammenstecken der beiden Kapselhälften eine Versiegelung des entstehenden Spaltes durchgeführt, wozu dem Fachmann entsprechende Verfahren aus dem Stand der Technik bekannt sind (z.B. Dispensen eines nicht wasserlöslichen Polymers in Form einer Lösung oder Dispersion).

Zur Beschichtung der Kapselhälften können verschiedene bekannte Verfahren eingesetzt werden, beispielsweise das Dip-Coating, wie auch in US 12/845994 oder US 2011/0033530 A1 beschrieben, oder auch Spray-Coating oder Powder-Layering-Verfahren.

Besonders beim Spray-Coating können auch mehrere unterschiedliche Materialien oder Materialmischungen effektiv parallel auf einer Kapselhälfte aufgebracht werden, z.B. indem bestimmte Bereiche durch Schattenmasken vor dem Kontakt mit dem Sprühnebel geschützt werden, oder die unterschiedlichen Materialien aus verschiedenen Richtungen aufgesprüht werden. Hierdurch ist es möglich, eine besonders schnelle und möglichst gleichmäßige Auflösung der Kapsel zu erreichen.

Die Verwendung von Schattenmasken, welche zwischen der Sprühvorrichtung und der zu beschichtenden Oberfläche angeordnet werden, und den Sprühnebel nur an den Stellen auf die Oberfläche treffen lassen, an denen sie entsprechende Öffnungen aufweisen, ist nicht auf die Beschichtung von Kapseln beschränkt, sondern kann auch bei anderen Verabreichungsformen, wie beispielsweise Tabletten, Pellets, Dragees usw. erfolgen.

Vorzugsweise bestehen die Schattenmasken aus Stahlblech, besonders bevorzugt aus nicht rostendem Stahlblech. Die Schattenmasken können auf ihrer der Sprühvorrichtung zugewandten Seite mit einem Trennmittel beschichtet werden, das eine nachfolgende Reinigung von dem über den abzuschattenden Bereichen der Oberfläche abgefangenen Sprühnebel erleichtert. Vorzugsweise sind die Schattenmasken der Geometrie der Verabreichungsform angepasst, so dass über einen möglichst großen Teil der Oberfläche ein möglichst geringer Abstand zu der Oberfläche ermöglicht wird, um ein Hintersprühen der abgeschatteten Bereiche so weit wie möglich zu vermeiden.

Durch Verwendung unterschiedlicher Kapselgrößen ist es möglich, mehrere solcher Schichten sequenziell anzuordnen, indem jeweils eine kleinere Kapsel, welche aus entsprechend beschichteten oder hergestellten Kapselhälften zusammengesetzt ist, in eine größere Kapsel eingebracht wird, deren Kapselhälften entsprechend den Anforderungen an die entsprechende Schicht löslich sind.

Eine beispielhafte Ausführung zeigt Ausführungsbeispiel 19.

Wird eine besonders schnelle Freisetzung gewünscht, so kann vor dem Einbringen der Kapseln in die jeweils größere Kapsel ein Disintegrant oder Super-Disintegrant, beispielsweise Explotab, in die Kapselböden und/oder Kapseldeckel eingebracht werden. Dies bewirkt auch eine bessere Freilegung der eingebrachten Kapsel, wenn sich bei der umhüllenden Kapsel nur eine Kapselhälfte auflöst.

Bei der Mischung von unterschiedlichen Dispersionen oder bei der Beschichtung mit unterschiedlichen Dispersionen aus mehreren Düsen oder Mehrstoffdüsen, ist es oft vorteilhaft, einen oder mehrere Stabilisatoren und/oder Emulgatoren zuzusetzen, bzw. die Menge gegenüber des oder der bei einer einzelnen Dispersion verwendeten Stabilisatoren und/oder Emulgatoren zu erhöhen. Eine entsprechende weitere bevorzugte Ausführungsform der Erfindung zeigt Ausführungsbeispiel 9f.

Bei vielen Ausführungsformen der Erfindung können statt Dispersionen grundsätzlich auch Lösungen von entsprechenden Polymeren verwendet werden, beispielsweise in organischen Lösungsmitteln gelöst oder als kolloidale Lösungen. Beispielsweise Eudragit E in einer kolloidalen Lösung mit Stearinsäure, weshalb die Erfindung dies als weitere bevorzugte Ausführungsformen vorsieht.

Eine weitere Möglichkeit, Materialien zu verarbeiten, die in gelöstem, dispergierten oder feuchten Zustand nicht gemeinsam verarbeitet werden können, besteht in der Verarbeitung dieser Materialien in trockenem oder weitgehend trockenem Zustand. In einer weiteren bevorzugten Ausführungsform sieht die Erfindung deshalb vor, eine oder mehrere Schichten aus verschiedenen Partikeln, vorzugsweise Polymerpartikel oder Copolymerpartikel, unterschiedlicher Löslichkeitseigenschaften herzustellen, indem ein Trockenbefilmungsverfahren angewendet wird. Bei einem Trockenbefilmungsverfahren werden die Polymerpartikel ohne Zugabe von Lösungsmitteln vermischt, und sodann auf das zu beschichtende Substrat aufgebracht. Während des Aufbringens der Partikel wird ebenfalls ein Weichmacher zugegeben (bei einem Sprühverfahren z.B. aus einer separaten Sprühdüse, teilweise auch zeitlich versetzt zur Aufbringung der Partikel), welcher ein Zusammenlagern und/ oder Anhaften der Partikel ermöglicht, so dass eine Filmbildung einsetzen kann. Gegebenenfalls werden auch geringe Mengen Lösungsmittel eingesetzt, welche die Polymerpartikel aufgrund der geringen Menge allerdings nicht auflösen können, zumindest nicht vollständig. Unter Umständen ist für eine ausreichende Filmbildung ein thermischer Nachbehandlungsprozess (Curing) erforderlich, ggf. nach Zugabe geringer Mengen von Lösungsmittel (beispielsweise Wasser, Isopropanol, etc).

Beispielhafte Realisierungen stellten die Ausführungsbeispiele 9c und 9d dar. Die zugehörigen Diagramme in den Zeichnungen 19 und 20 zeigen die Beständigkeit der einzelnen Schichten gegenüber Lösungen mit bestimmten pH Werten.

Entsprechend verwendbare Verfahren sind beispielsweise auch in "Dry coating: an innovative enteric coating method using a cellulose derivative", Sakae Obara, European Journal of Pharmaceutics and Biopharmaceutics 47 (1999), beschrieben. In dieser Veröffentlichung sind z.B. auch dem Fachmann bekannte Verfahren beschrieben, die eine Findung der optimalen Prozessparameter und Hilfsstoffe ermöglichen, auch wenn Polymere verwendet werden, die auf anderen Monomeren basieren. Besonders vorteilhaft ist es, wenn bei einem solchen Verfahren zumindest eines der verwendeten Polymere schon bei geringen Temperaturen erweicht und/oder bei geringen Wassermengen plastisch wird, wie beispielsweise Hydroxypropyl-Methylcellulose-Acetat-Succinat (HPMCAS). Eine weitere bevorzugte Ausführungsform der Erfindung verwendet ein darauf basierendes Polymer. Ein weiteres Beispiel für ein Polymer mit entsprechend geringer Glasübergangstemperatur ist Eudragit E (Evonik, Darmstadt).

Acrylatbasierte Polymermischungen, die mit dem beschriebenen Verfahren nicht sicher verarbeitet werden können, oder andere schwierig in dispergiertem Zustand mischbare Polymere, können beispielsweise mit dem Trockenbefilmungsverfahren verarbeitet werden, das in "Einfluss von Formulierungsparametern auf den Powder Layering und Dry Coating Prozess im Rotorgranulator", Jeannine Ebert, Saarbrücken, beschrieben ist.

Statt Pulver aus einem einzelnen Polymer werden verschiedene Polymere in Pulverform gemischt und sodann mit den dem Fachmann bekannten Trockenbefilmungsprozessen verarbeitet. Die entsprechenden Veröffentlichungen geben auch Anleitung zur Modifizierung der Prozesse um gewünschte Eigenschaften zu erreichen.

Auch in "Entwicklung eines lösungsmittelfreien Befilmungsverfahrens für feste Arzneiformen", Engelmann S., Dissertation, Freiburg im Breisgau, Albert-Ludwigs-Universität, 2004, werden entsprechende Verfahren zur Trockenbefilmung beschrieben. Weitere Möglichkeiten der Realisierung eines Trockenbefilmungsprozesses sind beschrieben in "PARTICLE COATING USING DRY POWDER TECHNOLOGY", L. Bilancetti, PARTEC 2007.

Pearnchob und Bodmeier beschrieben 2003 in "Dry polymer powder coating and comparison with conventional liquid-based coatings for Eudragit® RS, ethylcellulose and shellac", European Journal of Pharmaceutics and Biopharmaceutics, Trockenbefilmungsverfahren, wobei der Weichmachermischung geringe Anteile von Wasser zugesetzt sind.

Diese geringen Anteile von Wasser wirken sich aber nicht nachteilig auf die Verwendung von als Pulver gemischten Polymeren mit verschiedenen Lösungseigenschaften oder ionischen Eigenschaften aus, da der Kontakt mit dem Wasser als Lösungsmittel erst direkt beim Beschichtungsprozess zustande kommt.

Auch die in "A novel powder coating process for attaining taste masking and moisture protective films applied to tablets", Matteo Cerea et al., International Journal of Pharmaceutics 279 (2004), beschriebenen Trockenbefilmungsverfahren können nach Austausch der verwendeten Polymere und ggf. Änderung von Prozessparametern wie Verarbeitungstemperatur, Weichmacheranteil etc. verwendet werden.

Die genannten Publikationen geben ausreichend Anleitung für den Fachmann, die Prozesse an die jeweiligen zu verwendenden Polymere, Copolymere oder andere filmbildende Materialien anzupassen, und ggf. durch einfache Versuche zu optimieren.

Eine weitere bevorzugte Ausführungsform der Erfindung realisiert die parallele Anordnung der Materialien durch eine geometrisch definierte Aufbringung der Materialien, bzw. von Beschichtungen mit entsprechenden Eigenschaften. Hierzu werden die Beschichtungsmaterialien nacheinander oder aus verschiedenen Richtungen auf die ggf. schon vorbeschichteten Einheiten, wie z.B. Partikel, Pulver, Pellets, Tabletten, Kapseln o.ä. aufgesprüht oder auf eine andere Art und Weise aufgebracht, wobei sichergestellt wird, dass jeweils nicht die gesamte Oberfläche beschichtet wird, so dass die Oberfläche pro verwendeten Material mindestens einen Bereich aufweist, an dem nur dieses löslich oder permeabel werden muss, um die umgebende wässrige Lösung in Kontakt mit der nächsten weiter innen angeordneten Schicht kommen zu lassen.

Dies wird beispielsweise durch gezieltes besprühen mit Lösungen oder Dispersionen der verschiedenen Materialien aus verschiedenen Richtungen realisiert, oder durch mehrere aufeinander folgende Beschichtungsvorgänge, zwischen denen das Beschichtungsgut oder die Beschichtungsvorrichtung gedreht, gewendet oder auf andere Art und Weise in der geometrischen Anordnung verändert wird. (Zeichnung 6).

Beispielhafte Realisierungen dieser Ausführungsform stellten die Ausführungsbeispiele 10 und 11 dar. Die zugehörigen Diagramme in den Zeichnungen 21 und 22 zeigen die Beständigkeit der einzelnen Schichten gegenüber Lösungen mit bestimmten pH Werten.

Die effektiv parallele Anordnung der Materialien kann auch dadurch realisiert werden, dass Gemische aus Lösungen und Dispersionen, bzw. Emulsionen oder auch Pulvern verwendet werden, sei es dass diese Mischung vor einem Aufbringungsschritt, dabei, oder danach stattfindet, weshalb eine weitere bevorzugte Ausführungsform der Erfindung dies vorsieht.

Beispielsweise können Materialien, welche nicht in organischen Lösungsmitteln löslich sind, in diesen oder einem von diesen dispergiert werden, während andere Materialien bereits darin gelöst sind, oder nach der Herstellung der Dispersion darin gelöst werden. Die Dispersion kann auch in einem wässrigen Medium hergestellt und danach in ein organisches Lösungsmittel oder Lösungsmittelgemisch überführt werden.

Beispielhaft sei die Herstellung einer Chitosan-Dispersion aus in verdünnter Essigsäure gelöstem Chitosan genannt (beispielsweise durch Hinzufügen einer Natrium-Tri-Ployphosphat-Lösung nach Zugabe eines Stabilisators oder Emulgators, wie beispielsweise Polysorbat 80), welche anschließend in eine Mischung aus Aceton und Isopropanol überführt wird, in der Eudragit S 100 gelöst ist.

Anstatt das Gemisch vor dem Auftrag herzustellen, kann auch eine Mischung direkt bei dem Prozess erfolgen, beispielsweise durch Benutzung mehrere Sprühdüsen oder von Dreistoffdüsen, wodurch die Menge des benötigten Emulgators reduziert werden kann.

Auch können die Materialien in unterschiedlichen Aggregatzuständen verarbeitet werden. So kann eines der Materialien oder Materialgemische als Pulver vorliegen, während das andere Material oder Materialgemisch als Lösung oder Dispersion in den Prozess eingebracht wird.

Ausführungsbeispiel 16 zeigt eine mögliche Realisierung.

Beispiele und Hinweise zu verwendbaren Prozessen und Prozessparametern sind unter anderem auch in "Multiparticulate Chitosan-Dispersed System for Drug Delivery", SHIMONO et al., Chem. Pharm. Bull. 51(6) 620-624 (2003), "Chitosan dispersed system for colon-specific drug delivery", SHIMONO et al., International Journal of Pharmaceutics 245 (2002) 45□/54 oder US 7604820 B1 zu finden. Die verwendeten nicht wasserlöslichen Eudragit-Polymere können durch pH Wert abhängig lösliche Polymere ausgetauscht werden. Gegebenenfalls sind die Mengenverhältnisse anzupassen um gewünschte Viskositäten einstellen zu können und weitere Hilfsstoffe wie Emulgatoren oder Trennmittel hinzuzufügen, z.B. um das Verkleben der Partikel zu vermeiden, was für den Fachmann alltägliche Aufgaben darstellen.

Auch können unterschiedliche funktionale Partikel eingesetzt werden, beispielsweise Pulver aus anionischen und kationischen Polymeren, welche in einer Schicht eingebettet sind, deren hauptsächlich filmbildendes Polymer dann auch nicht wasserlöslich sein kann. Die Prozesse aus den vorgenannten Publikationen können beispielsweise entsprechend so abgewandelt werden, dass das Chitosanpulver durch eine solche Mischung aus mehreren Polymerpulvern ersetzt wird. Beispielsweise wird die Menge des verwendeten Chitosanpulvers halbiert, und ein HPMCAS-Pulver oder ein Pulver aus Eudragit S hinzu gegeben, so dass die ursprüngliche Pulvermenge wieder erreicht wird. Das HPMCAS-Pulver oder das Eudragit S Pulver wird auf die gleiche Partikelgröße (z.B. ca. 95µm Durchmesser) eingestellt.

Eine weitere beispielhafte Realisierung ist in Ausführungsbeispiel 15 gezeigt.

Solche Prozesse, die feste und mehr oder weniger flüssige Komponenten in einem Prozess zur Beschichtung verwenden, werden unter anderem auch als "Powder Layering" bezeichnet, und sind im Stand der Technik ausreichend beschrieben (z.B. in "Einfluss von Formulierungsparametern auf den Powder Layering und Dry Coating Prozess im Rotorgranulator", Jeannine Ebert, Saarbrücken). Dem Fachmann sind daher auch diverse Möglichkeiten bekannt, durch welche Parameteränderungen er die gewünschten Schichteigenschaften einstellen kann.

Ob die mehr oder weniger flüssigen Komponenten nur als Binder zum Einsatz kommen (z.B. in Ausführungsbeispiel 15), oder selbst für funktionale Merkmale der erzielten Schichten verantwortlich sind (z.B. in Ausführungsbeispiel 16), ist für den Beschichtungsprozess an sich weitgehend unerheblich.

Je nach Löslichkeitseigenschaften der verwendeten Pulver, ist es besonders bei Powder-Layering-Verfahren vorteilhaft, organische Binder- oder Polymerlösungen durch entsprechende wässrige Dispersionen auszutauschen.

Powder Layering Verfahren können auch eingesetzt werden, wenn Materialien zur Verfügung stehen, die sowohl oberhalb eines bestimmten oberen pH Wertes, als auch unterhalb eines bestimmten unteren pH Wertes löslich oder permeabel sind. Da dann keine effektiv parallele Anordnung verschiedener funktionaler Materialien erforderlich ist, können bestimmte Schichten der Formulierung entsprechend den ECDS-Pellets oder den Kapseln, beschrieben in "Multiparticulate Chitosan-Dispersed System for Drug Delivery" oder "Chitosan dispersed system for colon-specific drug delivery", hergestellt werden, wobei aber das dort verwendete Chitosan Pulver ausgetauscht wird, durch ein Pulver aus einem oder mehreren der vorgenannten Materialien.

Eine beispielhafte Realisierung zeigt Ausführungsbeispiel 36.

Eine weitere bevorzugte Ausführungsform der Erfindung sieht vor, dass solche Materialien zusätzlich empfindlich gegenüber Enzymen sind, die im Dickdarm von Mikroorganismen gebildet werden können. Besonders vorteilhaft an dieser Ausführungsform ist, dass ein Material sowohl bei Unterschreitung eines bestimmten unteren pH Wertes, als auch bei Überschreitung eines bestimmten oberen pH Wertes, sowie ebenfalls bei Vorhandensein einer ausreichenden Menge an im Dickdarm verfügbaren Enzymen löslich, permeabel oder abgebaut werden kann, und somit vorzugsweise als alleiniges die Freisetzung kontrollierendes Material einer Schicht dienen kann. Das im vorgenannten Ausführungsbeispiel verwendete Material erfüllt beispielsweise alle diese Eigenschaften.

In einer weiteren bevorzugten Ausführungsform der Erfindung ist vorgesehen, dass ein solches Material zusätzlich filmbildende Eigenschaften hat, so dass auf ein zusätzliches filmbildendes Material bei der Herstellung einer Schicht verzichtet werden kann.

Eine weitere bevorzugte Ausführungsform der Erfindung sieht vor, die parallele Anordnung durch Herstellung einer Matrix zu realisieren, bei der Partikel mit unterschiedlichen Löslichkeits- bzw. Permeabilitätseigenschaften mit dem zu beschichtenden oder einzuschließenden Partikeln intensiv vermischt und dann verpresst werden. Beispielsweise werden wirkstoffhaltige und vorbeschichtete Mikropellets vermischt mit mindestens zwei verschiedenen Pulvern, wobei mindestens ein Pulver aus einem Polymer oder Copolymer hergestellt ist, das unterhalb eines bestimmten unteren pH-Wertes löslich oder permeabel wird, und mindestens ein anderes Pulver aus einem Polymer oder Copolymer hergestellt ist, das oberhalb eines bestimmten oberen pH-Wertes löslich oder permeabel wird. Durch die intensive Vermischung sind die verschiedenen Polymerpartikel gleichmäßig verteilt, so dass bei Eintritt der Löslichkeit oder Permeabilität von einer Sorte Pulver die gesamte Matrix in ihrer Struktur geschwächt und aufgelöst, bzw. permeabel wird.

Beispiel 13 beschreibt eine entsprechende Realisierungsmöglichkeit.

Vor dem Vermischen und Verpressen können auch noch Hilfsstoffe zugesetzt werden, vorzugsweise solche, die der Fachmann üblicherweise bei der Tablettenpressung einsetzt.

Zwei mögliche Quellen für fehlerhaftes Freisetzungsverhalten bei der Verwendung von paralleler Anordnung verschiedener Materialien sind:
1. Die unbeabsichtigte vollständige Beschichtung mit einem Material, also dass kein Weg für eine Flüssigkeit durch die Schicht möglich ist, welche nur das andere Material löslich oder permeabel macht, oder
2. eine nicht vollständig geschlossene Schicht, so dass jegliche Flüssigkeit in Kontakt mit der darunter liegenden Schicht kommen kann. Bei relativ geringer Vorkommensrate ist ersterer Fehler nicht dramatisch, da so nur eine geringe Dosisreduktion eintritt, falls eine multipartikuläre Verabreichungsform gewählt wird (z.B. Kapsel mit beschichten Pellets). Zweitgenannter Fehler kann allerdings zu unbeabsichtigter verfrühter Freisetzung und somit eventuell systemischer Exposition führen.

Dergestalt fehlerhaft beschichtete Einheiten können allerdings aussortiert werden. Hierzu sieht die Erfindung in einer weiteren bevorzugten Ausführungsform vor, die Einheiten nach dem schichterzeugenden Prozessschritt, welcher eine Beschichtung, Verpressung, Dispersion oder Emulgierung sein kann, einer wässrigen Lösung auszusetzen, gegenüber welcher die erzeugte Schicht beständig ist, falls sie korrekt ausgebildet wurde, gegenüber der aber die darunter liegende Schicht löslich oder permeabel ist, so dass entsprechend fehlerhaft beschichtete Einheiten aufgelöst, bzw. deren Wirkstoffe freigesetzt werden und abgetrennt werden können, gegebenenfalls sind mehrere solche Schritte erforderlich, mit verschiedenen Lösungen, welche die korrekt erzeugte Schicht nicht lösen oder permeabel werden lassen, jedoch gegebenenfalls nacheinander alle darunter liegenden Schichten.

Beispielhafte Realisierungen für diese Ausführungsform der Erfindung stellen die Ausführungsbeispiele 8a und 8b dar. Die zugehörigen Diagramme in Zeichnung 17 zeigen die Beständigkeit der einzelnen Schichten gegenüber Lösungen mit bestimmten pH Werten.

Besonders bei der Realisierung der Schichten zwischen der inneren Schicht und dem oder den Wirkstoffen oder dem oder den wirkstoffhaltigen Kernen oder zwischen der inneren und innersten Schicht hat es sich als vorteilhaft erwiesen, zwischen die einzelnen pH-abhängigen Schichten zusätzliche Zwischenschichten anzuordnen, welche generell löslich in wässrigen Medien sind, um auch in den Fällen, in denen starke Überlappungen mit den unterschiedlichen Materialien gegeben sind, und deshalb eventuell nur kleine Bereiche der Schicht permeabel oder löslich werden, die wässrige Lösung in guten Kontakt zur nächst inneren Schicht kommen zu lassen.

Besonders vorteilhaft ist es, wenn diese Schicht eine oder mehrere chemische Verbindungen enthält, die bei Kontakt mit Wasser oder wässrigen Lösungen eine starke Volumenzunahme erfahren, z.B. Sprengmittel, von denen dem Fachmann mehrere verschiedene bekannt sind, beispielsweise (Croscarmellose, Crospovidon, Polyvinylpyrrolidon (z.B. Kollidon), Natrium-Stärke-Glykolat (z.B. Explotab®, Vivastar®)). Entsprechende Schichten und deren Herstellung sind dem Fachmann bekannt, beispielsweise aus EP0210540.

Durch die starke Volumenzunahme wird die darüber liegende Schicht abgesprengt, sobald Wasser oder eine wässrige Lösung durch einen löslich oder permeabel gewordenen Teil dieser darüber liegenden Schicht dringt, womit ihr die nachfolgende Schicht möglichst großflächig ausgesetzt ist (Beispiel 12).

Solche Schichten enthalten bevorzugt mehr als 3% Sprengmittel, mehr bevorzugt mehr als 6% Sprengmittel, noch mehr bevorzugt mehr als 15% Sprengmittel und besonders bevorzugt mehr als 25% Sprengmittel.

Da die Materialien, aus denen die verschiedenen Schichten aufgebaut sind, bzw. aus denen die Matrizen oder Umhüllungen hergestellt werden, unterschiedliche Permeabilitäten, Auflösungsgeschwindigkeiten, Quellfähigkeiten und andere Eigenschaften aufweisen, ist es vorteilhaft, die verschiedenen Schichten in unterschiedlicher Schichtdicke beziehungsweise unterschiedlichen Flächengewicht (g/cm²) oder unterschiedlicher Gesamtgewichtszunahme (im Stand der Technik teilweise auch als total weight gain (TWG) bezeichnet) auszuführen, bzw. die Matrizen aus unterschiedlich großen Partikeln aufzubauen, weshalb eine weitere bevorzugte Ausführungsform der Erfindung dies vorsieht.

Viele Materialien, welche bevorzugt unterhalb eines bestimmten pH Wertes permeabel oder löslich werden, haben auch oberhalb dieses pH Wertes gewisse Quellfähigkeiten, was eine gewisse unerwünschte Permeabilität mit sich bringen kann, welcher durch eine größere Auftragsmenge bei der Beschichtung oder durch die Verwendung größerer Partikel beim Verpressen entgegengewirkt werden kann.

Eine weitere bevorzugte Ausführungsform der Erfindung sieht deshalb vor, dass bevorzugt im sauren Bereich lösliche und/oder besonders im sauren Bereich erhöht permeable Materialien oder Materialmischungen, welche diese umfassen, in größerer Schichtstärke und/oder in Form größerer Partikel eingesetzt werden, als solche, die bevorzugt im neutralen oder basischen bereich löslich oder erhöht permeabel sind.

Bei Verwendung von Materialgemischen, die sowohl eher säurelösliche, als auch eher im basischen oder neutralen Bereich lösliche Materialien enthalten, sei es als Mischung von Lösungen, Dispersionen, Emulsionen, Pulvern, als nacheinander aufgebrachte, effektiv parallel angeordnete Schichten oder sonstiger beschriebener Ausführungen, sieht eine weitere bevorzugte Ausführungsform der Erfindung vor, dass die Gewichts- und/oder Volumenanteile, die Partikelgröße, die Schichtdicken, die Art und Menge der verwendeten Hilfsstoffe und anderer die Eigenschaften beeinflussender Parameter unterschiedlich eingestellt werden, so dass Auflösungsverhalten, Permeabilitätsverhalten, Freisetzungsverhalten und Beständigkeit unterhalb und oberhalb der entsprechenden pH Schwellwerte wunschgemäß sind.

Beispielsweise ist es vorteilhaft, den Anteil des funktionellen Polymers einer säurelöslichen chitosanhaltigen Teilschicht geringer einzustellen, als den der HPMCAS-haltigen Teilschicht, die im neutralen pH Bereich löslich sein soll, da bei gleichem Masseanteil die chitosanhaltige Teilschicht schneller quellen und permeabel werden würde, was unerwünscht sein kann.

Ein weiteres Beispiel ist eine geringere Schichtdicke einer Schicht aus einer Kombination von Chitosan und Ethylcellulose, ggf. zusammen mit anderen Hilfsstoffen, gegenüber einer Schicht, welche aus Eudragit FS 30D (ebenfalls ggf. mit anderen Hilfsstoffen, wie beispielsweise Triethylcitrat etc.) besteht. Durch den Anteil an Ethylcellulose, welche das Quellverhalten der Schicht im neutralen Bereich erwünschterweise reduziert, verlangsamt sich die Auflösungsgeschwindigkeit der Schicht im sauren Bereich, weshalb eine geringere Schichtdicke erforderlich sein kann, um die Auflösungsgeschwindigkeit im sauren Bereich, der Auflösungsgeschwindigkeit des Eudragit FS 30D im neutralen bis alkalischen Bereich anzupassen.

Die Verarbeitbarkeit von Materialien mit unterschiedlichen Eigenschaften, beispielsweise eher säurelösliche Materialien zusammen mit eher basenlöslichen Materialien, anionische Polymere oder Copolymere mit kationischen Polymeren oder Copolymeren etc., kann unter Umständen schwierig sein, wenn diese Materialien miteinander wechselwirken. Beispielsweise können anionische und kationische Polymere Interpolyelektrolytkomplexe (IPEC) bilden, welche unerwünschte Löslichkeitseigenschaften haben.

Dies kann einerseits durch die schon beschriebenen Methoden verringert werden, wie z.B. dem Zusatz von entsprechenden Emulgatoren, die Verarbeitung der Polymere und/oder Copolymere im ungelösten und nicht dispergierten Zustand, unter anderem bei den Trockenbefilmungstechniken (Dry Powder Coating), oder dadurch, dass die unterschiedlichen Materialien nur für eine kurze Zeit in gelöstem oder dispergierten Zustand in Kontakt kommen, wie z.B. beim Auftrag im Sprühverfahren aus separaten Düsen oder aus Dreistoffdüsen, oder wenn die Materialien in unterschiedlichen Aggregatzuständen in den Prozess eingebracht werden, wie z.B. bei der getrennten Zuführung als Lösung oder Dispersion und als Pulver in den Tablettencoater oder Granulator.

Andererseits kann die Verarbeitbarkeit, und auch die Funktionalität der Beschichtungen, auch dadurch verbessert werden, dass ein direkter Kontakt der unterschiedlichen Materialien verringert oder vermieden wird, indem die verwendeten Partikel aus unterschiedlichen Materialien, seien diese für die Herstellung von Dispersionen oder auch für Trockenbefilmungsprozesse oder zur Verpressung von Matrizen bestimmt, mit einer Schicht umhüllt sind oder werden, welche die Wechselwirkungen weitgehend unterbindet, weshalb eine weitere bevorzugte Ausführungsform der Erfindung dies vorsieht.

Dies kann beispielsweise eine dünne Schicht aus einem nicht wasserlöslichen Polymer sein, das eine ausreichende Permeabilität aufweist, so dass die für den pH Wert im Darminhalt verantwortlichen Ionen mit dem funktionalen Polymer wechselwirken können, jedoch eine Wechselwirkung der Polymerketten der anderen verwendeten Materialien während dem Beschichtungsprozess nicht stattfindet.

Geeignete Polymere sind beispielsweise Ethylcellulose und Amylose. Weitere Polymere mit entsprechenden Eigenschaften sind dem Fachmann bekannt (z.B. üblicherweise für Retardbeschichtungen eingesetzte Polymere wie Eudragit RS, Eudragit RL, Eudragit NE, etc.).

Die Stärke der Beschichtung, die für diesen Zweck erforderlich ist, liegt üblicherweise unter der Beschichtungsstärke, welche für eine retardierte Wirkstofffreisetzung benutzt wird.

Bei Verwendung von Eudragit NE über der Funktionalen Eudragit S-Schicht von Carboxymethylcellulose-basierten (AcDiSol®) Microspheres oder Mikropellets hat sich eine Schichtdicke von 12µm als vorteilhaft herausgestellt.

Auch auf Partikel (Pulverpartikel, Microspheres, Mikropellets etc.), die für Trockenbefilmungsprozesse oder für die gemischte Verarbeitung von flüssigen (Dispersionen, Lösungen etc.) und festen Materialien oder Materialgemischen verwendet werden, können solche Schichten aufgebracht werden, um Interaktionen zwischen den unterschiedlichen Materialien soweit wie nötig zu unterbinden.

Zur Ermittlung der für den jeweiligen Anwendungsfall geeigneten Schichtstärke können Versuchschargen mit unterschiedlichen Schichtstärken hergestellt und deren Auflösungsverhalten getestet werden. Üblicherweise ist bei einer unzureichenden funktionalen Auflösung (z.B. im sauren oder leicht basischen pH Bereich) die Schichtstärke zu verringern, und bei einer erhöhten Löslichkeit im mittleren pH Bereich, welche auf die Bildung von IPEC hinweisen kann, oder bei verfrühter Koagulation während dem Beschichtungsprozess, die Schichtstärke zu erhöhen.

Eine solche Beschichtung der verwendeten Partikel kann auch zusammen mit den weiteren beschriebenen Methoden, wie z.B. den Trockenbefilmungsprozessen oder der gemischten Verwendung von festen (Pulvern) und verflüssigten (Lösungen, Dispersionen, Emulsionen) Materialien eingesetzt werden.

Beispielsweise können die in den Ausführungsbeispielen 8a, 8b, 9c, 9d, 13 oder 15 verwendeten Partikel vor ihrer Verwendung im jeweiligen Beschichtungsprozess mit einer solchen dünnen Polymerschicht überzogen werden. Vorzugsweise wird diese in einem Wirbelschichtverfahren aufgetragen, wobei auch andere im Stand der Technik bekannte Beschichtungsverfahren zur Anwendung kommen können, wie z.B. Emulsions-Verdampfungs-Verfahren oder Lösungs-Verdampfungs-Verfahren. Ausführungsbeispiel 16 zeigt eine mögliche Realisierungsvariante.

Da besonders nach dem Austritt aus dem Magen größere Schwankungen, bzw. Überschwinger des pH Wertes auftreten können, beispielsweise wenn durch Verdauungssäfte größere Mengen an Bikarbonat in den Darm eingebracht werden, kann es vorteilhaft sein, die erste Schicht, die nach der Passage des Magens löslich oder permeabel werden soll, so auszugestalten, dass dieser Vorgang länger als eine bestimmte Zeit andauert, bzw. um mindestens eine bestimmte Zeit verzögert wird, so dass darunter liegende Schichten erst dann in relevantem Ausmaß mit dem Darminhalt in Kontakt kommen, wenn die Schwankungen oder Überschwinger weitestgehend abgeklungen sind.

Es hat sich gezeigt, dass solche Überschwinger, die teilweise pH Werte von 6,5 oder mehr erreichen können, oft innerhalb von 20 Minuten abgeklungen sind, in einigen Fällen erst nach etwa 40 Minuten, in wenigen Fällen allerdings bis zu einer Stunde andauern können.

Eine weitere bevorzugte Ausführungsform der Erfindung sieht deshalb vor, dass die innere Schicht so ausgeführt ist, dass sie bei entsprechenden pH Werten oberhalb des pH Wertes, oberhalb dessen sie löslich oder permeabel werden kann (der bestimmte letzte pH Wert dieser Schicht), innerhalb eines Zeitraums zwischen 15 und 120 Minuten aufgelöst oder permeabel wird, bevorzugt zwischen 25 und 90 Minuten, mehr bevorzugt zwischen 40 und 70 Minuten oder besonders bevorzugt zwischen 50 und 60 Minuten.

Ein ähnliches Verhalten kann in einer weiteren vorgesehenen bevorzugten Ausführungsform der Erfindung auch dadurch erreicht werden, dass unterhalb der inneren Schicht eine zusätzliche Schicht oder Schichtenfolge angeordnet wird, welche sich weitgehend unabhängig vom pH Wert nach einer bestimmten Zeit auflöst öder permeabel wird, so dass die darunter liegende Schicht auch dann zeitverzögert freigelegt wird, bzw. dem Darminhalt ausgesetzt wird, wenn die innere Schicht sich nach der Passage des Magens schnell auflöst oder permeabel wird. Die angestrebten Zeiten gelten entsprechend auch bei dieser Ausführungsform.

Eine beispielhafte Ausführung dieser Ausführungsform ist eine Abwandlung des Ausführungsbeispiels 11, bei welcher die Beschichtung aus Eudragit L 100 in einer Stärke von 20mg/cm² ausgeführt wird.

Eine weitere beispielhafte Ausführung wandelt Ausführungsbeispiel 12 dahingehend ab, dass vor der Beschichtung mit Eudragit L 100 zuerst eine zusätzliche Schicht aus 85% HPMC (Pharmacoat 606), 5% Talkum und 10% Croscarmellose, und dann eine dünne Schicht aus Eudragit NE 30D aufgebracht wird. Nach Auflösung der Schicht aus Eudragit L diffundiert Wasser durch die permeable Schicht aus Eudragit NE in die darunter liegende sprengmittelhaltige Schicht, welche nach einer Stunde soweit quillt, dass die permeable Schicht abgesprengt, und die darunter liegende Schichtenfolge freigelegt wird. Entsprechende Schichtaufbauten sind dem Fachmann bekannt. Beispielsweise sei "Development of pulsatile multiparticulate drug delivery system coated with aqueous dispersion Aquacoat ECD.", Mohamad A, Dashevsky A., Int J Pharm. 2006 Aug 2;318(1-2):124-31. Epub 2006 Apr 3, genannt. Hier wird z.B. ein alternativer möglicher Aufbau einer solchen verzögernden Schichtfolge gezeigt.

Eine weitere bevorzugte Ausführungsform der Erfindung sieht vor, zumindest bei der Herstellung einer Schicht, ein Aufschmelzverfahren zu verwenden, sei es als Sprühverfahren wie in US2011250244 oder in einem Schmelzextrusionsverfahren. Bei einem Sprühverfahren wird das schicht- oder matrixbildende Material oder Materialgemisch über seine Schmelztemperatur erhitzt und dann ähnlich einer Lösung oder Dispersion verarbeitet.

Beim Schmelzextrusionsverfahren werden die wirkstoffhaltigen Kerne, ggf. bereits mit einer oder mehreren Vorbeschichtungen versehen, zusammen mit dem Material oder Materialgemisch erhitzt, so dass zumindest das schicht- oder matrixbildende Material oder Materialgemisch über seine Schmelztemperatur erhitzt wird, die Kerne umfließt und somit die Freisetzung entsprechend kontrolliert.

Das Ergebnis der Extrusion kann mit zusätzlichen Schichten versehen werden, wozu das Extrudat beispielsweise zu Pellets verarbeitet werden kann. Durch entsprechende Wahl der Materialien und deren Schmelzpunkte können auf diese Art auch Mehrfachbeschichtungen realisiert werden.

Eventuell vorgesehene weitere Beschichtungen der Extrudate können aber auch mit den anderen aus den aufgeführten Beispielen ableitbaren Prozessen weiterverarbeitet werden, sowie mit weiteren Prozessen, die dem Fachmann aus dem Stand der Technik bekannt sind.

Zur Herstellung der Beschichtungen, welche zur Realisierung der verschiedenen Ausführungsformen der Erfindung aufgetragen werden, können in weiteren bevorzugten Ausführungsformen auch Lösungs- und Verdampfungs-Prozesse eingesetzt werden (solvent evaporation methods, bzw. emulsion solvent evaporation methods), wie sie z.B. in "Polymer-coated microparticles for the sustained release of nitrofurantoin", Jita Liu et al., "Enteric Micro-Particles for Targeted Oral Drug Delivery", Annalisa Dalmoro et al., oder "Handbook of Pharmaceutical Controlled Release Technology", D.L. Wise, beschrieben werden. Vorzugsweise werden solche Prozesse eingesetzt, wenn besonders kleine Partikel zu beschichten sind (z.B. Microspheres), und/oder wenn die gewünschten Beschichtungsstärken besonders gering sind, beispielsweise bevorzugt bei Schichtdicken von unter 30µm, mehr bevorzugt bei Schichtdicken unter 20µm, besonders bevorzugt bei Schichtdicken zwischen 7 und 15µm.

Bei der Herstellung funktionaler Schichten oder Matrizen aus, oder mit Anteilen fester oder weitgehend fester Materialien, beispielsweise bei Dry Powder Coating Prozessen, bei Verwendung von Dispersionen oder bei Prozessen, welche feste und flüssige Materialien kombiniert einsetzen, wie z.B. auch in "Chitosan dispersed system for colon-specific drug delivery", oder auch bei Verwendung von Dispersionen, können die funktionellen Eigenschaften der Schichten oder Matrizen unter anderem über die Größe der verwendeten Partikel eingestellt werden. Hierzu können beispielsweise homogene Partikelgrößenverteilungen eingesetzt werden, als auch heterogene. Eine weitere bevorzugte Ausführungsform der Erfindung sieht vor, die Verteilung der Partikelgrößen so auszuwählen, dass diese innerhalb einer Größenordnung liegen, also das Verhältnis von kleinstem zu größtem Partikel kleiner als 10 ist. Bevorzugt ist dieses Verhältnis kleiner als 4, mehr bevorzugt kleiner als 2 und besonders bevorzugt kleiner als 1,4. Bei möglichst homogenen Verteilungen ergeben sich gleichmäßigere funktionelle Eigenschaften der Schichten oder Matrizen über die gesamte Oberfläche, bzw. das gesamte Volumen.

Weitere Vorteile können bei manchen Ausführungsformen erzielt werden, wenn sich die Größe der Partikel nicht zu sehr von der mittleren Schichtdicke der herzustellenden Schicht unterscheidet. Gute Erfolge werden erreicht, wenn die mittleren Partikeldurchmesser zwischen 10% und 180% der angestrebten mittleren Schichtdicken liegen. Besonders vorteilhaft kann dies bei der Herstellung von Schichten im Powder-Layering-Verfahren sein. Nähert sich dieses Verhältnis an 95% an, kann sich die Funktionalität der Schicht, gemessen am Verhältnis der Permeabilität bzw. Löslichkeit in verschiedenen wässrigen Lösungen, in denen eine hohe Löslichkeit oder Permeabilität eher erwünscht, bzw. eher unerwünscht ist, verbessern.

Die Erfindung sieht deshalb in einer weiteren bevorzugten Ausführungsform vor, dass die Partikel mittlere Durchmesser von 10% bis 180% der Schichtdicke aufweisen, bevorzugt 20% bis 150%, mehr bevorzugt 40% bis 130%, besonders bevorzugt 60% bis 115% und speziell bevorzugt 90% bis 110%.

Um die Auflösungseigenschaften der Schichten oder Matrizen weiter zu verbessern, sieht die Erfindung in einer weiteren bevorzugten Ausführungsform vor, dass zumindest ein Teil der verwendeten Partikel statt monolithisch, wie z.B. bei einem einfachen Pulver, heterolithisch aufgebaut sind. Beispielhafte Ausführungen sind unter anderem beschichtete Pellets oder Granulate oder andere Partikelformen. Teilweise werden solche Partikel im Stand der Technik auch als heterogen bezeichnet. Eine beispielhafte Realisierungsmöglichkeit zeigt Ausführungsbeispiel 30.

Solche heterolithische Partikel können beispielsweise aus einem wasserlöslichen Kern, wie z.B. Zuckerpellets, HPMC-Granulat, sprühgetrockneten Microspheres usw. bestehen, welcher mit einem Material beschichtet ist, das vom pH Wert abhängige Löslichkeit oder Permeabilität aufweist. Der Kern kann auch Quellmittel, Sprengmittel, so genannte Distintegrants und/oder Superdisintegrants enthalten, bzw. aus ihnen bestehen, um die Auflösungseigenschaften der mit den Partikeln herzustellenden Schichten zu optimieren.

Die Verarbeitung kann dann ähnlich der bereits benannten Publikationen von SHIMONO et al. erfolgen, indem das dort verwendete Chitosan durch entsprechend beschichtete Partikel ersetzt wird, wobei sowohl Partikel mit einer Art funktioneller Beschichtung eingesetzt werden können, als auch Mischungen mit Partikeln, die unterschiedlich beschichtet sind, beispielsweise solche, die oberhalb eines bestimmten pH Wertes löslich werden, zusammen mit solchen, die unterhalb eines bestimmten, vorzugsweise niedrigeren pH Wertes löslich werden, wobei noch weitere, gegebenenfalls auch auf andere Art funktionalisierte Partikel beigemischt sein können, beispielsweise generell wasserlösliche Partikel, nicht lösliche Partikel, besonders stark quellbare Partikel und/oder bakteriell abbaubar beschichtete Partikel.

Diese Partikel oder entsprechende Mischungen können, ggf. mit Beifügungen weiterer Hilfsstoffe, auch zu Tabletten, Mini- oder Mikrotabletten verpresst werden, oder auch feucht- oder schmelzgranuliert werden.

Bezüglich der Verpressung und anderer Verarbeitungsarten sind dem Fachmann Maßnahmen bekannt, wie er erreichen kann, dass die funktionellen Beschichtungen auch beim Verpressen oder sonstigem Weiterverarbeiten intakt bleiben. Dies betrifft beispielsweise die Wahl der Kernmaterialien, unter anderem bezüglich der Komprimierbarkeit, des Elastizitätsmoduls etc., die Zugabe entsprechender Plastifizierer zu den Beschichtungsmaterialien, und einiges mehr.

Entsprechende Rezepturen und Formulierungsanleitungen sind in der entsprechenden Fachliteratur, in Broschüren der einschlägigen Hersteller (z.B. Evonik, Darmstadt) und in veröffentlichten Patentdokumenten zu finden. Ausführungsmöglichkeiten sind in den Ausführungsbeispielen 17 und 18 gezeigt.

Eine weitere bevorzugte Ausführungsform der Erfindung sieht vor, dass eine oder mehrere weitere Schichten, die innerste Schicht oder sowohl die innerste Schicht, als auch eine oder mehrere der darüber angeordneten Schichten nicht nur vom pH Wert abhängig löslich oder permeabel werden können, sondern dass diese zusätzlich auch empfindlich gegenüber bakteriellen Enzymen sind, so dass auch ohne einen entsprechenden Abfall des pH Wertes eine Freisetzung im Dickdarm erfolgen kann, wenn dort entsprechende bakterielle Enzyme freigesetzt werden.

Beispielhafte Ausführungen sind in den Ausführungsbeispielen 1b, 1c, 3 ,4 ,5 ,6 ,7 ,8a ,8b ,9a-e und 10 gezeigt. An Stelle von Chitosan können auch andere bakteriell abbaubare Polymer verwendet werden, beispielsweise Guar, Amylose, Pektin. Gegebenenfalls muss dann ein weiteres Polymer hinzugefügt werden, welches die Abhängigkeit der Schicht vom pH Wert der sie umgebenden wässrigen Lösung wieder herstellt.

Mit den beschriebenen und implizierten Ausführungsformen der Erfindung mit innerster Schicht, bei denen der bestimmte zweite pH Wert der innersten Schicht mindestens so hoch ist, wie der maximal im Gastrointestinaltrakt zu erwartenden pH-Wert, und bevorzugt mindestens so hoch ist, wie der maximal im Dünndarm zu erwartende pH Wert, wird sichergestellt, dass der oder die Wirkstoffe, bzw. ein oder mehrere wirkstoffhaltige Kerne, nicht freigesetzt werden, solange der pH-Wert der umgebenden wässrigen Lösung monoton steigt, weil nach der Auflösung, bzw. nach dem permeabel Werden jeder Schicht die nächste Schicht freigesetzt, bzw. der wässrigen Lösung ausgesetzt wird, die bei monoton steigenden pH-Werten erst bei einem noch höheren pH-Wert löslich oder permeabel wird, was sich so lange fortsetzt, bis die innerste Schicht erreicht ist, welche sich zu höheren pH-Werten hin gar nicht mehr auflöst, jedenfalls nicht innerhalb von höheren pH-Werten, die nicht den maximal im Gastrointestinaltrakt zu erwartenden pH-Wert übersteigen, und bevorzugt nicht innerhalb von höheren pH-Werten, die nicht den maximal im Dünndarm zu erwartenden pH-Wert übersteigen.

Da aber bei nicht monoton steigenden pH-Werten, also dann, wenn der pH-Wert sich nach Überschreitung des letzten pH Wertes der inneren Schicht, und somit nach deren Auflösung oder permeabel Werdung, senkt, und zwar unter den bestimmten unteren pH Wert der Schicht, deren bestimmter oberer pH Wert noch nicht überschritten wurde, bzw. unter den bestimmten ersten pH Wert der innersten Schicht, diese Schicht löslich oder permeabel wird, werden dabei alle noch vorhandenen Schichten löslich oder permeabel, da deren bestimmter unterer bzw. bestimmter erster pH Wert somit ebenfalls unterschritten ist, so dass bei einem entsprechenden Absinken des pH-Werts der oder die Wirkstoffe, bzw. der oder die wirkstoffhaltigen Kerne freigesetzt werden.

Mit den beschriebenen und implizierten Ausführungsformen der Erfindung ohne innerste Schicht, oder bei denen der bestimmte zweite pH Wert der innersten Schicht niedriger ist, als der maximal im Dünndarm zu erwartenden pH-Wert, werden der oder die Wirkstoffe, bzw. ein oder mehrere wirkstoffhaltige Kerne, ebenfalls nicht freigesetzt, solange der pH-Wert der umgebenden wässrigen Lösung monoton steigt, allerdings nur so lange, wie der bestimmte obere pH Wert der am weitesten innen liegenden weiteren Schicht oder der bestimmte zweite pH Wert der innersten Schicht nicht überschritten wird. Nach einer entsprechenden Überschreitung wird bei diesen Ausführungsformen die Freisetzung ausgelöst. Die Freisetzung bei nicht monoton steigenden pH Werten entspricht den Ausführungsformen mit innerster Schicht, bei denen der bestimmte zweite pH Wert der innersten Schicht mindestens so hoch ist, wie der maximal im Dünndarm zu erwartenden pH-Wert.

Da für die Freisetzung im Dickdarm keine bakteriellen Enzyme erforderlich sind, sieht eine bevorzugte Ausführungsform der Erfindung deshalb eine Formulierung zur gezielten Freisetzung eines oder mehrerer Wirkstoffe oder eines oder mehrerer wirkstoffhaltiger Kerne innerhalb des Verdauungstrakts vor, bei der die Freisetzung nicht direkt durch die Anwesenheit bakterieller Enzyme im umgebenden Medium ausgelöst wird. Mehr bevorzugt werden für die Realisierung der Beschichtungen oder auf sonstige Art ausgestalteten Umhüllungen keine Materialien eingesetzt, die im Bereich zwischen pH 5 und pH 8 unlöslich sind, und gleichzeitig ausreichend empfindlich gegenüber Enzymen von Dickdarmbakterien sind, um eine durch solche Enzyme getriggerte Freisetzung der Wirkstoffe zu ermöglichen.

Da der Abfall des pH Wertes im Verdauungstrakt nach Erreichen der Ileozäkalklappe unabhängig von der inneren Zusammensetzung der oralen Verabreichungsform erfolgt, sieht die Erfindung eine weitere bevorzugte Ausführungsform vor, bei der die Auflösung oder das permeabel werden einzelner oder mehrerer verwendeter Materialien nicht darauf angewiesen ist, dass der wirkstoffhaltige Kern Säuren oder Säure freisetzende Stoffe enthält.

Es ist in einer weiteren bevorzugten Ausführungsform der Erfindung vorgesehen, dass dem oder den Wirkstoffen oder dem oder den wirkstoffhaltigen Kernen ein den pH Wert desselben oder derselben erhöhende Substanz beigemischt ist, wobei der pH Wert vorzugsweise eingestellt wird auf zwischen pH 3 und pH 8, mehr bevorzugt auf zwischen pH 4 und pH 7, besonders bevorzugt auf zwischen pH 5 und pH 6. Der pH Wert liegt dann vorzugsweise relativ mittig in dem im Gastrointestinaltrakt zu erwartenden Bereich, in dem auch die Schwellwerte zwischen Löslichkeit und Unlöslichkeit bzw. Permeabilität und keiner Permeabilität der verwendeten Schichten bevorzugt liegen.

Es ist auch eine weitere bevorzugte Ausführungsform vorgesehen, bei welcher mindestens ein Wirkstoff selbst, oder mindestens ein Material, das vom einem oder mehreren Wirkstoffkernen umfasst wird, bzw. das dieser oder diese enthalten, den pH Wert einer wässrigen Lösung erhöhen kann, sofern diese wässrige Lösung selbst einen pH Wert von unter 3, bevorzugt einen pH Wert im Bereich von unter 5, besonders bevorzugt einen pH Wert von unter 7 hat.

Durch die sehr sichere Freisetzung des oder der Wirkstoffe erst nach Erreichen des Dickdarms wird das Risiko einer unbeabsichtigten Absorption im Dünndarm äußerst gering gehalten, weshalb die Erfindung besonders dazu geeignet ist, einen oder mehrere Wirkstoffe zu verabreichen, welche bei unbeabsichtigter Freisetzung im Dünndarm in der verwendeten Menge toxisch sind oder unerwünschte Wirkungen hervorrufen, die das Verhältnis von Nutzen zu Risiko verschlechtern.

Eine weitere bevorzugte Ausführungsform der Erfindung sieht deshalb vor, die beschriebenen Formulierungen zur Verabreichung eines oder mehrerer solcher Wirkstoffe zu verwenden. Beispielsweise wird die Erfindung verwendet zur Verabreichung von immunsuppressiven Wirkstoffen, Steroiden, Corticosteroiden, Schwermetallen, Zytostatika, zytotoxischen Wirkstoffen, Prednisolon, Budesonid, MAO-Hemmer.

Da durch die Freisetzung im Dickdarm der Einfluss von Verdauungsenzymen auf den oder die zu verabreichenden Wirkstoffe minimiert wird, empfiehlt die Erfindung besonders solche Stoffe mittels der beschriebenen Formulierungen zu verabreichen, die empfindlich gegenüber den Bedingungen im oberen Verdauungstrakt sind, beispielsweise Peptide, Impfstoffe und Hormone.

Weitere Stoffe, welche die Erfindung als Wirkstoffe vorsieht, und die sowohl einzeln als auch in Kombination zweier oder mehrerer Wirkstoffe mit Hilfe der Erfindung und ihrer Ausführungsformen gezielt freigesetzt werden können, sind beispielsweise:
Acebutolol, Acetylcystein, Acetylsalicylsäure, Aciclovir, Alprazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure,
Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Selegilin, Chloramphenicol, Chlorhexidin, Chlor-pheniramin, Chlortalidon, Cholin, Cyclosporin, Cilastatin, Cimetidin, Ciprofloxacin,
Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin,
Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure,
Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrat, Isotretinoin, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Imipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. - Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin,
Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Pentoxifyllin, Phenoxymethylpenicillin, Phenylephrin, Phenylpropanolamin, Phenytoin, Piroxicam, Polymyxin B, Povidon-lod, Pravastatin, Prazepam, Prazosin, Prednisolon, Prednison, Bromocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Folinsäure, Zidovudin, somatostatin, insulin, calcitonin, vasopressin, gastrin, EGF (epidermal growth factor), .alpha.-hANP (.alpha.-human atrial natriuretic peptide), enkephalin, endorphin, GM-CSF (granulocyte macrophage colony-stimulating factor), G-CSF (granulocyte colony-stimulating factor), human growth hormone, glucagon, t-PA (tissue plasminogen activator), TNF (tumor necrosis factor), TCGF (T cell growth factor), ACTH (adrenocorticotrophic hormone), interleukins, interferon, EPO (erythropoietin), urokinase, neocarcinostatin, bradykinin, immunoglobulin and its digestion product, various allergens and their digestion products, ketoprofen, ibuprofen, diclofenac, indometacin, ketrolac, fenbufen, loxoprofen, tenidap, piroxicam, tenoxicam, salazosulfapyridine, pipethanate hydrochloride, mepenzolate bromide, und sennosides A und B.

Die Erfindung sieht in einer weiteren bevorzugten Ausführungsform vor, dass der oder die Wirkstoffe in einer sicheren und effektiven Menge in der Formulierung enthalten sind. Da bei der Verabreichung von einem oder mehreren Wirkstoffen mittels der verschiedenen Ausführungsformen der Erfindung die Sicherheit erhöht, bzw. das Risiko unerwünschter Wirkungen verringert ist, gegenüber Verabreichungsformen, welche den oder die Wirkstoffe nur mit einer geringeren Sicherheit im Dickdarm freisetzen, sieht eine weitere bevorzugte Ausführungsform der Erfindung vor, dass die Menge der umfassten Wirkstoffe bis zu 20% höher ist, bevorzugt bis zu 50% höher und weiter bevorzugt um mehr als 50% höher, als in Verabreichungsformen, welche den oder die Wirkstoffe nicht sicher im Dickdarm freisetzen.

Die Verwendung der beschriebenen aber auch anderer Wirkstoffe, in einer der in den verschiedenen Ausführungsformen dieser Erfindung beschriebenen oder nahe gelegten Verabreichungsformen eignet sich insbesondere zur Behandlung von entzündlichen Darmerkrankungen, wie beispielsweise Colitis Ulcerosa oder Morbus Crohn, von neurologischen Störungen, wie beispielsweise Depressionen oder Aufmerksamkeitsdefizitsyndromen, von Stoffwechselstörungen wie beispielsweise Diabetes, von neoplastischen Krankheiten wie beispielsweise kolorektalen Karzinomen, weshalb die Erfindung in weiteren bevorzugten Ausführungsformen solche Verwendungen vorsieht.

Der oder die wirkstoffhaltigen Kerne können auch bereits freisetzungskontrollierende Eigenschaften besitzen, wie z.B. Beschichtungen mit retardierenden Eigenschaften, Matritzen mit sustained Release Eigenschaften, Verwendung von Kernen aus biologisch abbaubaren Materialien, weshalb die Erfindung in weiteren bevorzugten Ausführungsformen vorsieht, bei allen dargestellten und durch Kombination von verschiedenen bevorzugten Ausgestaltungsmöglichkeiten realisierbaren Ausführungsformen auch solche Kerne zu verwenden.

In einer weiteren bevorzugten Ausführungsform der Erfindung wird auf die Herstellung der innersten Schicht verzichtet, so dass die erste auf den oder die Wirkstoffe oder den oder die wirkstoffhaltigen Kerne aufgebrachte Schicht, welche ein entsprechend der beschriebenen inneren, innersten oder weiteren Schichten vom pH Wert abhängiges Löslichkeits- oder Permeabilitätsverhalten aufweist, die oder eine der beschriebenen weiteren Schichten ist.

In einer weiteren bevorzugten Ausführungsform weist diese Schicht einen bestimmten oberen pH Wert auf, der nicht höher ist, als der höchste pH Wert, der bei der Zielgruppe im Dünndarm zu erwarten ist.

In einer weiter bevorzugten Ausführungsform ist der bestimmte obere pH Wert dieser Schicht niedriger als der höchste pH Wert, der bei der Zielgruppe im Dünndarm zu erwarten ist, und zwar bevorzugt so viel niedriger, dass bei Individuen, bei denen der maximal im Dünndarm zu erwartende pH Wert erreicht wird, sich diese Schicht frühestens dann ausreichend aufgelöst hat, bzw. permeabel geworden ist, wenn die Ileozäkalklappe erreicht ist.

Dies wird beispielsweise dadurch erreicht, dass der pH Wert, oberhalb dem die Schicht löslich oder permeabel wird, im Dünndarm der Individuen der Zielgruppe maximal eine bestimmte Zeit vor dem Erreichen der Ileozäkalklappe erreicht wird, und die Zeit, die benötigt wird, bis diese Schicht bei dem erreichten pH Wert ausreichend aufgelöst, bzw. permeabel geworden ist, um den oder die Wirkstoffe oder den oder die wirkstoffhaltigen Kerne freizugeben, mindestens so groß ist, wie die vorgenannte maximale Zeit.

In einer weiteren bevorzugten Ausführungsform ist unter dieser Schicht, bei geometrisch parallel angeordneten Schichten vorzugsweise unter der Teilschicht, die bei höheren pH Werten löslich oder permeabel ist, eine Schicht angeordnet, die sich zeitverzögert auflöst oder permeabel wird, damit die entsprechenden zeitlichen Bedingungen erfüllt werden.

Realisiert ist eine solche Ausführungsform in Beispiel 14, welches die Ausführungsform aber nicht auf diese Realisierungsmöglichkeit beschränken soll. Auch Beispiel 38 zeigt eine mögliche Realisierung.

Vorzugsweise löst sich die zeitverzögert lösliche Schicht innerhalb eines Zeitraums von mehr als 10 Minuten auf, mehr bevorzugt innerhalb von mehr als 20 Minuten, besonders bevorzugt innerhalb von mehr als 45 Minuten. Der Zeitraum beträgt vorzugsweise nicht mehr als 60 Minuten, mehr bevorzugt nicht mehr als 40 Minuten und besonders bevorzugt nicht mehr als 25 Minuten. Eine zeitverzögert ausreichend permeabel werdende Schicht wird bevorzugt nach den gleichen Zeiträumen ausreichend permeabel. Vorzugsweise entsprechen die vorgenannten Zeiträume auch der Zeit vor Erreichen des Dickdarms, ab der in der Zielgruppe frühestens der bestimmte obere pH Wert der am weitesten innen gelegenen weiteren Schicht überschritten wird. Durch diese Verzögerung kann erreicht werden, dass die am weitesten innen gelegene weitere Schicht nicht selbst besonders langsam löslich sein muss, so dass es auch zur Freisetzung kommt, wenn ihr bestimmter oberer pH Wert erst sehr kurz vor Erreichen des Dickdarms überschritten wird. Besonders bevorzugt dienen diese Zeiträume als Definition für "kurz" im Ausdruck "kurz vor dem Erreichen des Dickdarms" im Sinne verschiedener Ausführungsformen der Erfindung.

Bei Ausführungsformen, bei denen die weitere Schicht oder die weiteren Schichten durch effektiv parallel angeordnete Teilschichten realisiert werden, von denen mindestens eine erste Teilschicht oberhalb eines bestimmten oberen pH Wertes, und mindestens eine zweite Teilschicht unterhalb eines bestimmten unteren pH Wertes löslich oder permeabel wird, tragen die Teile der direkt über der ersten Teilschicht angeordneten Schicht, die oberhalb eines bestimmten pH Wertes löslich oder permeabel werden, und die Teile der direkt unter der zweiten Teilschicht angeordneten Schicht, die unterhalb eines bestimmten pH Wertes löslich oder permeabel werden, nicht wesentlich zur vom Verlauf des pH Wertes abhängigen Freisetzung bei. Die Teile der direkt über der ersten Teilschicht angeordneten Schicht, die oberhalb eines bestimmten pH Wertes löslich oder permeabel werden, werden dadurch, dass ihr bestimmter oberer pH Wert niedriger liegt, als derjenige der ersten Teilschicht, ohnehin vor dieser löslich oder permeabel. Die Teile der direkt unter der zweiten Teilschicht angeordneten Schicht, die unterhalb eines bestimmten pH Wertes löslich oder permeabel werden, werden dadurch, dass ihr bestimmter unterer pH Wert höher liegt, als derjenige der zweiten Teilschicht, ohnehin löslich oder permeabel, sobald diese löslich oder permeabel geworden ist, ohne dass es einer weiteren Änderung des pH Wertes bedarf.

Beispielhaft sei von einer Verabreichungsform ausgegangen, die aus einer innersten Schicht, welche unterhalb von pH 6,0 löslich wird, einer weiteren Schicht, welche aus effektiv parallel angeordneten Materialgemischen besteht, von denen das eine unterhalb von pH 5,5 löslich wird, das andere oberhalb von pH 6,5, und einer inneren Schicht, welche oberhalb von pH 6,0 löslich wird, besteht. Wird die effektiv parallele Anordnung der Materialgemische der weiteren Schicht durch eine geometrisch nebeneinander liegende (also effektiv parallele) Ausführung von Teilschichten realisiert, so ist der unter der Teilschicht mit dem Materialgemisch, welches unterhalb von pH 5,5 löslich ist, liegende Teil der innersten Schicht nicht erforderlich, da dieser sich nach Auflösung der darüber liegenden Teilschicht der weiteren Schicht ebenfalls unmittelbar auflöst. Ebenso ist der über der Teilschicht mit dem Materialgemisch, welches oberhalb von pH 6,5 löslich ist, liegende Teil der inneren Schicht nicht erforderlich, da dieser nur in einem pH Bereich beständig ist, in dem auch die darunter liegende Teilschicht der weiteren Schicht ebenfalls beständig ist, und deshalb nicht zur Funktionalität beiträgt.

Deshalb sieht eine weitere bevorzugte Ausführungsform der Erfindung vor, einen oder mehrere dieser Teile einer oder mehrerer entsprechender Schichten nicht auszubilden oder nach der Ausbildung wieder zu entfernen. Um solche Schichtteile nicht auszubilden, können die entsprechenden Bereiche beispielsweise beim Aufbringen der Schichten oder Teilschichten abgedeckt werden, beispielsweise durch Anordnung von Schattenmasken beim Aufsprühen von schichtbildenden Lösungen. Entsprechende Bereiche können aber auch bei anderen Arten der geometrisch definierten Ausbildung von Schichten ausgespart werden, beispielsweise durch definiertes unvollständiges Eintauchen in schichtbildende Lösungen oder Dispersionen.

Sind Teile von Schichten, die oberhalb eines bestimmten oberen pH Wertes löslich oder permeabel werden, direkt unter einer Teilschicht angeordnet, die unterhalb eines bestimmten unteren pH Wertes löslich oder permeabel wird, ist zumindest in dem Bereich, in dem diese überlappen, eine der beiden Schichten nicht wesentlich für die vom Verlauf des pH Wertes abhängige Freisetzung, weshalb es die Erfindung in einer weiteren bevorzugten Ausführungsform vorsieht, im überlappenden Bereich eine der beiden Schichten nicht auszubilden oder nach der Ausbildung wieder zu entfernen.

In einer weiteren bevorzugten Ausführungsform der Erfindung werden Teilschichten oder Teile von Schichten, die entsprechend den vorgenannten Varianten nicht ausgebildet oder nach der Ausbildung wieder entfernt werden, zumindest im Randbereich der Überlappung ausgebildet oder nicht entfernt, um eine unbeabsichtigte Freisetzung zu vermeiden, wenn die Teile der jeweiligen Schichten oder die Teilschichten und andere Schichten oder Teilschichten beispielsweise aufgrund von Fertigungstoleranzen nicht lückenlos aneinander stoßen oder ineinander übergehen, oder wenn eine Überlappung erstrebenswert ist.

In einer weiteren bevorzugten Ausführungsform werden eine oder mehrere Bereiche, in denen Teilschichten mit unterschiedlichen Empfindlichkeiten gegenüber pH Werten wässriger Lösungen aneinander stoßen, durch Aufbringung von in wässrigen Lösungen unlöslicher Materialien oder Materialmischungen abgedeckt. Beispielhaft seien Verabreichungsformen genannt, bei denen entsprechende Teilschichten auf Kapselkörpern und Kapselkappen aufgebracht sind, und bei denen die Stoßstelle oder der beim Zusammenstecken der Kapselhälften entstehende Spalt mittels Banderolisierung oder mittels Einbringung eines nicht wasserlöslichen Polymers versiegelt wird.

Eine weitere Möglichkeit, den oder die Wirkstoffe, oder einen oder mehrere wirkstoffhaltige Kerne auch dann sicher beim Erreichen des Dickdarms freizusetzen, wenn der Abfall des pH Wertes beim Eintritt in den Dickdarm geringer ist, als die interindividuelle Variabilität des maximalen pH Wertes im Dünndarm, besteht in der effektiv parallelen Anordnung von zwei oder mehr Schichtfolgen von inneren und innersten Schichten, jeweils mit oder ohne weiteren Schichten, weshalb die Erfindung dies in einer weiteren bevorzugten Ausführungsform vorsieht.

Diese effektiv parallele Anordnung von Schichtfolgen kann ähnlich realisiert werden, wie die parallele Anordnung verschiedener Materialien oder Materialgemische zur Herstellung der in den vorgenannten Ausführungsformen beschriebenen weiteren Schicht oder Schichten.

Beispielsweise kann die parallele Anordnung durch geometrisch definiertes Aufbringen der Schichtfolgen erfolgen, wie z.B. durch Aufsprühen der Schichtfolgen aus unterschiedlichen Richtungen, durch Maskierung bestimmter Bereiche mithilfe von Schattenmasken oder durch separate Herstellung der Schichtfolgen und anschließende mechanische parallele Anordnung dieser. Ebenso kann die effektiv parallele Anordnung erfolgen, indem Partikel, beispielsweise Pulverpartikel oder Microspheres, mit entsprechenden Schichtfolgen beschichtet werden, und diese dann effektiv parallel angeordnet werden, beispielsweise durch Verpressung von wirkstoffhaltigen Partikeln mit einer Mischung von mit unterschiedlichen Schichtfolgen beschichteten Partikeln oder durch die Verwendung von mit unterschiedlichen Schichtfolgen beschichteten Partikeln in Powder-Layering-Verfahren. Durch die effektiv parallele Anordnung dieser Schichtfolgen bewirkt die Auflösung oder das permeabel werden einer dieser Schichtfolgen, dass der Darminhalt in Kontakt mit dem oder den Wirkstoffen oder dem oder den wirkstoffhaltigen Kernen kommt, und der oder die Wirkstoffe freigesetzt werden können.

Die einzelnen Schichtfolgen bestehen jeweils aus einer inneren Schicht, welche oberhalb eines bestimmten letzten pH Wertes löslich oder permeabel wird, und einer innersten Schicht, welche unterhalb eines bestimmten ersten pH Wertes löslich oder permeabel wird, wobei die innerste Schicht so angeordnet ist, dass diese erst dann dem Darminhalt ausgesetzt wird, wenn die innere Schicht löslich oder permeabel geworden ist.

Zwischen der innersten und der inneren Schicht können die einzelnen Schichtfolgen auch noch eine oder mehrere weitere Schichten enthalten, wie sie bereits in den vorgenannten Ausführungsformen der Erfindung beschrieben sind.

Jede dieser einzelnen Schichtfolgen ermöglicht dem Darminhalt mit der darunter liegenden Struktur, die üblicherweise aus einem oder mehreren Wirkstoffen, einem oder mehreren wirkstoffhaltigen Kernen, aber auch aus zusätzlichen funktionalen oder nichtfunktionalen Schichten bestehen kann, in Kontakt zu treten, sobald ein bestimmter pH Wert überschritten wurde, und sich danach der pH Wert unter einen bestimmten pH Wert oder um einen bestimmten pH Wert abgesenkt hat.

Dadurch ist es möglich, dass auch ein geringer Abfall des pH Wertes ausgenutzt werden kann, um einen oder mehrere Wirkstoffe kontrolliert freizusetzen.

Eine einzelne Schichtfolge ohne weitere Schichten ermöglicht dies nur dann, wenn der maximal in Dünndarm erreichte pH Wert höher wird, als der bestimmte letzte pH Wert ihrer inneren Schicht, aber nicht höher als die Summe des bestimmten ersten pH Wertes ihrer innersten Schicht und des Abfalls des pH Wertes beim Eintritt in den Dickdarm, also nur innerhalb eines bestimmten Bereiches von pH Werten.

Durch die Anpassung der bestimmten ersten und letzten pH Werte ihrer innersten und inneren Schichten kann der bestimmte Bereich von pH Werten, innerhalb dem die einzelnen Schichtfolgen die Freisetzung beim Abfall des pH Wertes um einen bestimmten Wert ermöglichen, eingestellt werden. Der Bereich einer einzelnen Schichtfolge ist um den Abstand des bestimmten ersten pH Wertes der innersten Schicht vom bestimmten letzten pH Wert der inneren Schicht kleiner als der Wert, um den der pH Wert maximal absinken muss, um eine Freisetzung sicher einzuleiten.

Durch die effektiv parallele Anordnung von zwei oder mehr solcher Schichtfolgen mit unterschiedlich eingestellten Bereichen, kann der Bereich von pH Werten, in dem der oder die Wirkstoffe oder der oder die wirkstoffhaltigen Kerne freigesetzt werden, vergrößert werden, so dass es für eine sichere Freisetzung nicht erforderlich ist, dass der Abfall des pH Wertes beim Eintritt in den Dickdarm größer ist, als die interindividuelle Variabilität des maximal im Dünndarm erreichten pH Wertes.

Durch Verwendung von weiteren Schichten zwischen den inneren und innersten Schichten bei einer oder mehreren der jeweiligen Schichtfolgen, wie sie auch bei den Ausführungsformen der Erfindung beschrieben sind, die nur eine einzelne Schichtfolge verwenden, kann der Bereich von pH Werten, in dem eine einzelne Schichtfolge die Freisetzung beim Abfall des pH Wertes sicherstellt, vergrößert werden, so dass weniger parallel angeordnete Schichtfolgen erforderlich sind, oder bei gleicher Anzahl parallel angeordneter Schichtfolgen der Bereich von pH Werten, in dem die Formulierung eingesetzt werden kann, vergrößert werden kann, weshalb eine weitere bevorzugte Ausführungsform der Erfindung dies vorsieht.

Die effektiv parallele Anordnung von zwei oder mehr Schichtfolgen mit weiteren Schichten ermöglicht es, bei den einzelnen Schichtfolgen eine geringere Anzahl von weiteren Schichten einzusetzen, als es bei Verwendung einer einzigen Schichtfolge erforderlich wäre.

Vorzugsweise werden die Bereiche der einzelnen Schichtfolgen so eingestellt, dass sie aneinander anschließen, weshalb die Erfindung dies in einer weiteren bevorzugten Ausführungsform vorsieht. In einer weiteren, noch mehr bevorzugten Ausführungsform sieht die Erfindung vor, dass die einzelnen Schichtfolgen so realisiert werden, dass die Bereiche sich leicht überlappen, um auch beim Auftreten von Fertigungstoleranzen sicherzustellen, dass keine Lücken zwischen den Bereichen auftreten.

Auch bei der effektiv parallelen Anordnung von zwei oder mehr Schichtfolgen kann es vorteilhaft sein, zumindest bei einer der Schichtfolgen den bestimmten zweiten pH Wert der innersten Schicht kleiner zu wählen, als der in der Zielgruppe maximal im Dünndarm zu erwartende pH Wert, oder zumindest bei einer der Schichtfolgen auf die Ausbildung der innersten Schicht zu verzichten, weshalb die Erfindung dies in weiteren bevorzugten Ausführungsformen vorsieht. Bei solchen Ausführungsformen kann auch dann eine Freisetzung erreicht werden, wenn der pH Wert über einen bestimmten Wert ansteigt, und zwar entweder über den vorgenannten bestimmten zweiten pH Wert, den bestimmten oberen pH Wert der am weitesten innen angeordneten weiteren Schicht der Schichtfolge ohne innerste Schicht, oder den bestimmten oberen pH Wert der inneren Schicht jener Schichtfolge, welche weder eine innerste Schicht, noch eine weitere Schicht aufweist. Um die effektiv parallele Anordnung der einzelnen Schichtfolgen zu vereinfachen, können in weiteren bevorzugten Ausführungsformen der Erfindung innerste Schichten von Schichtfolgen, deren bestimmter erster pH Wert höher ist, zusätzlich auch unterhalb von Schichtfolgen mit niedrigeren bestimmten ersten pH Werten angeordnet sein, bzw. sich darunter erstrecken.

Ebenso können innere Schichten von Schichtfolgen, deren bestimmter letzter pH Wert niedriger ist, zusätzlich auch oberhalb von Schichtfolgen mit höheren bestimmten ersten pH Werten angeordnet sein, bzw. sich darüber erstrecken, wie in Ausführungsbeispiel 24 gezeigt.

Entsprechende zusätzliche Anordnungen oder Erstreckungen können jeweils über die ganze Oberfläche der Verabreichungsform erfolgen, oder auch nur über bestimmte Teilbereiche. Auch bei der Realisierung einer oder mehrerer weiterer Schichten sind entsprechende Erstreckungen möglich und bei weiteren bevorzugten Ausführungsformen der Erfindung vorgesehen.

Die effektiv parallele Anordnung von Schichtfolgen kann auch gemischt mit der seriellen Anordnung von innerer, innerster und weiteren Schichten erfolgen.

Beispielhafte Realisierungen einiger Ausführungsformen zeigen die Ausführungsbeispiele 22 bis 24.

Mit den beschriebenen Ausführungsformen und mit von einem Fachmann entsprechend abwandelbaren Variationen kann sichergestellt werden, dass die nicht vor der Passage der Ileozäkalklappe freigesetzte Menge bevorzugt zu mehr als 30% vor der Passage der ersten Flexur des Dickdarms freigesetzt wird, mehr bevorzugt, dass sie zu mehr als 50% vor der Passage der ersten Flexur des Dickdarms freigesetzt wird, besonders bevorzugt, dass sie zu mehr als 75% vor der Passage der ersten Flexur des Dickdarms freigesetzt wird.

Die beschriebenen Ausführungsformen der Erfindung sind unter anderem dazu geeignet, multipartikuläre Verabreichungsformen herzustellen, weshalb multipartikuläre Verabreichungsformen mit den beschriebenen Merkmalen eine weitere bevorzugte Ausführungsform der Erfindung darstellen.

Insbesondere eignen sich die beschriebenen Ausführungsformen der Erfindung dazu, singuläre Verabreichungsformen bereitzustellen, da auch längere Verweilzeiten dieser Verabreichungsformen vor der Ileozäkalklappe, wie sie bei nichtmultipartikulären Verabreichungsformen häufiger vorkommen können, nicht zu einer verfrühten Freisetzung führen, da ein ausreichend großer Abfall des pH Wertes erst nach der Passage der Ileozäkalklappe erfolgt. Besonders bevorzugt werden für singuläre Verabreichungsformen Ausführungsformen verwendet, welche eine innerste Schicht aufweisen, deren bestimmter zweiter pH Wert mindestens so hoch ist, wie der maximal im Dünndarm zu erwartenden pH-Wert.

Mit entsprechend bevorzugten Ausführungsformen der Erfindung kann erreicht werden, dass vor dem Erreichen des Dickdarms maximal 50% des oder der Wirkstoffe freigesetzt werden, bevorzugt maximal 30%, mehr bevorzugt maximal 10%, besonders bevorzugt maximal 5% und speziell bevorzugt maximal 1%.

Die Merkmale der Erfindung können auch in Kombinationen verwendet werden, die nicht explizit in Beispielen erwähnt sind, aber doch als bevorzugte Ausführungsformen der Erfindung anzusehen sind. Abhängig von der konkreten Aufgabenstellung kann es zielführend sein, einige Merkmale in ihren besonders bevorzugten Ausgestaltungen zu implementieren, andere, optionale Merkmale dagegen eventuell gar nicht auszubilden. Die vorliegende Beschreibung, die aufgeführten und auf sie verwiesenen Schriften und die Ausführungsbeispiele ermöglicht es dem Fachmann unter Berücksichtigung seines Fachwissens und der einschlägigen Fachliteratur die beschriebenen Merkmale auch in weiteren sinnvollen Kombinationen anzuwenden, sowie die beispielhaft dargestellten Realisierungswege abzuwandeln, um diverse Eigenschaften in für seine konkrete Anwendung vorteilhaftere Bereiche zu bringen.

Geeignete Prozesse um die beschriebenen Schichten herzustellen, können auch folgenden Werken entnommen werden: Bauer, Lehman, Osterwald, Rothgang "Coated Pharmaceutical Dosage Forms", 1998, Wissenschaftliche Verlagsgesellschaft mbH Stuttgart und CRC Press LCC, Boca Raton, Fla., USA or McGinity, "Aqueous Polymeric Coatings for Pharmaceutical Dosage Forms, Second Edition, Revised and Expanded", 1997, Marcel Dekker Inc., New York, USA.

Die folgenden Ausführungsbeispiele demonstrieren einige Ausführungsformen der Erfindung. Die Erfindung und die entsprechend beschriebenen Varianten sollen aber nicht auf diese Ausführungsformen beschränkt sein.

### Ausführungsbeispiele:

Vorbereitung der Wirkstoffe, beziehungsweise der wirkstoffhaltigen Kerne:

### 1. Hartgelatinekapseln mit Probiotikafüllung

Lyophilisierte Escherichia coli (100000 KBE), 200mg Laktose, 50mg mikrokristalline Cellulose und 20mg Magnesiumstearat werden gemischt und in Hartgelatinekapseln der Größe 2 abgefüllt

### 2. a. Prednisolonbeschichtete Pellets

Nonpareille-Pellet-Kerne aus mikrokristalliner Cellulose werden im Wirbelstrombeschichter mit Prednisolon beschichtet.

### 2. b. Prednisolonhaltige Mikrotabletten (im Stand der Technik teilweise auch als Minitabletten bezeichnet)

Prednisolon, Lactose, Hydroxypropylmethylcellulose und Magnesiumstearat werden im Verhältnis 8:2:6:1 vermischt und zu konvexen Mikrotabletten verpresst, mit einem Durchmesser von 1,5mm und einer Dicke von 1,2mm.

### 2. c. Prednisolonhaltige Mikropellets

Prednisolon, mikrokristalline Cellulose, Hydroxypropylmethylcellulose und Magnesiumstearat werden im Verhältnis 8:2:6:1 in eine wässrige Lösung gemischt. Sodann werden im Sprühtrocknungsverfahren aus dieser Lösung Mikropellets erzeugt.

### 3. Wolframbeschichtete Pellets

Nonpareille-Pellet-Kerne aus mikrokristalliner Cellulose werden im Wirbelstrombeschichter mit Natriumwolframat aus einer wässrigen Natriumwolframatlösung beschichtet.

### Beispiel 4

Hartgelatinekapseln mit Probiotikafüllung werden beschichtet wie in Beispiel 5, wobei die erste Beschichtung mit dem ungepfropften Chitosan nicht ausgeführt wird.

Nach der Magenpassage löst sich die mittlere Schicht auf, wenn der pH Wert im Duodenum oder Jejunum 6 überschreitet. Erhöht sich der pH Wert weiter, bis er kurz vor dem Erreichen des Dickdarms pH 7 überschreitet, wird das gepfropfte Chitosan löslich und der Kapselinhalt wird am Beginn des Dickdarms freigesetzt. Bei einem niedrigeren Verlauf des pH Wertes wird zwar auch pH 6 überschritten, allerdings erst im Verlauf des Ileums, wo sich die mittlere Schicht auflöst. Nach dem Durchtritt durch die Ileozäkalklappe sinkt der pH Wert unter 5 ab, wodurch das gepfropfte Chitosan löslich, und die Freisetzung ausgelöst wird.

Die Kapseln sind bestimmt zur oralen Verabreichung, wobei sich die zuletzt aufgebrachte Schicht im Magen auflöst, die als vorletzte aufgebrachte Schicht im Dünndarm, nachdem der pH-Wert über 6 gestiegen ist, und die zuerst aufgebrachte Schicht wenn entweder pH 5 unterschritten oder pH 7 überschritten wird. Der Arbeitsbereich erstreckt sich von pH 6 bis ca. pH 7,5. Die Toleranz gegenüber Schwankungen des pH Wertes innerhalb des Dünndarms beträgt eine pH Stufe.

Nach der Passage der Ileozäkalklappe werden 80% des Wirkstoffes innerhalb des Dickdarms freigesetzt.

### Beispiel 5:

Hartgelatinekapseln mit Probiotikafüllung werden beschichtet mit Chitosan.

Dazu wird aus Chitosan, das zu 90% deacetyliert ist, eine 2%ige wässrige Lösung hergestellt, nachdem dem Wasser Essigsäure zugesetzt wurde, in einer Menge, dass das molare Äquivalent basierend auf den Aminogruppen des Chitosan 1,9 ist.

Dann wird Dekaglycerin-Monopalmitinsäureester zugesetzt, in einer Menge von 15 Gewichtsprozent der Menge des Chitosan. Die Mischung wird gerührt, bis eine gleichmäßige Chitosanlösung entstanden ist. Die Beschichtung erfolgt mit einem Hicoater HC-LABO.

Die Auftragsmenge beträgt 4mg/cm². Diese Beschichtung ist löslich bei einem pH-Wert von unter 6,5.

Danach erfolgt eine Beschichtung mit Chitosan, welches zu 30% mit Chlorogensäure gepfropft ist, und die unlöslich ist im pH Bereich von 5 bis 7, aber löslich über pH 7 und unter pH 5.

Die Beschichtung entspricht der ersten Beschichtung, wobei das zu 90% deacetylierte Chitosan vor dem Herstellen der Beschichtungslösung zu 30% mit Chlorogensäure gepfropft wird. Die Pfropfung erfolgt wie in "Enzymatic Grafting of a Natural Product onto Chitosan to Confer Water Solubility Under Basic Conditions".

Die Auftragsmenge wird auf 6,5mg/cm² eingestellt.

Sodann werden die beschichteten Kapseln nochmals beschichtet mit einem Enteric Coating basierend auf Eudragit L 100, mit folgender Zusammensetzung der Sprühsuspension:

11,3% Eudragit L 100, 3,7% Talkum, 5,6% Triethylcitrat, 3,8% IN NH3 (1.7% NH3), und Wasser. Die Beschichtungsstärke ist 5mg/cm².

Diese Beschichtung ist löslich bei einem pH-Wert von über 6.

Danach erfolgt noch eine Beschichtung mit Kollicoat Smartseal 30D in einer Aeromatic Streal bei einer Zulufttemperatur von 55° und einer Sprührate von 8g/min bei einem Druck von 1,5 Bar und einem Düsendurchmesser von 0,8mm. Die Produkttemperatur bleibt dabei unter 40°, die Beschichtungsstärke ist 4,5mg/cm². Die Trocknung erfolgt für 5 Minuten bei 55° und für weitere ca. 10 Minuten bei 45° so lange bis die Auslasstemperatur 40° erreicht.

Die Sprühsuspension enthält 33,33% Kollicoat Smartseal 30D, 1,5% Tributylcitrat, 0,1% butyliertes Hydroxytoluol, 6% Talkum und als restlichen Inhaltsstoff Wasser.

Diese Beschichtung ist löslich bei einem pH-Wert von bis zu 5,5.

Die Kapseln sind bestimmt zur oralen Verabreichung, wobei sich die zuletzt aufgebrachte Schicht im Magen auflöst, die als vorletzte aufgebrachte Schicht im Dünndarm, nachdem der pH-Wert über 6 gestiegen ist, die nächste Schicht wenn entweder pH 5 unterschritten oder pH 7 überschritten wird, und die zuerst aufgebrachte Schicht schließlich im Zäkum, sobald der pH-Wert unter 6,5 fällt, bzw. gleich nachdem sich die vorhergehende Schicht beim Abfall unter pH 5 aufgelöst hatte.

### Beispiel 5b:

Hartgelatinekapseln mit Probiotikafüllung werden beschichtet wie in Beispiel 5, wobei die erste Beschichtung allerdings genau so ausgeführt wird, wie die letzte Beschichtung (Prozess und Beschichtungsmaterialien wie Beschichtung 4 in Beispiel 5).

Die Kapseln sind bestimmt zur oralen Verabreichung, wobei sich die zuletzt aufgebrachte Schicht im Magen auflöst, die als vorletzte aufgebrachte Schicht im Dünndarm, nachdem der pH-Wert über 6 gestiegen ist, die nächste Schicht wenn entweder pH 5 unterschritten oder pH 7 überschritten wird, und die zuerst aufgebrachte Schicht schließlich im Zäkum, sobald der pH-Wert unter 5,5 fällt, bzw. gleich nachdem sich die vorhergehende Schicht beim Abfall unter pH 5 aufgelöst hatte.

### Beispiel 5c:

Hartgelatinekapseln mit Probiotikafüllung werden beschichtet mit einem Poly(MMA-DEAEMA)-Copolymer. Das Copolymer und die daraus hergestellte Sprühlösung, sowie der Beschichtungsprozess entsprechen der letzten Beschichtung aus Beispiel 5, wobei bei der Herstellung des Copolymers der Anteil an DEAEMA gegenüber dem standardmäßigen Kollicoat Smartseal 30D erhöht ist, so dass das Copolymer bereits bei Unterschreitung eines pH-Wertes von 6,0 löslich wird (Schicht C, Zeichnung 6).

Danach erfolgt eine Beschichtung mit Chitosan, welches zu 30% mit Chlorogensäure gepfropft ist, und die unlöslich oder nur wenig und langsam löslich ist im pH Bereich von 5 bis 7, aber löslich über pH 7 und unter pH 5.

Dazu wird Chitosan, das zu 90% deacetyliert ist, zu 30% mit Chlorogensäure gepfropft. Die Pfropfung erfolgt wie in "Enzymatic Grafting of a Natural Product onto Chitosan to Confer Water Solubility Under Basic Conditions".

Aus diesem gepfropften Chitosan wird eine 2%ige wässrige Lösung hergestellt, nachdem dem Wasser Essigsäure zugesetzt wurde, in einer Menge, dass das molare Äquivalent basierend auf den Aminogruppen des Chitosan 1,5 ist.

Surelease (Ethylcellulose, Colorcon, Harleysville USA) wird 1:1 mit Wasser verdünnt. Die Surelease-Lösung und die ChitosanLösung werden im Verhältnis 1:1 (w/w) gemischt und gerührt bis eine gleichmäßige Durchmischung erreicht ist.

Die Beschichtung erfolgt mit einem Hicoater HC-LABO.

Die Auftragsmenge wird auf 7,5mg/cm² eingestellt.

Sodann werden die beschichteten Kapseln nochmals beschichtet mit einem Enteric Coating basierend auf Eudragit L 100 (Prozess wie die dritte Beschichtung in Beispiel 5).

Danach erfolgt eine letzte Beschichtung mit Kollicoat Smartseal 30D wie die letzte Beschichtung aus Beispiel 5.

Die Kapseln sind bestimmt zur oralen Verabreichung, wobei sich die zuletzt aufgebrachte Schicht im Magen auflöst, die als vorletzte aufgebrachte Schicht im Dünndarm, nachdem der pH-Wert über 6 gestiegen ist, die nächste Schicht wenn entweder pH 5 unterschritten oder pH 7 überschritten wird, und die zuerst aufgebrachte Schicht schließlich im Zäkum, sobald der pH-Wert unter 6 fällt, bzw. gleich nachdem sich die vorhergehende Schicht beim Abfall unter pH 5 aufgelöst hatte.

### Beispiel 6:

Hartgelatinekapseln mit Probiotikafüllung werden beschichtet mit Chitosan, welches unterhalb von pH 6,5 löslich ist (Prozess wie die erste Schicht in Beispiel 5).

Danach erfolgt eine Beschichtung mit Chitosan, welches zu 50% mit Chlorogensäure gepfropft ist, und unlöslich ist im pH Bereich von 5,5 bis 6,5.

Die Beschichtung entspricht der ersten Beschichtung, wobei das zu 90% deacetylierte Chitosan vor dem Herstellen der Beschichtungslösung zu 50% mit Chlorogensäure gepfropft wird.

Die Auftragsmenge wird auf 7,5mg/cm² eingestellt.

Sodann werden die beschichteten Kapseln nochmals beschichtet mit einem Enteric Coating basierend auf Eudragit L 100 (Prozess wie die dritte Beschichtung in Beispiel 5).

Danach erfolgt eine letzte Beschichtung mit Kollicoat Smartseal 30D wie die letzte Beschichtung aus Beispiel 5.

Die Kapseln sind bestimmt zur oralen Verabreichung, wobei sich die zuletzt aufgebrachte Schicht im Magen auflöst, die als vorletzte aufgebrachte Schicht im Dünndarm, nachdem der pH-Wert über 6 gestiegen ist, die nächste Schicht wenn entweder 5,5 unterschritten oder 6,5 überschritten wird, und die zuerst aufgebrachte Schicht schließlich im Zäkum, sobald der pH-Wert unter 6,5 fällt, bzw. gleich nachdem sich die vorhergehende Schicht beim Abfall unter pH 5,5 aufgelöst hatte.

### Beispiel 7:

Wolframbeschichtete Pellets werden beschichtet mit einer zitronensäurehaltigen HPMC-Schicht. Die wasserbasierte Sprühlösung ist zusammengesetzt aus 2,8% Pharmacoat 606 und 1,2% Talkum, wobei Zitronensäure zugesetzt wird, bis ein pH Wert von 5,0 erreicht ist.

Die Beschichtungsstärke ist 4,2mg/cm².

Danach erfolgt eine Beschichtung mit Chitosan (Prozess wie erste Schicht bei Beispiel 5).

Danach erfolgt eine Beschichtung mit Chitosan, welches zu 80% deacetyliert und zu 30% mit Chlorogensäure gepfropft ist, und unlöslich ist im pH Bereich von 6 bis 7. Die Herstellung der Chitosanlösung erfolgt ansonsten entsprechend der ersten Beschichtung.

Die Schichtstärke beträgt 6mg/cm².

Danach erfolgt eine Beschichtung mit Chitosan, welches zu 50% mit Chlorogensäure gepfropft ist, und unlöslich ist im pH Bereich von 5,5 bis 6,5, wie die entsprechende Beschichtung in Beispiel 6.

Sodann werden die beschichteten Pellets nochmals beschichtet mit einem Enteric Coating basierend auf Eudragit L 100 (Prozess wie die dritte Beschichtung in Beispiel 5).

Die so beschichteten Pellets werden in Hartgelatinekapseln abgefüllt.

Die Freisetzung des Wolframs erfolgt vollständig im Dickdarm, selbst wenn die interindividuelle Variabilität des pH Wertes im Dünndarm der Zielgruppe 1,5 pH Stufen beträgt (pH 6 bis pH 7,5), der Abfall des pH Wertes nach Passage der Ileozäkalklappe aber nur maximal 1 pH Stufe.

Schwankungen des pH Wertes innerhalb des Dünndarms um bis zu 0,4 pH Stufen beeinträchtigen die Sicherheit der Freisetzung nicht.

Ohne die "weiteren Schichten" wäre bei Erfordernis der gleichen Variabilitätstoleranz und des gleichen maximal erforderlichen pH Abfalls eine sichere Freisetzung im Dickdarm nur bei Individuen möglich, deren pH Wert vor dem Erreichen der Ileozäkalklappe zwischen pH 6 und pH 6,5 liegt, bzw. es müsste ein stärkerer pH Abfall nach der Passage der Ileozäkalklappe erreicht oder die Variabilitätstoleranz niedriger angesetzt werden.

Durch weitere Zwischenschichten "weitere Schichten" mit entsprechend modifizierten Polymeren und Verwendung anderer Schlussbeschichtungen (z.B. Eudragit L 30 D-55) kann die tolerierte interindividuelle Variabilität des pH Wertes vor dem Erreichen der Ileozäkalklappe weiter vergrößert werden, ohne dass ein stärkerer Abfall des pH-Wertes bei der Passage der Ileozäkalklappe erforderlich ist.

### Beispiel 8a:

Prednisolonhaltige Mikropellets werden zusammen mit Mikropellets aus Chitosan zu einer Matrix verpresst. Die Chitosan-Mikropellets werden dazu durch Sprühtrocknung einer 4%igen Chitosanlösung (mit Essigsäure auf einen pH-Wert von 5,2 eingestellt) hergestellt. Die Matrixpartikel werden zu Granulat mit Partikelgrößen zwischen 0,25 und 0,35mm vermahlen. Danach wird in einer wässrigen Lösung mit einem pH-Wert von 7,5 das Prednisolon, welches nicht komplett in der Matrix eingeschlossen ist, innerhalb 90 Minuten herausgelöst.

Die Granulatpartikel werden sodann mit Granulat gleicher Partikelgröße aus Chitosan, welches zu 30% mit Chlorogensäure gepfropft ist, und im Bereich zwischen pH 5 und pH 7 beständig gegenüber wässrigen Lösungen ist, zu Tabletten verpresst, welche anschließend mit Eudragit L 100 beschichtet werden (Prozess wie die dritte Beschichtung in Beispiel 5).

Abschließend erfolgt eine letzte Beschichtung mit Kollicoat Smartseal 30D wie die letzte Beschichtung aus Beispiel 5, allerdings mit 2% mehr Weichmacher.

Bei Freisetzungsversuchen ergibt sich eine Freisetzung von 65% des Prednisolons nach der simulierten Passage durch die Ileozäkalklappe.

### Beispiel 8b:

Prednisolonhaltige Mikropellets werden wie in Beispiel 8a verarbeitet. Vor der Beschichtung mit dem Eudragit L 100 werden die Tabletten allerdings für 30 Minuten in einer wässrigen Lösung mit einem pH Wert von 5,8 gewaschen und wieder getrocknet.

Bei Freisetzungsversuchen ergibt sich eine Freisetzung von mehr als 90% des Prednisolons nach der simulierten Passage durch die Ileozäkalklappe. 30% des Prednisolons werden vor Erreichen der ersten Flexur des Dickdarms freigesetzt.

### Beispiel 9a:

Wolframbeschichtete Pellets werden beschichtet mit Chitosan. Prozess wie bei der ersten Schicht bei Beispiel 5.

Sodann werden die beschichteten Pellets nochmals beschichtet mit einer Dispersion bestehend aus einer Mischung von Dispersionspartikeln, von denen 50% löslich sind bei einem pH Wert von über 7 und 50% löslich bei einem pH Wert von unter 5,5.

Die Sprühsuspension ist folgendermaßen zusammengesetzt:
Teil 1:
   1,2% Triethylcitrat und 7,5% Talkum werden in 58,3% Wasser aufgelöst, sodann wird 33% Kollicoat Smartseal 30D eingerührt. Die Suspension wird mittels NH3, oder Zitronensäure auf einen pH-Wert von 6,2 eingestellt.
Teil 2:
   1,2% Triethylcitrat und 7,5% Talkum werden in 58,3% Wasser aufgelöst, sodann wird 33% Eudragit FS 30D eingerührt. Die Suspension wird mittels NH3, NaOH oder Zitronensäure auf einen pH-Wert von 6,2 eingestellt.

Die beiden Lösungen werden 30 Minuten vor Beginn des Sprühvorganges unter langsamem Rühren vermischt und für diese 30 Minuten weiter langsam gerührt.

Die Schichtdicke wird eingestellt auf 4,5mg/cm².

Sodann werden die beschichteten Kapseln nochmals beschichtet mit einem Enteric Coating basierend auf Eudragit L 100 (Prozess wie die dritte Beschichtung in Beispiel 5).

Die so beschichteten Pellets werden in Hartgelatinekapseln abgefüllt.

### Beispiel 9b:

Wolframbeschichtete Pellets werden beschichtet mit Chitosan (Prozess wie bei der ersten Schicht bei Beispiel 5).

Danach werden die Pellets beschichtet mit einer zitronensäurehaltigen HPMC-Schicht. Die wasserbasierte Sprühlösung ist zusammengesetzt aus 2,8% Pharmacoat 606 und 1,2% Talkum, wobei Zitronensäure zugesetzt wird, bis ein pH Wert von 5,0 erreicht ist.

Die Beschichtungsstärke ist 4,2mg/cm². Die Sprühmenge pro Zeit wird so niedrig eingestellt, dass die Chitosanschicht nicht wesentlich angelöst wird.

Sodann werden die beschichteten Pellets nochmals beschichtet mit einer Dispersion bestehend aus einer Mischung von Dispersionspartikeln, von denen ca. 50% löslich sind bei einem pH Wert von über 7 und ca. 50% löslich bei einem pH Wert von unter 5,5 analog Beispiel 9a.

Sodann werden die beschichteten Kapseln nochmals beschichtet mit einem Enteric Coating basierend auf Eudragit L 100 (Prozess wie die dritte Beschichtung in Beispiel 5).

Die so beschichteten Pellets werden in Hartgelatinekapseln abgefüllt.

Das Wolfram wird nicht vor Erreichen der Ileozäkalklappe freigesetzt. Die Freisetzung erfolgt nach der Passage der Ileozäkalklappe. Mehr als 50% werden vor der ersten Flexur freigesetzt.

### Beispiel 9c:

Wolframbeschichtete Pellets werden beschichtet mit Chitosan (Prozess wie bei der ersten Schicht bei Beispiel 5).

Danach werden die Pellets beschichtet mit einer zitronensäurehaltigen HPMC-Schicht. Die wasserbasierte Sprühlösung ist zusammengesetzt aus 2,8% Pharmacoat 606 und 1,2% Talkum, wobei Zitronensäure zugesetzt wird, bis ein pH Wert von 5,0 erreicht ist.

Die Beschichtungsstärke ist 4,2mg/cm². Die Sprühmenge wird so niedrig eingestellt, dass die Chitosanschicht nicht wesentlich angelöst wird.

Sodann werden die beschichteten Pellets nochmals beschichtet in einem Trockenbefilmungsprozess.

Hierzu werden 40% Hydroxypropyl-Methylcellulose-Acetat-Succinat (HPMCAS; Shin-Etsu AQOAT®, Typ AS-HF; Shin-Etsu Chemical Co., Ltd., Niigata, Japan) und 40% Eudragit EPO mit 20% Talkum vermischt und in einem Wirbelschichtverfahren (Flowcoater FLO-5) von unten auf die Pellets aufgebracht, während die Pellets gleichzeitig aus einer anderen Düse von oben mit einer Weichmachermischung besprüht werden. Der Weichmacher besteht aus einer Mischung von 60% Triethylcitrat und 40% acetyliertem Monoglycerid (Myvacet®, Typ 9-45,
Eastman, TN, USA). Die zugeführte Menge der Weichmachermischung beträgt 50 Gewichtsprozent der Gesamtpolymermenge. Die Schichtdicke wird auf 8,5mg/cm² eingestellt.

Anschließend wird die Schicht ebenfalls in einem Wirbelschichtverfahren mit Wasser besprüht. Die Wassermenge beträgt 9% des Produktgewichtes. Danach werden die Pellets bei 60° für 20 Minuten in einem Ofen thermisch nachbehandelt, um die Filmbildung abzuschließen.

Sodann werden die beschichteten Kapseln nochmals beschichtet mit einem Enteric Coating basierend auf Eudragit L 100 (Prozess wie die dritte Beschichtung in Beispiel 5).

Die so beschichteten Pellets werden in Hartgelatinekapseln abgefüllt.

Die Kapseln sind bestimmt zur oralen Verabreichung, wobei sich die Hartgelatinekapsel im Magen auflöst, das Enteric Coating im Dünndarm, nachdem der PH-Wert über 6 gestiegen ist, die weitere Schicht wenn der pH Wert unter 5 sinkt, oder über 6,5 steigt, und die zuerst aufgebrachte Schicht schließlich im Zäkum, sobald der PH-Wert wieder unter 6,5 fällt.

Ein Test in verschiedenen Lösungen, welche die Bedingungen im Gastrointestinaltrakt simulieren, bestätigt, dass die beschichteten Pellets beständig sind (in dem Sinne, dass der Wirkstoff nicht freigesetzt wird) in künstlichem Magensaft (PH 3), künstlichem Duodenalsaft (PH 5), künstlichem Jejunalsaft (6,3) und künstlichem Ilealsaft (PH 7), sowohl bei Tests mit den einzelnen Flüssigkeiten, als auch bei Testung aller Flüssigkeiten in der angegebenen Reihenfolge.

Wird die beschichtete Kapsel nach einer Verweilzeit von 20 Minuten im Jejunal- oder Ilealsaft in ein Medium mit einem um mehr als 1 Einheiten niedrigeren PH-Wert verbracht (z.B. in ein künstliches Blinddarm- oder Dickdarmmilieu (PH 6)), so wird der wirkstoffhaltige Kapselinhalt innerhalb von 25 Minuten freigesetzt.

### Beispiel 9d:

Wolframbeschichtete Pellets werden beschichtet mit Chitosan (Prozess wie bei der ersten Schicht bei Beispiel 5).

Danach werden die Pellets beschichtet mit einer zitronensäurehaltigen HPMC-Schicht. Die wasserbasierte Sprühlösung ist zusammengesetzt aus 2,8% Pharmacoat 606 und 1,2% Talkum, wobei Zitronensäure zugesetzt wird, bis ein pH Wert von 5,0 erreicht ist.

Die Beschichtungsstärke ist 4,2mg/cm². Die Sprühmenge wird so niedrig eingestellt, dass die Chitosanschicht nicht wesentlich angelöst wird.

Sodann werden die beschichteten Pellets nochmals beschichtet in einem Trockenbefilmungsprozess.

Hierzu werden 40% Hydroxypropyl-Methylcellulose-Acetat-Succinat (HPMCAS; Shin-Etsu AQOAT®, Typ AS-HF; Shin-Etsu Chemical Co., Ltd., Niigata, Japan) und 40% Kollicoat Smartseal 30D (letzteres per Sprühtrocknung der verdünnten Polymerdispersion zu mikronisiertem Pulver verarbeitet) mit 20% Talkum vermischt und in einem Wirbelschichtverfahren (Flowcoater FLO-5) von unten auf die Pellets aufgebracht, während die Pellets gleichzeitig aus einer anderen Düse von oben mit einer Weichmachermischung besprüht werden. Der Weichmacher besteht aus einer Mischung von 60% Triethylcitrat und 40% acetyliertem Monoglycerid (Myvacet®, Typ 9-45, Eastman, TN, USA). Die zugeführte Menge der Weichmachermischung beträgt 50 Gewichtsprozent der Gesamtpolymermenge.

Die Schichtdicke wird auf 9mg/cm² eingestellt.

Anschließend wird die Schicht ebenfalls in einem Wirbelschichtverfahren bei einer Produkttemperatur von 50° für 20 Minuten mit Wasser besprüht. Die Wassermenge beträgt 9% des Produktgewichtes.

Sodann werden die beschichteten Kapseln nochmals beschichtet mit einem Enteric Coating basierend auf Eudragit L 100 (Prozess wie die dritte Beschichtung in Beispiel 5).

Die so beschichteten Pellets werden in Hartgelatinekapseln abgefüllt.

### Beispiel 9e:

Wolframbeschichtete Pellets werden beschichtet mit Chitosan (Prozess wie bei der ersten Schicht bei Beispiel 5).

Sodann werden die beschichteten Pellets nochmals beschichtet mit einer Dispersion bestehend aus einer Mischung von Dispersionspartikeln, von denen 50% löslich sind bei einem pH Wert von über 7 und 50% löslich bei einem pH Wert von unter 5,5.

Die Schicht wird durch gleichzeitiges Aufsprühen von zwei Dispersionen aus zwei separaten Sprühdüsen realisiert.

Die Sprühsuspensionen sind folgendermaßen zusammengesetzt:
Suspension 1:
   1,8% Triethylcitrat und 7,5% Talkum werden in 57,7% Wasser aufgelöst, sodann wird 33% Kollicoat Smartseal 30D eingerührt.
Suspension 2:
   1,2% Triethylcitrat und 7,5% Talkum werden in 58,3% Wasser aufgelöst, sodann wird 33% Eudragit FS 30D eingerührt.

Die Schichtdicke wird eingestellt auf 4,5mg/cm².

Sodann werden die beschichteten Pellets nochmals beschichtet mit einem Enteric Coating basierend auf Eudragit L 100 (Prozess wie die dritte Beschichtung in Beispiel 5).

Die so beschichteten Pellets werden in Hartgelatinekapseln abgefüllt.

### Beispiel 9f:

Wolframbeschichtete Pellets werden beschichtet mit Chitosan (Prozess wie bei der ersten Schicht bei Beispiel 5).

Sodann werden die beschichteten Pellets nochmals beschichtet mit einer Dispersion bestehend aus einer Mischung von Dispersionspartikeln, von denen 50% löslich sind bei einem pH Wert von über 7 und 50% löslich bei einem pH Wert von unter 5,5.

Die Schicht wird durch gleichzeitiges Aufsprühen von zwei Dispersionen aus zwei separaten Sprühdüsen realisiert.

Die Sprühsuspensionen sind folgendermaßen zusammengesetzt:
Suspension 1:
   1,2% Triethylcitrat, 3,5% Polysorbat 80 und 7,5% Talkum werden in 54,8% Wasser aufgelöst, sodann wird 33% Kollicoat Smartseal 30D eingerührt.
Suspension 2:
   1% Triethylcitrat, 3,5% Polysorbat 80 und 7,5% Talkum werden in 55% Wasser aufgelöst, sodann wird 33% Eudragit FS 30D eingerührt.

Die Schichtdicke wird eingestellt auf 5,2mg/cm².

Sodann werden die beschichteten Pellets nochmals beschichtet mit einem Enteric Coating basierend auf Eudragit L 100 (Prozess wie die dritte Beschichtung in Beispiel 5).

Die so beschichteten Pellets werden in Hartgelatinekapseln abgefüllt.

### Beispiel 10:

Prednisolonhaltige Mikrotabletten werden beschichtet mit Chitosan (Prozess wie bei der ersten Schicht bei Beispiel 5).

Sodann werden die beschichteten Mikrotabletten flach auf einem feinen Gitterrost angeordnet und aus oberhalb der Tabletten angeordneten Sprühdüsen besprüht mit einer Sprühsuspension aus 43% Eudragit FS 30D, 6,45% Talkum, 0,65% Triethylcitrat und als restlichem Inhaltsstoff Wasser. Durch das Gitter hindurch erfolgt von unten eine gleichzeitige Trockenluftzufuhr mit einer Temperatur von 55°. Die Sprühmenge pro Zeiteinheit wird so eingestellt, dass das gelöste Polymer an der Oberseite und an den Seitenflächen der Mikrotabletten trocknet, bevor es an den Seitenflächen zur Unterseite herunter laufen kann.

Nach vollständiger Beschichtung der Ober- und Seitenflächen werden die Mikrotabletten gewendet, und der Beschichtungsvorgang mit der Kollicoat Smartseal 30D-Lösung aus Beispiel 5 in entsprechender Schichtstärke (4,5mg/cm²) wiederholt. Die Sprühmenge pro Zeiteinheit wird so eingestellt, dass ein Verlaufen der Suspension auf die Unterseite der Mikrotabletten vermieden wird.

Danach werden die Mikrotabletten mit Eudragit L100 beschichtet (Prozess wie die dritte Beschichtung in Beispiel 5) und in Hartgelatinekapseln abgefüllt.

### Beispiel 11:

Prednisolonhaltige Mikrotabletten (Körper W, Zeichnung 6) werden beschichtet mit einem Poly(MMA-DEAEMA)-Copolymer. Das Copolymer und die daraus hergestellte Sprühlösung, sowie der Beschichtungsprozess entsprechen der letzten Schicht aus Beispiel 5, wobei bei der Herstellung des Copolymers der Anteil an DEAEMA gegenüber dem standardmäßigen Kollicoat Smartseal 30D erhöht ist, so dass das Copolymer bereits bei Unterschreitung eines pH-Wertes von 6,0 löslich wird (Schicht C, Zeichnung 6).

Sodann werden die beschichteten Mikrotabletten flach auf einem feinen Gitterrost angeordnet und aus oberhalb der Tabletten angeordneten Sprühdüsen mit folgender Sprühlösung besprüht: 25% Eudragit L 12,5, 25% Eudragit S 12,5, 3,1% Talkum, 0,6% Triethyl citrate, 46,3% Isopropanol (97%ig)

Durch das Gitter hindurch erfolgt von unten eine gleichzeitige Trockenluftzufuhr mit einer Temperatur von 55°. Die Sprühmenge pro Zeiteinheit wird so eingestellt, dass das gelöste Polymer an der Oberseite und an den Seitenflächen der Mikrotabletten trocknet, bevor es an den Seitenflächen zur Unterseite herunter laufen kann (Schicht C1a, Zeichnung 6). Die Beschichtungsstärke ist 5mg/cm². Diese Beschichtung ist löslich bei einem PH-Wert von über 6,5.

Nach vollständiger Beschichtung der Ober- und Seitenflächen werden die Mikrotabletten gewendet, und der Beschichtungsvorgang mit der Kollicoat Smartseal 30D-Lösung aus Beispiel 5 in entsprechender Schichtstärke (4,5mg/cm²) wiederholt. Die Sprühmenge pro Zeiteinheit wird so eingestellt, dass ein Verlaufen der Suspension auf die Unterseite der Mikrotabletten vermieden wird (Schicht C1b, Zeichnung 6).

Danach werden die Mikrotabletten mit Eudragit L 100 beschichtet (Prozess wie die dritte Beschichtung in Beispiel 5, Schicht E, Zeichnung 6) und in Hartgelatinekapseln abgefüllt.

### Beispiel 12:

Prednisolonhaltige Mikrotabletten werden beschichtet und verkapselt wie in Beispiel 11 beschrieben. Allerdings werden die Mikrotabletten nach der ersten Beschichtung (vor der Anordnung auf dem Gitterrost) beschichtet mit einer Schicht aus 85% HPMC (Pharmacoat 606), 5% Talkum und 10% Croscarmellose.

Diese Schicht weist bei Kontakt mit wässrigen Lösungen ein sehr starkes Quellvermögen auf.

### Beispiel 13:

Prednisolonhaltige Mikropellets werden beschichtet wie bei der ersten Beschichtung in Beispiel 11. Die Schichtstärke wird allerdings um 50% erhöht, und der Anteil an Tributylcitrat verdoppelt.

Sodann werden 20% beschichtete prednisolonhaltige Mikropellets mit 40% Hydroxypropyl-Methylcellulose-Acetat-Succinat Pulver und 40% Kollicoat Smartseal 30D (letzteres per Sprühtrocknung der verdünnten Polymerdispersion zu Pulver verarbeitet) vermischt und zu Mikrotabletten verpresst, welche anschließend mit Eudragit L 100 beschichtet werden (Prozess wie die dritte Beschichtung in Beispiel 5).

Abschließend werden die Mikrotabletten in Hartgelatinekapseln gefüllt.

### Beispiel 14:

Kapseln der Größe 1 aus Hydroxypropylmethylcellulose (HPMC), die mit Mesalazin gefüllt sind, werden mit der Dip-Methode beschichtet mit einer achtprozentigen wässrigen HPMC-Lösung. Dazu werden die Kapseln zu zwei Dritteln in die HPMC-Lösung getaucht und dann in einem 40 Grad warmen Luftstrom getrocknet, bis sie bei Berührung nicht mehr kleben. Sodann werden sie mit der gegenüberliegenden Seite zu zwei Dritteln in die HPMC-Lösung getaucht, um eine überlappende Schichtbildung zu gewährleisten. Anschließend erfolgt ebenfalls eine Trocknung im warmen Luftstrom, und dann bei Raumtemperatur für 24 Stunden.

Danach werden die Kapseln mit der gleichen Methode aber nur einem einseitigen Dip-Vorgang zwei weitere Male zu zwei Dritteln mit einer HPMC-Lösung beschichtet, welche zusätzlich noch 5% Eudragit RS enthält, und danach nochmals die gleichen zwei Drittel einmal mit Eudragit FS 30D.

Sie werden dann auf der gegenüberliegenden Seite ebenfalls zu zwei Dritteln mit Kollicoat Smartseal 30D beschichtet, so dass die beiden Beschichtungen leicht überlappen. Dem Kollicoat Smartseal 30D werden zuvor 4,5% Triethylcitrat (15% bezogen auf das Polymergewicht) zugemischt.

Anschließend werden die so beschichteten Kapseln für 24 Stunden bei Raumtemperatur getrocknet.

Danach werden die Kapseln mit der gleichen Methode wie bei der ersten Beschichtung mit Kollicoat MAE 30DP beschichtet, dem 5% Triethylcitrat zugemischt wurde.

Abschließend werden die beschichteten Kapseln in Hartgelatinekapseln der Größe 00 gefüllt.

Der Wirkstoff wird weder im Magen freigesetzt, noch wenn nach dem Magen der pH Wert im Dünndarm auf pH 6,5 steigt. Erst wenn der pH Wert nach der Passage durch die Ileozäkalklappe auf unter pH 5,5 sinkt, oder der pH Wert gegen Ende der Dünndarmpassage auf über 7 steigt, wird das Mesalazin freigesetzt. Durch die mehrfache Unterbeschichtung der Eudragit FS 30D Schicht mit HPMC und Eudragit RS dauert die Auflösung nach Überschreitung von pH 7 so lange, dass die Wirkstofffreigabe erst nach der Passage der Ileozäkalklappe erfolgt.

### Beispiel 15:

Hartgelatinekapseln mit Probiotikafüllung werden beschichtet mit einem Poly(MMA-DEAEMA)-Copolymer. Das Copolymer und die daraus hergestellte Sprühlösung, sowie der Beschichtungsprozess entsprechen der letzten Schicht aus Beispiel 5, wobei bei der Herstellung des Copolymers der Anteil an DEAEMA gegenüber dem standardmäßigen Kollicoat Smartseal 30D erhöht ist, so dass das Copolymer bereits bei Unterschreitung eines pH-Wertes von 7 löslich wird.

500g der so beschichteten Hartgelatinekapseln werden in einen CF-Granulator gegeben, der auf 250 Umdrehungen pro Minute und 150 l/min Einlassluft mit 30° eingestellt wird. Eine Suspension aus 300g einer vierprozentigen Eudragit RS Lösung (12g Polymer in 288g Ethanol), 12g Chitosan-Pulver und 12g Eudragit S Pulver (jeweils mit einer mittleren Partikelgröße von 95µm), wird mit einer Zuführungsrate von 3,5 g/min auf das Kapselbett getropft.

Nach Trocknung der Schicht werden die beschichteten Kapseln nochmals beschichtet mit einem Enteric Coating basierend auf Eudragit L100 und Eudragit S100.

Zusammensetzung der Sprühlösung:
25% Eudragit L 12,5, 25% Eudragit S 12,5, 3,1% Talkum, 0,6% Triethyl citrate, 46,3% Isopropanol (97%ig)

Prozess Equipment:
Glatt GPGC 1.1 - top spray, Düse: Schlick 970/0

Setup:
Düsenöffnung: 1,2mm, Sprühdruck: 2 Bar, Filter Rüttelzeit: 5s, Filter Rüttelintervall: 30s, Zulufttemperatur: 33°,
Auslasstemperatur: 25°, Sprührate: 15g/min/kg, Differenzdruck Boden/Produkt: 850mbar, Differenzdruck Filter: 450mbar,
Trocknungsluftvolumen: 65m³/h, Trocknungszeit bei 40°: 2h
Die Beschichtungsstärke ist 5mg/cm². Diese Beschichtung ist löslich bei einem PH-Wert von über 6,5.

Danach erfolgt eine letzte Beschichtung mit Kollicoat Smartseal 30D wie die letzte Beschichtung aus Beispiel 5.

Die so beschichteten Kapseln geben ihren Inhalt frei, wenn der pH Wert nach der Magenpassage über 6,5 ansteigt, und danach wieder unter 6,5 fällt.

Erfolgt ein Anstieg des pH Wertes über 7, wird der Kapselinhalt auch dann freigesetzt, wenn der pH Wert danach nur unter einen Wert von 7 fällt.

### Beispiel 15b:

Kapseln werden wie in Beispiel 15 hergestellt, jedoch wird die Sprührate bei der ersten Beschichtung halbiert und es werden HPMC Kapseln verwendet. Die vierprozentige Eudragit RS Lösung wird durch eine mit Wasser auf 12% Polymergehalt verdünnte und auf pH 6,75 eingestellte Eudragit NE 30 D Dispersion ersetzt.

### Beispiel 16:

Hartgelatinekapseln mit Probiotikafüllung werden beschichtet mit einem Poly(MMA-DEAEMA)-Copolymer. Das Copolymer und die daraus hergestellte Sprühlösung, sowie der Beschichtungsprozess entsprechen der letzten Beschichtung aus Beispiel 5, wobei bei der Herstellung des Copolymers der Anteil an DEAEMA gegenüber dem standardmäßigen Kollicoat Smartseal 30D erhöht ist, so dass das Copolymer bereits bei Unterschreitung eines pH-Wertes von 7 löslich wird.

500g der so beschichteten Hartgelatinekapseln werden in einen CF-Granulator gegeben, der auf 250 Umdrehungen pro Minute und 150 l/min Einlassluft mit 30° eingestellt wird. Eine Lösung aus 600g einer zweiprozentigen Chitosan Lösung (12g Polymer und 10g Essigsäure in 578g Wasser) wird mit einer Zuführungsrate von 6 g/min auf das Kapselbett getropft. Gleichzeitig werden 12g Eudragit S Pulver mit einer mittleren Partikelgröße von 95µm, das zuvor im Wirbelschichtcoater mit einer 10µm starken Schicht Eudragit RS beschichtet wurde, mit einer Zuführungsrate von 120mg/Minute auf das Kapselbett verteilt.

Nach Trocknung der Schicht werden die beschichteten Kapseln mit einen Enteric Coating beschichtet (analog Beispiel 15) und danach erfolgt eine letzte Beschichtung mit Kollicoat Smartseal 30D wie die letzte Beschichtung aus Beispiel 5.

Die so beschichteten Kapseln geben ihren Inhalt frei, wenn der pH Wert nach der Magenpassage über 6,5 ansteigt, und danach wieder unter 6,5 fällt.

Erfolgt ein Anstieg des pH Wertes über 7, wird der Kapselinhalt auch dann freigesetzt, wenn der pH Wert danach nur unter einen Wert von 7 fällt.

### Beispiel 17:

Carboxymethylcellulose-Microspheres mit einem mittleren Durchmesser von 50µm werden beschichtet mit Eudragit FS 30D. Die Schichtstärke wird auf 25µm eingestellt.

Carboxymethylcellulose-Microspheres mit einem mittleren Durchmesser von 50µm werden beschichtet mit Kollicoat Smartseal 30D. Die Schichtstärke wird auf 25µm eingestellt.

Hartgelatinekapseln mit Probiotikafüllung werden beschichtet mit einem Poly(MMA-DEAEMA)-Copolymer. Das Copolymer und die daraus hergestellte Sprühlösung, sowie der Beschichtungsprozess entsprechen der letzten Beschichtung aus Beispiel 5, wobei bei der Herstellung des Copolymers der Anteil an DEAEMA gegenüber dem standardmäßigen Kollicoat Smartseal 30D erhöht ist, so dass das Copolymer bereits bei Unterschreitung eines pH-Wertes von 6,5 löslich wird.

500g der so beschichteten Hartgelatinekapseln werden in einen CF-Granulator gegeben, der auf 250 Umdrehungen pro Minute und 150 l/min Einlassluft mit 30° eingestellt wird. Eine Suspension aus 300g einer vierprozentigen Eudragit RS Lösung (12g Polymer in 288g Ethanol), 12g mit Kollicoat Smartseal beschichtete Mikropellets und 12g mit Eudragit FS 30D beschichtete Mikropellets, wird mit einer Zuführungsrate von 3,5 g/min auf das Kapselbett getropft.

Nach Trocknung der Schicht werden die beschichteten Kapseln mit einen Enteric Coating beschichtet (analog der dritten Beschichtung aus Beispiel 5) und danach erfolgt eine letzte Beschichtung mit Kollicoat Smartseal 30D wie die letzte Beschichtung aus Beispiel 5.

Die so beschichteten Kapseln geben ihren Inhalt frei, wenn der pH Wert nach der Magenpassage über 6 ansteigt, und danach wieder unter pH 5,5 fällt.

Erfolgt ein Anstieg des pH Wertes über 7, wird der Kapselinhalt auch dann freigesetzt, wenn der pH Wert danach nur unter einen Wert von 6,5 fällt.

### Beispiel 18:

Hartgelatinekapseln mit Probiotikafüllung werden beschichtet mit Chitosan, entsprechend der ersten Beschichtung aus Beispiel 5. 500g der so beschichteten Hartgelatinekapseln werden in einen CF-Granulator gegeben, der auf 250 Umdrehungen pro Minute und 150 l/min Einlassluft mit 30° eingestellt wird. Eine Suspension aus 300g einer vierprozentigen Eudragit RS Lösung (12g Polymer in 288g Ethanol), 12g mit Kollicoat Smartseal beschichtete Mikropellets und 12g mit Eudragit FS 30D beschichtete Mikropellets, wird mit einer Zuführungsrate von 3,5 g/min auf das Kapselbett getropft.

Nach Trocknung der Schicht werden die beschichteten Kapseln mit einen Enteric Coating beschichtet (analog der dritten Beschichtung aus Beispiel 5) und danach erfolgt eine letzte Beschichtung mit Kollicoat Smartseal 30D wie die letzte Beschichtung aus Beispiel 5.

Die so beschichteten Kapseln geben ihren Inhalt frei, wenn der pH Wert nach der Magenpassage über 6 ansteigt, und danach wieder unter pH 5,5 fällt.

Erfolgt ein Anstieg des pH Wertes über 7, wird der Kapselinhalt auch dann freigesetzt, wenn der pH Wert danach nur unter einen Wert von 6,5 fällt.

### Beispiel 19:

Kapselböden (im Stand der Technik teilweise auch als Kapselkörper bezeichnet) der Größe 0 werden mit einer reduzierten Wandstärke von 60µm hergestellt und wie in US 2011/0033530 A1 beschrieben beschichtet mit einer 40µm dicken funktionalen Schicht (analog Beispiel 3 des genannten Dokuments). Hierzu verbleiben sie an dem Tauchstift. Allerdings wird statt Eudragit L 100-55 als funktionales Polymer Eudragit L 100 eingesetzt (Methacrylsäure - Methylmethacrylat Copolymer (1:2)), so dass die Schicht nicht oberhalb von pH 5,5 sondern erst oberhalb von pH 6,0 löslich wird. Der Zusatz von Triethylcitrat wird von 10% auf 13% erhöht. Die Kapselböden werden bei der Herstellung der funktionalen Beschichtung allerdings nur so weit eingetaucht, dass die funktionale Beschichtung sich nach dem Zusammenstecken mit einer Kapselkappe zwei Millimeter unter die Kapselkappe erstreckt.

Kapselböden der Größe 1 werden hergestellt und beschichtet wie vorab beschrieben. Allerdings wird statt Eudragit L 100 als funktionales Polymer ein Methacrylsäure - Methylmethacrylat Copolymer (1:1,5) entsprechend Eudragit S100 bzw. Eudragit L 100, aber mit einem zwischen diesen beiden Copolymeren liegenden Monomerverhältnis, eingesetzt, so dass die Schicht erst oberhalb von pH 6,5 löslich wird.

Kapselböden der Größe 2 werden hergestellt und beschichtet wie vorab beschrieben. Allerdings wird statt Eudragit L 100 als funktionales Polymer Eudragit S100 eingesetzt (Methacrylsäure - Methylmethacrylat Copolymer (1:2)), so dass die Schicht erst oberhalb von pH 7,0 löslich wird.

Kapselkappen der Größe 1 werden mit einer reduzierten Wandstärke von 60µm hergestellt und wie in US 2011/0033530 A1 beschrieben beschichtet mit einer 40µm dicken funktionalen Polymerschicht (analog zu Beispiel C6 des genannten Dokuments). Allerdings wird statt Eudragit FS 30D als funktionales Polymer Kollicoat Smartseal 30 D eingesetzt. Aufgrund der geringen Viskosität der Dispersion wird die Schicht in mehreren Tauchgängen erzeugt.

Kapselkappen der Größe 0 werden hergestellt und beschichtet wie vorab beschrieben. Allerdings wird statt Kollicoat Smartseal 30 D als funktionales Polymer ein Polymer eingesetzt, das wie Kollicoat Smartseal 30 D hergestellt wird, wobei allerdings ein erniedrigter Anteil an Diethylaminoethylmethacrylat-Monomeren eingesetzt wird, so dass das Polymer erst unterhalb von pH 5 löslich wird.

Kapselkappen der Größe 2 werden hergestellt und beschichtet wie vorab beschrieben. Allerdings wird statt Kollicoat Smartseal 30 D als funktionales Polymer ein Polymer eingesetzt, das wie Kollicoat Smartseal 30 D hergestellt wird, wobei allerdings ein erhöhter Anteil an Diethylaminoethylmethacrylat-Monomeren eingesetzt wird, so dass das Polymer schon unterhalb von pH 6 löslich wird.

Kapselböden und Kapselkappen der Größe 3 werden hergestellt und beschichtet wie vorab beschrieben. Allerdings wird statt Kollicoat Smartseal 30 D als funktionales Polymer ein Polymer eingesetzt, das wie Kollicoat Smartseal 30 D hergestellt wird, wobei allerdings ein erhöhter Anteil an Diethylaminoethylmethacrylat-Monomeren eingesetzt wird, so dass das Polymer schon unterhalb von pH 6,5 löslich wird.

Kapselböden und Kapselkappen der Größe 00 werden mit einer reduzierten Wandstärke von 60µm hergestellt und wie in US 2011/0033530 A1 beschrieben beschichtet mit einer 40µm dicken Schicht aus Eudragit L 100-55 und Eudragit NE 30 D (Beispiel 1 des genannten Dokuments). Die funktionale Schicht wird oberhalb von pH 5,5 löslich.

Die beschichteten Kapselböden der Größe 3 werden mit einem Wirkstoff, beispielsweise 250mg Mesalazin, befüllt und mit den beschichteten Kapselkappen der Größe 3 verschlossen. Der Spalt zwischen den Kapselhälften wird mit der gleichen Polymerdispersion, mit der die Kapselkappe beschichtet wurde, versiegelt. Hierzu wird diese in den Spalt dispenst und getrocknet.

Die Kapseln der Größe 3 werden in beschichtete Kapselböden der Größe 2 gesteckt, und diese mit beschichteten Kapselkappen der Größe 2 verschlossen. Der Spalt zwischen den Kapselhälften wird mit der gleichen Polymerdispersion, mit der die Kapselkappe beschichtet wurde, versiegelt. Hierzu wird diese in den Spalt dispenst und getrocknet.

Die Kapseln der Größe 2 werden in beschichtete Kapselböden der Größe 1 gesteckt, und diese mit beschichteten Kapselkappen der Größe 1 verschlossen. Der Spalt zwischen den Kapselhälften wird mit der gleichen Polymerdispersion, mit der die Kapselkappe beschichtet wurde, versiegelt. Hierzu wird diese in den Spalt dispenst und getrocknet.

Die Kapseln der Größe 1 werden in beschichtete Kapselböden der Größe 0 gesteckt, und diese mit beschichteten Kapselkappen der Größe 0 verschlossen. Der Spalt zwischen den Kapselhälften wird mit der gleichen Polymerdispersion, mit der die Kapselkappe beschichtet wurde, versiegelt. Hierzu wird diese in den Spalt dispenst und getrocknet.

Die Kapseln der Größe 0 werden in beschichtete Kapselböden der Größe 00 gesteckt, und diese mit beschichteten Kapselkappen der Größe 00 verschlossen. Der Spalt zwischen den Kapselhälften wird mit der gleichen Polymerdispersion, mit der die Kapselkappe beschichtet wurde, versiegelt. Hierzu wird diese in den Spalt dispenst und getrocknet.

Die Kapseln der Größe 00 werden in Kapselböden der Größe 000 gesteckt, und diese mit Kapselkappen der Größe 000 verschlossen.

Die Kapseln setzen den Wirkstoff sicher im Dickdarm frei, wenn der Abfall des pH Wertes nach der Passage der Ileozäkalklappe mindestens eine pH Stufe beträgt, und der maximale pH Wert im Dünndarm zwischen 5,5 und 7,5 liegt.

Die üblicherweise verwendeten Kapselgrößen und ihre Maße sind dem Fachmann aus der einschlägigen Literatur bekannt. Beispielsweise sind diese in "Die Kapsel, Grundlagen, Technologie and Biopharmazie einer modernen Arzneiform", Fahrig W. and Hofer U. (1983), Wissenschaftliche Verlagsgesellschaft mbH Stuttgart, beschrieben.

Werden für die Beschichtung Polymere oder Copolymere eingesetzt, deren Grenzwerte (Schwellwerte) bei niedrigeren und/oder höheren pH Werten liegen, kann der Arbeitsbereich der Formulierung (der Bereich an pH Werten, innerhalb dessen die Freisetzung bei einem Abfall des pH Wertes um einen bestimmten Wert stattfindet) nach unten auf bis zu pH 2 und nach oben auf bis zu pH 9 ausgeweitet werden. Bevorzugt wird ein Arbeitsbereich von pH 3 bis 9 angestrebt, mehr bevorzugt von pH 4 bis pH 8, besonders bevorzugt von pH 5,5 bis pH 7,5.

Solche Änderungen des Arbeitsbereiches können auch bei den anderen Ausführungsbeispielen, Ausführungsformen und Varianten vorgenommen werden.

### Beispiel 20:

Prednisolonhaltige Mikrotabletten werden beschichtet mit Chitosan (Prozess wie bei der ersten Schicht bei Beispiel 5). Danach werden die Mikrotabletten auf einem feinen Gitterrost angeordnet und 30 Sekunden lang mit einem 0,05-molaren Phosphatpuffer mit einem pH Wert von 8 besprüht. Die Sprührate wird so eingestellt, dass die Tablettenoberfläche nach 4 Sekunden mit einem durchgehenden Flüssigkeitsfilm benetzt ist. Die Mikrotabletten werden gewendet und nochmals für 30 Sekunden besprüht. Danach werden sie für weitere 30 Sekunden auf beiden Seiten mit deionisiertem Wasser besprüht, mit der dreifachen Sprührate verglichen mit der Pufferlösung. Anschließend werden die Mikrotabletten mit einem warmen Luftstrom getrocknet.

Sodann werden die beschichteten Mikrotabletten flach auf einem feinen Gitterrost angeordnet und aus oberhalb der Tabletten angeordneten Sprühdüsen besprüht mit einer Sprühsuspension aus 43% Eudragit FS 30D, 6,45% Talkum, 0,65% Triethylcitrat und als restlichem Inhaltsstoff Wasser. Durch das Gitter hindurch erfolgt von unten eine gleichzeitige Trockenluftzufuhr mit einer Temperatur von 55°. Die Sprühmenge pro Zeiteinheit wird so eingestellt, dass das gelöste Polymer an der Oberseite und an den Seitenflächen der Mikrotabletten trocknet, bevor es über die Seitenflächen hinaus zur Unterseite herunter laufen kann. Die Beschichtungsstärke ist eingestellt auf 6mg/cm². Diese Beschichtung ist löslich bei einem PH-Wert von über 7.

Nach vollständiger Beschichtung der Ober- und Seitenflächen werden die Mikrotabletten gewendet, und der Beschichtungsvorgang mit der Kollicoat Smartseal 30D-Lösung aus Beispiel 5 in entsprechender Schichtstärke (4,5mg/cm²) wiederholt. Die Sprühmenge pro Zeiteinheit wird so eingestellt, dass ein Verlaufen der Dispersion auf die Unterseite der Mikrotabletten vermieden wird.

Danach werden die Mikrotabletten mit Eudragit L 100 beschichtet (Prozess wie die dritte Beschichtung in Beispiel 5) und in Hartgelatinekapseln abgefüllt.

### Beispiel 21:

Oblongtabletten der Größe 4x4x14mm werden mit der Dip-Methode beschichtet mit einer zweiprozentigen Chitosan-Lösung (2% Chitosan in eineinhalbprozentiger Essigsäure gelöst). Dazu werden die Kapseln zu zwei Dritteln in die Chitosan-Lösung getaucht und dann in einem 55 Grad warmen Luftstrom getrocknet, bis sie bei Berührung nicht mehr kleben. Dieser Vorgang wird dreimal wiederholt. Sodann werden sie mit dem gegenüberliegenden Ende zu zwei Dritteln in die Chitosan-Lösung getaucht, um eine überlappende Schichtbildung zu gewährleisten. Anschließend erfolgt ebenfalls eine Trocknung im warmen Luftstrom. Dieser Vorgang wird ebenfalls dreimal wiederholt. Danach erfolgt eine Trocknung bei Raumtemperatur für 24 Stunden.

Danach werden die Oblongtabletten für 140 Sekunden zu zwei Dritteln in einen 0,1-molaren Phosphatpuffer mit pH 7,8 getaucht und danach zum Entfernen der Reste der Pufferlösung in deionisiertes Wasser. Beide Flüssigkeiten befinden sich durch einen Magnetrührer in leichter Strömung. Anschließend erfolgt eine Trocknung im warmen Luftstrom.

Sodann erfolgt die gleiche Nachbehandlung am anderen Ende der Oblongtablette, ebenfalls zu zwei Dritteln, sodass sich die nachbehandelten Bereiche überlappen.

Nach einer anschließenden Trocknung über 24 Stunden werden die Oblongtabletten zu zwei Dritteln in Eudragit FS 30D (gemischt mit 10% Triethylcitrat bezogen auf die Polymermenge) getaucht und dann in einem 50 Grad warmen Luftstrom getrocknet, bis sie bei Berührung nicht mehr kleben.

Sie werden dann an dem gegenüberliegenden Ende ebenfalls zu zwei Dritteln in Kollicoat Smartseal 30D eingetaucht, so dass die beiden Beschichtungen leicht überlappen. Dem Kollicoat Smartseal 30D werden zuvor 4,5% Triethylcitrat (15% bezogen auf das Polymergewicht) zugemischt. Danach werden sie in einem 50 Grad warmen Luftstrom getrocknet, bis sie bei Berührung nicht mehr kleben.

Anschließend werden die so beschichteten Oblongtabletten für 24 Stunden bei Raumtemperatur getrocknet.

Danach werden die Oblongtabletten mit der gleichen Methode von beiden Enden her zu jeweils zwei Dritteln mit Kollicoat MAE 30DP beschichtet, dem 5% Triethylcitrat zugemischt wurde.

Abschließend werden die beschichteten Oblongtabletten in Hartgelatinekapseln der Größe 00 gefüllt.

Der Wirkstoff wird weder im Magen freigesetzt, noch wenn nach dem Magen der pH Wert im Dünndarm auf pH 6,5 steigt. Erst wenn der pH Wert nach der Passage durch die Ileozäkalklappe auf unter pH 5,5 sinkt, oder der pH Wert gegen Ende der Dünndarmpassage auf über 7 steigt, und danach nach der Passage durch die Ileozäkalklappe auf unter pH 6,5 sinkt wird der Wirkstoff der Oblongtablette freigesetzt

### Beispiel 22:

Kapselkappen der Größe 1 aus Gelatine oder vorzugsweise HPMC werden mit einer reduzierten Wandstärke von 50µm hergestellt und wie in US 2011/0033530 A1 beschrieben beschichtet mit einer 25µm dicken funktionalen Polymerschicht (analog zu Beispiel C6 des genannten Dokuments). Allerdings wird statt Eudragit FS 30D als funktionales Polymer Kollicoat Smartseal 30 D eingesetzt. Aufgrund der geringen Viskosität der Dispersion wird die Schicht in mehreren Tauchgängen erzeugt. Diese Schicht wird unterhalb von pH 5,5 löslich.

Sodann werden die Kapselkappen mit einer zweiten 25µm dicken funktionalen Schicht beschichtet (analog Beispiel 3 des genannten Dokuments). Allerdings wird statt Eudragit L 100-55 als funktionales Polymer Eudragit L 100 eingesetzt (Methacrylsäure - Methylmethacrylat Copolymer (1:2)), so dass die Schicht nicht oberhalb von pH 5,5 sondern erst oberhalb von pH 6,0 löslich wird. Der Zusatz von Triethylcitrat wird von 10% auf 13% erhöht. Erst nach den erfolgten Beschichtungsvorgängen werden die Kapselkappen von den Tauchstiften abgestreift.

Kapselböden (im Stand der Technik teilweise auch als Kapselkörper bezeichnet) der Größe 1 werden hergestellt und beschichtet wie vorab bei den Kapselkappen beschrieben. Allerdings wird statt Kollicoat Smartseal 30 D als funktionales Polymer ein Polymer eingesetzt, das wie Kollicoat Smartseal 30 D hergestellt wird, wobei allerdings ein erhöhter Anteil an Diethylaminoethylmethacrylat-Monomeren eingesetzt wird, so dass die erste Beschichtung schon unterhalb von pH 6,5 löslich wird.

Statt Eudragit L 100 wird als funktionales Polymer Eudragit S100 eingesetzt (Methacrylsäure - Methylmethacrylat Copolymer (1:2)), so dass die zweite Schicht erst oberhalb von pH 7,0 löslich wird.

Die beschichteten Kapselböden werden mit einem Wirkstoff, beispielsweise 400mg Mesalazin, befüllt und mit den beschichteten Kapselkappen verschlossen. Der Spalt zwischen den Kapselhälften wird mit einer nicht wasserlöslichen Polymerdispersion versiegelt. Hierzu wird diese in den Spalt dispenst und getrocknet. Beispielsweise können Eudragit RS, Eudragit NE oder Ethylcellulose eingesetzt werden.

Die Kapseln der Größe 1 werden in Kapselböden der Größe 0 gesteckt, und diese mit Kapselkappen der Größe 0 verschlossen.

Die Kapseln setzen den Wirkstoff sicher im Dickdarm frei, wenn der Abfall des pH Wertes nach der Passage der Ileozäkalklappe mindestens 1,5 pH Stufen beträgt, und der maximale pH Wert im Dünndarm zwischen 6 und 8 liegt.

### Beispiel 23:

Carboxymethylcellulose-Microspheres werden in drei Gruppen aufgeteilt.
Gruppe 1 wird beschichtet mit einem Poly(MMA-DEAEMA)-Copolymer (Poly(Methylmethacrylat-Diethylaminoethylmethacrylat)), das unterhalb von pH 5,5 löslich wird. Sodann wird eine Schicht aus einem Poly(MMA-AA)-Copolymer oder einem Poly(Methacrylsäure - Methylmethacrylat)-Copolymer aufgetragen, welches oberhalb von pH 5,8 löslich wird.
Gruppe 2 wird beschichtet mit einem Poly(MMA-DEAEMA)-Copolymer, das unterhalb von pH 6,0 löslich wird. Sodann wird eine Schicht aus einem Poly(MMA-AA)-Copolymer oder einem Poly(Methacrylsäure - Methylmethacrylat)-Copolymer aufgetragen, welches oberhalb von pH 6,3 löslich wird.
Gruppe 3 wird beschichtet mit einem Poly(MMA-DEAEMA)-Copolymer, das unterhalb von pH 6,5 löslich wird. Sodann wird eine Schicht aus einem Poly(MMA-AA)-Copolymer oder einem Poly(Methacrylsäure - Methylmethacrylat)-Copolymer aufgetragen, welches oberhalb von pH 6,8 löslich wird.

Bei den Beschichtungen kommen zusätzlich zu den Copolymeren noch Weichmacher, Antiklebemittel und ggf. weitere Hilfsstoffe zum Einsatz.

500g Hartgelatinekapseln der Größe 2 mit Wirkstofffüllung werden in einen CF-Granulator gegeben, der auf 250 Umdrehungen pro Minute und 150 l/min Einlassluft mit 30° eingestellt wird. 300g einer vierprozentigen Eudragit RS Lösung wird mit einer Zuführungsrate von 3,5 g/min auf das Kapselbett getropft. Gleichzeitig wird eine Mischung aus den drei Gruppen von beschichteten Microspheres (jeweils 8g, also insgesamt 24g Microspheres) mit einer Zuführungsrate von 280mg/Minute auf das Kapselbett verteilt.

Nach Trocknung der Schicht werden die beschichteten Kapseln in Hartgelatinekapseln der Größe 0 verbracht.

Die Kapseln setzen den Wirkstoff sicher im Dickdarm frei, wenn der Abfall des pH Wertes nach der Passage der Ileozäkalklappe mindestens 0,8 pH Stufen beträgt, und der maximale pH Wert im Dünndarm zwischen 5,8 und 7,3 liegt.

### Beispiel 24:

Wirkstoffhaltige planparallele Tabletten, Durchmesser 13mm, Höhe 3mm, Facette 0,5mm, werden zu Rollen gestapelt und zwischen zwei Haltescheiben an den Stirnseiten der Rollen gehalten. Dann werden die Rollen mit einer organischen Polymethylmethacrylat-Lösung (Aceton als Lösungsmittel) im Sprühverfahren beschichtet. Sprühmenge, Trockenluftmenge und Trockenlufttemperatur werden so eingestellt, dass sich an den Tablettenrändern ein gleichmäßiger Polymerfilm bildet, die Tablettenflächen jedoch weder beschichtet werden, noch so stark zusammenkleben, dass sie nicht mehr ohne Beschädigung getrennt werden könnten.

Nach dem Trennen aus den Rollenstapeln werden die so an ihren Randflächen wasserunlöslich beschichteten Tabletten flach auf einem feinen Gitterrost angeordnet. Die Oberseite der Tabletten wird mit einer Schattenmaske aus Edelstahlblech abgedeckt, die quadratische Aussparungen von 3mm mal 3mm in einem rechtwinkligen Wiederholungsraster von 5mm mal 5mm entlang seiner X- und Y-Achse aufweist. Zwischen den Aussparungen befinden sich also Stege von 2mm Breite.

Durch diese Aussparungen hindurch erfolgen Beschichtungsvorgänge mittels oberhalb der Schattenmaske angeordneter Sprühdüsen. Ebenfalls von oberhalb der Schattenmaske erfolgt die Zufuhr von 55°C warmer Trockenluft, welche hauptsächlich an den Tablettenzwischenräumen durch den feinen Gitterrost abgesaugt wird.

Zuerst erfolgt eine Beschichtung mit einem Poly(MMA-DEAEMA)-Copolymer, welches unterhalb von pH 6,8 löslich ist.

Nachdem die Schattenmaske um 2,5mm entlang ihrer X-Achse versetzt wurde, erfolgt eine Beschichtung mit einem Poly(MMA-DEAEMA)-Copolymer, welches unterhalb von pH 6,3 löslich ist.

Nachdem die Schattenmaske um 2,5mm entlang ihrer Y-Achse versetzt wurde, erfolgt eine Beschichtung mit einem Poly(MMA-DEAEMA)-Copolymer, welches unterhalb von pH 5,8 löslich ist.

Nachdem die Schattenmaske nochmals um 2,5mm entlang ihrer X-Achse versetzt wurde (vorzugsweise entgegen der vorherigen Versetzungsrichtung entlang der X-Achse), erfolgt eine Beschichtung mit einem Poly(MMA-DEAEMA)-Copolymer, welches unterhalb von pH 5,3 löslich ist.

Anschließend wird Schattenmaske nochmals um 2,5mm entlang ihrer Y-Achse versetzt vorzugsweise entgegen der vorherigen Versetzungsrichtung entlang der Y-Achse).

Sodann erfolgt eine Beschichtung mit einem Poly(Methacrylsäure - Methylmethacrylat)-Copolymer, welches oberhalb von pH 7 löslich ist.

Nachdem die Schattenmaske um 2,5mm entlang ihrer X-Achse versetzt wurde, erfolgt eine Beschichtung mit einem Poly(Methacrylsäure - Methylmethacrylat)-Copolymer, welches oberhalb von pH 6,5 löslich ist.

Nachdem die Schattenmaske um 2,5mm entlang ihrer Y-Achse versetzt wurde, erfolgt eine Beschichtung mit einem Poly(Methacrylsäure - Methylmethacrylat)-Copolymer, welches oberhalb von pH 6 löslich ist.

Nachdem die Schattenmaske entfernt wurde, erfolgt eine Beschichtung mit einem Poly(Methacrylsäure - Methylmethacrylat)-Copolymer, welches oberhalb von pH 5,5 löslich ist.

Anschließend werden die Tabletten mit einem Poly(MMA-DEAEMA)-Copolymer, welches unterhalb von pH 5,5 löslich ist, beschichtet.

Sodann werden die Tabletten gewendet und auf der gegenüberliegenden Seite in der gleichen Weise beschichtet, wie auf der ersten Seite.

Die Beschichtungsstärken werden jeweils auf 4,5g pro cm² eingestellt. Mittels Zusatz von Weichmachern wird sichergestellt, dass bei der verwendeten Trockenlufttemperatur eine zuverlässige Filmbildung erreicht wird.

Durch die Schattenmaskenöffnungen von 3mm im Raster von 5mm wird sichergestellt, dass die einzelnen Schichtfolgen sich geometrisch leicht überlappen, so dass keine unbeschichteten Lücken entstehen.

Die Tabletten setzen den Wirkstoff frei, wenn der pH Wert nach der Magenpassage über pH 5,5 steigt, und danach wieder abfällt. Bis zu einem maximal im Dünndarm vorkommenden pH Wert von 7,5 ist ein Abfall um mehr als 0,7 pH Stufen ausreichend um die Freisetzung auszulösen.

Werden zur Beschichtung der zweiten Tablettenseite Polymere oder Copolymere eingesetzt, deren Grenzwerte des pH Wertes, oberhalb oder unterhalb deren sie löslich oder permeabel werden, um 0,25 pH Stufen nach oben versetzt sind, so ist bereits ein Abfall des pH Wertes um 0,5 Stufen ausreichend, um eine Freisetzung auszulösen, sofern der maximal im Dünndarm vorkommende pH Wert zwischen 5,5 und 7,5 liegt.

Werden für die Beschichtung Polymere oder Copolymere eingesetzt, deren Grenzwerte (Schwellwerte) bei niedrigeren und/oder höheren pH Werten liegen, kann der Arbeitsbereich der Formulierung (der Bereich an pH Werten, innerhalb dessen die Freisetzung bei einem Abfall des pH Wertes um einen bestimmten Wert stattfindet) nach unten auf bis zu pH 2 und nach oben auf bis zu pH 9 ausgeweitet werden. Bevorzugt wird ein Arbeitsbereich von pH 3 bis 9 angestrebt, mehr bevorzugt von pH 4 bis pH 8, besonders bevorzugt von pH 5,5 bis pH 7,5.

Sind in der Zielgruppe für die Anwendung der Tabletten nach der Magenpassage positive Überschwinger des pH Wertes zu erwarten, kann zwischen der Entfernung der Schattenmaske und der Beschichtung mit dem Poly(Methacrylsäure - Methylmethacrylat)-Copolymer, welches oberhalb von pH 5,5 löslich ist, eine zusätzliche Beschichtung erfolgen, welche zeitverzögert aufgelöst wird, so dass eventuelle positive Überschwinger des pH Wertes bis zur vollständigen Auflösung dieser Schicht zumindest weitgehend abgeklungen sind. Diese zusätzliche Beschichtung kann beispielsweise aus einem sich langsam auflösenden Polymer bestehen, oder auch aus einer Schichtenfolge, z.B. aus einer Sprengschicht und einer darüber liegenden gering permeablen Schicht, wie bereits vorstehend zum Erreichen einer Zeitverzögerung beschrieben.

Um die feinen Abstufungen der Löslichkeits-Grenzwerte einstellen zu können, müssen nicht unbedingt jeweils für jede Beschichtung spezielle Polymere oder Copolymere mit eigenen Monomerverhältnissen synthetisiert werden. In gewissen Bereichen ist eine Einstellung der Löslichkeits-Grenzwerte auch durch Mischung von Copolymeren mit verschiedenen Monomeranteilen möglich, wie beispielsweise durch Mischung von organischen Sprühlösungen von Eudragit L und Eudragit S in unterschiedlichen Gewichtsanteilen.

### Beispiel 25:

Paracetamol (Acetaminophen) E-CDS Pellets werden hergestellt wie in "Multiparticulate Chitosan-Dispersed System for Drug Delivery", Norihito SHIMONO et al., Chem. Pharm. Bull. 51(6) 620-624 (2003), beschrieben. Allerdings werden die wirkstoffbeladenen Kerne nach dem Schritt "Preparation of Drug Cores" zuerst beschichtet mit einer Schicht, die unter pH 6 löslich, darüber aber unlöslich ist.

Dazu wird eine Poly(MMA-DEAEMA) Copolymer Dispersion entsprechend Kollicoat Smartseal 30 D hergestellt (siehe WO 2009/016258 A1, Beispiel 1), bei dem der DEAEMA-Anteil soweit erhöht wird, dass der Schwellwert für die Löslichkeit von pH 5,5 auf pH 6,0 angehoben wird.

Eine Sprühsuspension wird hergestellt bestehend aus 33% der beschriebenen Dispersion, 1,5% Triethylcitrat, 8% Talkum, und 57,5% Wasser.

Die Beschichtung erfolgt in einer Aeromatic Streal mittels Top spray.

Die Einlasslufttemperatur beträgt 55°, die Produktmenge 0,5kg, die Sprührate 6g/min, der Düsendurchmesser 0,8mm, der Sprühdruck 1,5 Bar. Die Beschichtung erfolgt bis zu einem Gewichtszuwachs von 30%. Danach erfolgt eine Trocknung für ca. 10 bis 15 min. bei 55°C.

Danach erfolgt die Beschichtung wie unter "Preparation of CDS Pellets" beschrieben, wobei allerdings kein Chitosanpulver verwendet wird, sondern ein Pulver mit vergleichbarer Partikelgröße, das wie folgt hergestellt wird:

Chitosanacetat (mittleres Molekulargewicht des Chitosans ca. 22000 Dalton) wird in der 150-fachen Menge (Gewicht/Gewicht) deionisiertem Wasser gelöst. Bernsteinsäureanhydrid wird unter 7 Minuten intensiven Rührens zugegeben in einer Menge von 32% des Polymertrockengewichtes. Nach 70 Minuten Reaktionszeit bei Raumtemperatur wird der pH Wert der Lösung mithilfe von 0,2 N Natriumhydrogencarbonat auf 8,6 eingestellt, und die Lösung für 10 Stunden bei Raumtemperatur moderat gerührt.

Danach wird die Lösung mit einer Dialysemembran mit einem Cut-Off von 3500 Dalton dialysiert gegen Wasser, welches mit Natriumhydroxid auf pH 10,3 eingestellt wird. Danach erfolgt eine weitere Dialyse gegen deionisiertes, mit Kohlensäure angereichertes Wasser unter einer CO2 Atmosphäre mit einem Druck von 3,5 bis 4 Bar. Je nach gewünschter Höhe der Löslichkeits-pH-Grenzwerte, bzw. der entsprechenden Auflösungsgeschwindigkeit oberhalb oder unterhalb des unlöslichen Bereiches kann auch eine Dialyse gegen eine Essigsäure-, Natronlauge-, oder Ammoniaklösung erfolgen (ggf. auch gegen eine Mischung von Kohlen- und Essigsäurelösung oder eine Mischung von Natriumhydroxid- und Ammoniaklösung), wobei die Konzentration der nach der Trocknung verbleibenden Ionen zur Einstellung der Auflösungsgeschwindigkeiten dienen kann.

Das getrocknete Reaktionsprodukt ist löslich in wässrigen Lösungen bei pH Werten unterhalb von pH 5,2 und oberhalb von pH 6,3, dazwischen ist es nicht löslich. Der Bereich, in dem das Reaktionsprodukt unlöslich ist, lässt sich durch Erhöhung der Menge des Bernsteinsäureanhydrids in Richtung niedrigerer pH Werte absenken, und durch Verringerung der Menge anheben. Die Breite des unlöslichen Bereiches lässt sich durch die Wahl des Molekülgewichts des verwendeten Chitosans beeinflussen.

Das Reaktionsprodukt wird durch Sprühtrocknung, Trocknung und Vermahlung oder andere geeignete Verfahren zu Pulver der entsprechenden Partikelgröße (im Mittel ca. 85µm) verarbeitet.

Nach dieser Beschichtung erfolgt die enterische Beschichtung wie unter "Preparation of E-CDS Pellets" beschrieben.

Im Magensaft bei pH 1,2 erfolgt innerhalb von 2 Stunden eine Freisetzung von unter 2 Prozent des Paracetamols. Im darauf folgenden Darmsaft bei pH 6 erfolgt innerhalb von weiteren 2 Stunden eine Freisetzung von unter 4 Prozent des Paracetamols (inklusive der im Magensaft freigesetzten Menge). Im darauf folgenden Darmsaft bei pH 6,7 erfolgt innerhalb weiterer 2 Stunden eine Freisetzung von unter 5 Prozent des Paracetamols (inklusive der im Magensaft und Darmsaft bei pH 6 freigesetzten Menge). Im darauf folgenden Zäkumsaft bei pH 5,6 erfolgt innerhalb von einer Stunde eine Freisetzung von über 50% des Paracetamols. Schließt sich an den Darmsaft bei pH 6,0 direkt ein Zäkumsaft mit pH 4,8 an, erfolgt ebenfalls eine Freisetzung von über 50% des Paracetamols innerhalb einer Stunde.

### Beispiel 26:

Gelatine-Hartkapseln der Größe 3 werden mit jeweils 195mg Mesalazin und 25mg Explotab gefüllt und verschlossen.

Sodann erfolgt eine Beschichtung mit Hydroxypropylcellulose. Dafür werden 66g Klucel EF in 660g deionisiertem Wasser aufgelöst und in einer O'Hara Labcoat 1 aus einer 1,2mm Düse mit 1,1 bar auf 1,5kg gefüllte Kapseln gesprüht. Die Einlassluft wird auf 150m³ pro Stunde und 35°C eingestellt, die Sprührate beträgt 4g/min. Je nach Feuchtigkeitsgehalt der Einlassluft kann die Sprührate erhöht werden, oder muss weiter verringert werden, um ein Erweichen der Kapseln zu vermeiden. Die Kapseln werden bei Raumtemperatur für 12 Stunden getrocknet.

Danach erfolgt eine Beschichtung mit einer Poly(MMA-DEAEMA) Copolymer Dispersion, die entsprechend Kollicoat Smartseal 30 D hergestellt wird (siehe WO 2009/016258 A1, Beispiel 1). Allerdings ist der DEAEMA-Anteil so eingestellt, dass der Schwellwert für die Löslichkeit von pH 5,5 auf pH 6,0 angehoben wird.

820g der so hergestellten Dispersion mit 30% Polymeranteil werden mit 25g Triethylcitrat, 120g Talkum und 1000g Wasser zu einer Sprühlösung vermischt. Die Beschichtung erfolgt mit dem gleichen Prozess wie die Beschichtung mit Hydroxypropylcellulose. Allerdings kann aufgrund der Vorbeschichtung eine Sprührate von 15g/min eingesetzt werden (in den ersten 10 Minuten auf 9g/min reduziert).

200g eines getrockneten Chitosan-Bernsteinsäureanhydrid-Reaktionsproduktes wie in Beispiel 25 beschrieben wird in kohlensäurehaltigem Wasser homogenisiert und unter CO2 Atmosphäre (Gasdruck 3,5 Bar) und intensivem Rühren über Nacht gelöst. Sodann wird die Lösung unter 2 Bar CO2 mit deionisiertem Wasser auf eine Viskosität von 100 mPa*s eingestellt, und wie bei den beiden vorhergehenden Beschichtungsprozessen aufgesprüht, wobei eine 0,8mm Düse zum Einsatz kommt. Die Sprührate beträgt zu Beginn 4g/min, nach erfolgtem Aufsprühen von 10% der Lösung 10g/min.

Alternativ kann die Lösung anstatt mit CO2 auch mit einer wässrigen Ammoniak Lösung hergestellt werden, wobei ein pH von ca. 7,5 eingestellt wird. Die Herstellung der Sprühlösung unter Verwendung von Natronlauge (ebenfalls ca. pH 7,5) oder Essigsäure (ca. pH 4,4) ist ebenfalls möglich, wobei sich allerdings die Löslichkeits-Grenzwerte zu niedrigeren oder höheren pH Werten hin verschieben können, was ggf. durch eine Veränderung der Menge des Bernsteinsäureanhydrids bei der Herstellung des Polymers kompensiert werden muss.

Die Kapseln werden danach für 24 Stunden bei 30°C getrocknet.

Sodann erfolgt eine weitere Beschichtung mit Hydroxypropylcellulose, analog der zuerst aufgebrachten Schicht.

Danach erfolgt eine enterische Beschichtung. Dafür werden 820g Eudragit L 30 D-55 mit 123g Talkum, 24g Triethylcitrat und 1000g Wasser zu einer Sprühlösung vermischt. Der Sprühprozess erfolgt wie bei der Beschichtung mit der Poly(MMA-DEAEMA) Copolymer Dispersion. Anschließend erfolgt eine Trocknung bei Raumtemperatur für 24 Stunden.

Im Magensaft (pH 1,2 für 2 Stunden) und im Dünndarmsaft (pH 6 für 2 Stunden und pH 6,7 für weitere 2 Stunden) erfolgt keine Freisetzung von Mesalazin. Im Zäkumsaft (pH 5,6 nach dem Dünndarmsaft mit pH 6,7, bzw. pH 4,8 nach dem Dünndarmsaft mit pH 6,0) reißt die Kapselhülle nach 30 Minuten und das Mesalazin wird freigesetzt.

### Beispiel 27:

Paracetamol (Acetaminophen) CDS Pellets werden hergestellt wie in "Multiparticulate Chitosan-Dispersed System for Drug Delivery", Norihito SHIMONO et al., Chem. Pharm. Bull. 51(6) 620-624 (2003), beschrieben.

Danach erfolgt eine weitere Beschichtung wie unter "Preparation of CDS Pellets" beschrieben, wobei allerdings kein Chitosanpulver verwendet wird, sondern ein Pulver mit vergleichbarer Partikelgröße, das wie folgt hergestellt wird: Chitosanacetat (mittleres Molekulargewicht des Chitosans ca. 15000 Dalton) wird in der 150-fachen Menge (Gewicht/Gewicht) deionisiertem Wasser gelöst. Bernsteinsäureanhydrid wird unter 7 Minuten intensiven Rührens zugegeben in einer Menge von 31% des Polymertrockengewichtes. Nach 70 Minuten Reaktionszeit bei Raumtemperatur wird der pH Wert der Lösung mithilfe von 0,2 N Natriumhydrogencarbonat auf 8,6 eingestellt, und die Lösung für 10 Stunden bei Raumtemperatur moderat gerührt. Danach wird die Lösung mit einer Dialysemembran mit einem Cut-Off von 3500 Dalton dialysiert gegen Wasser, welches mit Natriumhydroxid auf pH 10,3 eingestellt wird. Danach erfolgt eine weitere Dialyse gegen Wasser, welches mit Ammoniak auf pH 8,2 eingestellt wird.

Das getrocknete Reaktionsprodukt ist löslich in wässrigen Lösungen bei pH Werten unterhalb von pH 5,8 und oberhalb von pH 7,1, dazwischen ist es nicht löslich. Der Bereich, in dem das Reaktionsprodukt unlöslich ist, lässt sich durch Erhöhung der Menge des Bernsteinsäureanhydrids in Richtung niedrigerer pH Werte absenken, und durch Verringerung der Menge anheben. Die Breite des unlöslichen Bereiches lässt sich durch die Wahl des Molekülgewichts des verwendeten Chitosans beeinflussen.

Das Reaktionsprodukt wird durch Sprühtrocknung, Trocknung und Vermahlung oder andere geeignete Verfahren zu Pulver der entsprechenden Partikelgröße (im Mittel ca. 85µm) verarbeitet.

Nach dieser Beschichtung erfolgt die enterische Beschichtung wie unter "Preparation of E-CDS Pellets" beschrieben. Allerdings wird das Eudragit L100-55 durch Eudragit L 100 ersetzt und zusätzlich noch Triethylcitrat (10 Gewichtsprozent bezogen auf das Eudragit L 100) zugegeben.

Im Magensaft bei pH 1,2 erfolgt innerhalb von 2 Stunden eine Freisetzung von unter 2 Prozent des Paracetamols. Im darauf folgenden Darmsaft bei pH 6,8 erfolgt innerhalb von weiteren 2 Stunden eine Freisetzung von unter 4 Prozent des Paracetamols (inklusive der im Magensaft freigesetzten Menge). Im darauf folgenden Darmsaft bei pH 7,4 erfolgt innerhalb weiterer 2 Stunden eine Freisetzung von unter 5 Prozent des Paracetamols (inklusive der im Magensaft und Darmsaft bei pH 6,8 freigesetzten Menge). Im darauf folgenden Zäkumsaft bei pH 6,0 erfolgt innerhalb von einer Stunde eine Freisetzung von ca. 30% des Paracetamols.

Schließt sich an den Darmsaft bei pH 6,8 direkt ein Zäkumsaft mit pH 5,4 an, erfolgt ebenfalls eine Freisetzung von ca. 30% des Paracetamols innerhalb einer Stunde.

In den darauf folgenden 5 Stunden erfolgt die Freisetzung mit ca. 10% des Paracetamols pro Stunde. Danach sinkt die Freisetzungsrate leicht ab.

### Beispiel 28:

Wirkstoffhaltige schnellzerfallende Pellets werden hergestellt wie in EP 0925060 Beispiel 1 beschrieben.

Die Pellets werden beschichtet, wie in "Multiparticulate Chitosan-Dispersed System for Drug Delivery", Norihito SHIMONO et al., Chem. Pharm. Bull. 51(6) 620-624 (2003), unter "Preparation of CDS Pellets" beschrieben.

Danach erfolgen die gleichen Beschichtungsvorgänge wie in Beispiel 27 der vorliegenden Erfindung nach der Herstellung der CDS Pellets beschrieben.

Der Beginn der Freisetzung erfolgt vergleichbar mit der in Beispiel 27 beschriebenen Abhängigkeit vom Verlauf der pH Werte. Allerdings erfolgt nach der Verbringung in Zäkumsaft die Freisetzung zu mehr als 70% innerhalb von 30 Minuten.

### Beispiel 29:

Wirkstoffhaltige Pellets werden hergestellt und beschichtet wie in Beispiel 28 beschrieben.

Allerdings erfolgt zwischen den beiden Beschichtungsvorgängen mit den verschiedenen Chitosan-Pulvern (unmodifiziert und modifiziert) eine zusätzliche Beschichtung mit niedrig substituierter Hydroxypropylcellulose (20% Gewichtszuwachs) wie in EP0210540 Beispiel 2 beschrieben.

Die Freisetzung erfolgt vergleichbar mit der in Beispiel 28 beschriebenen Charakteristik, allerdings erfolgt nach der Verbringung aus Darmsaft bei pH 7,4 in Zäkumsaft mit pH 6,0 bereits nach 20 Minuten eine Freisetzung zu mehr als 70%.

### Beispiel 30:

Paracetamol Pellets werden hergestellt wie in Beispiel 25.

Allerdings wird als Pulver für die zweite Beschichtung nicht direkt das modifizierte Chitosan benutzt, sondern Sucrose-Microspheres, die mit dem modifizierten Chitosan beschichtet sind.

Die beschichteten Microspheres werden folgendermaßen hergestellt:
Sucrose-Microspheres, beispielsweise hergestellt in einem Liquiflash-Prozess, wie in US5874110 Example I beschrieben, werden in Hexafluorisopropanol dispergiert, in dem wie in Beispiel 25 modifiziertes Chitosan gelöst ist (85 Gewichtsprozent modifiziertes Chitosan bezogen auf die Masse der Microspheres). Nach dem Überleiten in flüssiges Paraffin, das 4% Span 80 enthält (gerührt mit 150 rpm), wird das Hexafluorisopropanol verdampft (4 Stunden Rühren bei 30°C). Anschließend werden die beschichteten Microspheres durch Filtrierung gewonnen und mehrmals mit Petrolether (40-60°) gewaschen. Danach werden die Microspheres bei Raumtemperatur für 24 Stunden getrocknet.

Die beschichteten Microspheres werden sodann an Stelle des Pulvers aus modifiziertem Chitosan verwendet.

Die Freisetzungscharakteristik entspricht weitgehend der in Bespiel 25 beschriebenen. Allerdings wird nach Verbringung in Zäkumsaft innerhalb von 20 Minuten eine Freisetzung von über 50% des Paracetamols erreicht.

### Beispiel 31:

Unbeschichtete Paracetamol (Acetaminophen) Pellets werden beschichtet mit einer Schicht, die unter pH 7 löslich, darüber aber unlöslich ist.

Dazu wird ein Poly(N-acryloyl-N'-ethyl piperazin-co-methyl methacrylat)-Copolymer hergestellt, wie beschrieben in "Solution Properties of Water-Soluble "Smart" Poly(N-acryloyl-N'-ethyl piperazine-co-methyl methacrylate)", G. Roshan Deen, Polymers 2012, 4, 32-45; doi:10.3390/polym4010032. Der AcrNEP Anteil beträgt 52mol%.

Die Beschichtung erfolgt wie in "Multiparticulate Chitosan-Dispersed System for Drug Delivery", Norihito SHIMONO et al. unter "Preparation of E-CDS Pellets" beschrieben.

Allerdings wird die Sprühlösung hergestellt, aus dem Poly(MMA-AcrNEP)-Copolymer, Talkum, Triethylcitrat, Aceton, Ethanol (95%) (6:3:0,5:45,5:45).

Sodann erfolgt eine weitere Beschichtung, wobei allerdings statt des Poly(MMA-AcrNEP)-Copolymers ein PHEA-g-C₁₈10-IM50 Polymer eingesetzt wird, das wie in "Tunable phase transition behaviors of pH-sensitive polyaspartamides having various cationic pendant groups", Han Woong Park, Colloid Polym Sci (2009) 287:919-926, beschrieben hergestellt wird.

Diese Beschichtung ist löslich in wässrigen Lösungen unterhalb von pH 6,5 und oberhalb von pH 7,2.

Nach dieser Beschichtung erfolgt die enterische Beschichtung wie unter "Preparation of E-CDS Pellets" beschrieben. Allerdings wird das Eudragit L100-55 durch eine Mischung von 80% Eudragit S100 und 20% Eudragit L 100 ersetzt und zusätzlich noch Triethylcitrat (10 Gewichtsprozent bezogen auf das Eudragit S/L Gemisch) zugegeben.

Diese Beschichtung ist löslich in wässrigen Lösungen oberhalb von pH 6,8.

### Beispiel 32:

Unbeschichtete Paracetamol (Acetaminophen) Pellets werden beschichtet mit einer Schicht, die unter pH 7,7 löslich, darüber aber unlöslich ist.

Dazu wird ein Poly(N-acryloyl-N'-ethyl piperazin-co-methyl methacrylat)-Copolymer hergestellt, wie beschrieben in "Solution Properties of Water-Soluble "Smart" Poly(N-acryloyl-N'-ethyl piperazine-co-methyl methacrylate)", G. Roshan Deen, Polymers 2012, 4, 32-45; doi:10.3390/polym4010032. Der AcrNEP Anteil beträgt 58mol%.

Die Beschichtung erfolgt wie in "Multiparticulate Chitosan-Dispersed System for Drug Delivery", Norihito SHIMONO et al. unter "Preparation of E-CDS Pellets" beschrieben.

Allerdings wird die Sprühlösung hergestellt, aus dem Poly(MMA-AcrNEP)-Copolymer, Talkum, Triethylcitrat, Aceton, Ethanol (95%) (6:3:0,5:45,5:45).

Sodann erfolgt eine weitere Beschichtung, wobei allerdings statt des Poly(MMA-AcrNEP)-Copolymers ein PHEA-g-C₁₈10-IM90 Polymer eingesetzt wird, das wie in "Tunable phase transition behaviors of pH-sensitive polyaspartamides having various cationic pendant groups", Han Woong Park, Colloid Polym Sci (2009) 287:919-926, beschrieben hergestellt wird.

Diese Beschichtung ist löslich in wässrigen Lösungen unterhalb von pH 6,2 und oberhalb von pH 7,8.

Danach erfolgt eine Beschichtung mit einem modifizierten Chitosan wie bereits als zweite Beschichtung in Beispiel 25 beschrieben. Die Schicht ist löslich unterhalb von pH 5,2 und oberhalb von pH 6,3.

Sodann erfolgt eine weitere Beschichtung, ähnlich der zweiten in diesem Beispiel beschriebenen Beschichtung mit dem PHEA-g-C₁₈10-IM90 Polymer, wobei allerdings statt PHEA-g-C₁₈10-IM90 Polymers ein PHEA-g-C₁₈10-PY45 Polymer (hergestellt wie in "Tunable phase transition behaviors of pH-sensitive polyaspartamides having various cationic pendant groups" beschrieben, aber mit verändertem Gehalt an Aminomethylpyridin) eingesetzt wird.

Diese Beschichtung ist löslich in wässrigen Lösungen unterhalb von pH 4,3 und oberhalb von pH 5,7.

Nach dieser Beschichtung erfolgt die enterische Beschichtung wie unter "Preparation of E-CDS Pellets" beschrieben. Allerdings wird das Eudragit L 100-55 durch ein Copolymer ersetzt, das wie Eudragit L100-55 hergestellt wird, wobei allerdings das Monomerverhältnis so verändert wird (der Anteil an Methacrylsäure wird erhöht), dass das Copolymer bereits oberhalb von pH 4,7 löslich wird.

Diese Beschichtung ist löslich in wässrigen Lösungen oberhalb von pH 4,7.

Die Pellets werden sodann in Hartgelatinekapseln abgefüllt.

Der Arbeitsbereich dieser Formulierung erstreckt sich über einen Bereich des maximal im Dünndarm erreichten pH Wertes von 4,8 bis 8,9

### Beispiel 33:

Kapselkappen der Größe 1 aus Gelatine oder vorzugsweise HPMC werden mit einer reduzierten Wandstärke von 40µm hergestellt und beschichtet mit drei 20µm dicken funktionalen Polymerschichten. Hierzu verbleiben sie an dem Tauchstift, bis die letzte Beschichtung fertig gestellt ist. Die erste Beschichtung erfolgt wie in US 2011/0033530 A1 beschrieben (analog zu Beispiel C6 des genannten Dokuments). Allerdings wird statt Eudragit FS 30D als funktionales Polymer eine Poly(MMA-DEAEMA) Copolymer Dispersion entsprechend Kollicoat Smartseal 30 D hergestellt (siehe WO 2009/016258 A1, Beispiel 1), bei dem der DEAEMA-Anteil soweit erhöht wird, dass der Schwellwert für die Löslichkeit von pH 5,5 auf pH 5,8 angehoben wird. Aufgrund der geringen Viskosität der Dispersion wird die Schicht ggf. in mehreren Tauchgängen erzeugt. Diese Schicht wird unterhalb von pH 5,8 löslich.

Sodann werden die Kapselkappen mit einer zweiten 20µm dicken funktionalen Schicht beschichtet. Zur Herstellung der Tauchlösung wird analog Beispiel 25 Chitosanacetat (mittleres Molekulargewicht des Chitosans ca. 22000 Dalton) in der 150-fachen Menge (Gewicht/Gewicht) deionisiertem Wasser gelöst. Bernsteinsäureanhydrid wird unter 7 Minuten intensiven Rührens zugegeben in einer Menge von 32% des Polymertrockengewichtes. Nach 70 Minuten Reaktionszeit bei Raumtemperatur wird der pH Wert der Lösung mithilfe von 0,2 N Natriumhydrogencarbonat auf 8,6 eingestellt, und die Lösung für 10 Stunden bei Raumtemperatur moderat gerührt. Danach wird die Lösung mit einer Dialysemembran mit einem Cut-Off von 3500 Dalton dialysiert gegen Wasser, welches mit Natriumhydroxid auf pH 10,3 eingestellt wird. Danach erfolgt eine weitere Dialyse gegen Wasser, welches mit Ammoniak auf pH 8,2 eingestellt wird. Die Lösung wird auf eine Viskosität von ca. 300 mPa*s eingestellt. Aufgrund des geringeren Feststoffgehaltes gegenüber einer Dispersion, sind ggf. mehrere Tauch- und Trockenvorgänge erforderlich, um die gewünschte Schichtdicke zu erreichen.

Schließlich werden die Kapselkappen mit einer dritten 20µm dicken funktionalen Schicht analog Beispiel 3 der US 2011/0033530 A1 beschichtet. Der pH Wert der Tauchlösung wird auf 5,0 eingestellt, und die Wassermenge entsprechend angepasst, um die gewünschte Schichtdicke nach der Trocknung zu erreichen. Diese Schicht wird oberhalb von pH 5,5 löslich, nachdem sie der Magensäure ausgesetzt war.

Kapselböden (im Stand der Technik teilweise auch als Kapselkörper bezeichnet) der Größe 1 werden hergestellt und beschichtet wie vorab bei den Kapselkappen beschrieben. Allerdings wird für die erste Beschichtung als funktionales Polymer ein Polymer eingesetzt, das wie Kollicoat Smartseal 30 D hergestellt wird, wobei allerdings ein nochmals erhöhter Anteil an Diethylaminoethylmethacrylat-Monomeren eingesetzt wird, so dass die erste Beschichtung schon unterhalb von pH 6,8 löslich wird.

Alternativ kann für die erste Beschichtung ein Poly(MMA-AcrNEP)-Copolymer mit 52 mol% AcrNEP verwendet werden, das unterhalb von pH 7 löslich ist. Dies wird als Pulver mit einer mittleren Partikelgröße von ca. 150nm bereitgestellt, und in der dreifachen Menge Wasser, dem 1,5% Natriumlaurylsulfat und 2% Stearinsäure zugesetzt sind, dispergiert. Sodann wird diese Dispersion mit der gleichen Menge Kollicoat Smartseal 30 D vermischt, und 15% PEG 35000 und 15% Triethylcitrat (beides bezogen auf den Polymergehalt der Dispersionsmischung) zugegeben, um die Tauchlösung zu erhalten.

Für die zweite Beschichtung wird statt des modifizierten Chitosans ein PHEA-g-C₁₈10-IM50 Polymer eingesetzt, das wie in "Tunable phase transition behaviors of pH-sensitive polyaspartamides having various cationic pendant groups", Han Woong Park, Colloid Polym Sci (2009) 287:919-926, beschrieben hergestellt wird, und sowohl unterhalb von pH 6,5, als auch oberhalb von pH 7,2 löslich ist. Dieses wird in einer wässrigen Ammoniaklösung bei pH 8,6 gelöst und auf eine Viskosität von ca. 300 mPa*s eingestellt.

Für die dritte Beschichtung wird statt Eudragit L 100-55 für die Redispersion als funktionales Polymer ein Gemisch aus 80% Eudragit S100 und 20% Eudragit L 100 (beides gemeinsam in Isopropanol gelöst, intensiv gemischt, und danach sprühgetrocknet, um ein redispergierbares Pulver zu erhalten) eingesetzt, so dass die dritte Schicht erst oberhalb von pH 6,8 löslich wird. Der Zusatz von Triethylcitrat wird von 10% auf 15% erhöht. Die Dispersion wird mit Natriumhydroxidlösung auf einen pH Wert von 6,4 eingestellt.

Die beschichteten Kapselböden werden mit einem Wirkstoff, beispielsweise 400mg Mesalazin, befüllt und mit den beschichteten Kapselkappen verschlossen. Der Spalt zwischen den Kapselhälften wird mit einer nicht wasserlöslichen Polymerdispersion versiegelt, beispielsweise mit Eudragit NE 30D. Hierzu wird diese in den Spalt dispenst und getrocknet.

Die Kapseln der Größe 1 werden in Kapselböden der Größe 0 gesteckt, und diese mit Kapselkappen der Größe 0 verschlossen.

Die Kapseln setzen den Wirkstoff sicher im Dickdarm frei, wenn der Abfall des pH Wertes nach der Passage der Ileozäkalklappe mindestens 1,1 pH Stufen beträgt, und der maximale pH Wert im Dünndarm zwischen 5,5 und 7,9 (bzw. 8,1 bei Verwendung des Poly(MMA-AcrNEP)-Copolymers) liegt.

### Beispiel 34:

Gelatine-Hartkapseln der Größe 3 werden mit jeweils 195mg Mesalazin und 25mg Explotab gefüllt und verschlossen.

Sodann erfolgt eine Beschichtung mit Hydroxypropylcellulose. Dafür werden 66g Klucel EF in 660g deionisiertem Wasser aufgelöst und in einer O'Hara Labcoat 1 aus einer 1,2mm Düse mit 1,1 bar auf 1,5kg gefüllte Kapseln gesprüht. Die Einlassluft wird auf 150m³ pro Stunde und 35°C eingestellt, die Sprührate beträgt 5g/min. Je nach Feuchtigkeitsgehalt der Einlassluft kann die Sprührate erhöht werden, oder muss weiter verringert werden, um ein Erweichen der Kapseln zu vermeiden. Die Kapseln werden bei Raumtemperatur für 12 Stunden getrocknet.

Danach erfolgt eine Beschichtung mit einer Poly(MMA-DEAEMA) Copolymer Dispersion, die entsprechend Kollicoat Smartseal 30 D hergestellt wird (siehe WO 2009/016258 A1, Beispiel 1). Allerdings ist der Anteil an DEAEMA so eingestellt, dass der Schwellwert für die Löslichkeit von pH 5,5 auf pH 6,5 angehoben wird.

820g der so hergestellten Dispersion mit 30% Polymeranteil werden mit 25g Triethylcitrat, 125g Talkum und 1000g Wasser zu einer Sprühlösung vermischt. Die Beschichtung erfolgt mit dem gleichen Prozess wie die Beschichtung mit Hydroxypropylcellulose. Allerdings kann aufgrund der Vorbeschichtung eine Sprührate von 11g/min eingesetzt werden (in den ersten 10 Minuten auf 6g/min reduziert).

420g der so beschichteten Hartgelatinekapseln werden in einen CF-Granulator gegeben, der auf 250 Umdrehungen pro Minute und 150 l/min Einlassluft mit 30° eingestellt wird. Eine Suspension aus 300g einer mit Wasser auf 12% Polymergehalt verdünnten und auf pH 6,75 eingestellten Eudragit NE 30 D Dispersion, 12g Poly(MMA-DEAEMA)-Copolymer-Pulver und 12g Poly(MA-MMA-MAA)-Copolymer-Pulver (hergestellt aus Kollicoat Smartseal 30D, bzw. Eudragit FS 30D, per Schmelzextrusion mit anschließender Granulierung, jeweils mit einer mittleren Partikelgröße von 95µm), wird mit einer Zuführungsrate von 3,5 g/min auf das Kapselbett getropft.

Nach Trocknung der Schicht werden die beschichteten Kapseln mit einen Enteric Coating beschichtet. Die Sprühlösung wird folgendermaßen zusammengesetzt:
820g Eudragit L 30 D-55, 120g Talkum, 25g Triethylcitrat, 1000g Wasser.

Die Beschichtung erfolgt mit dem gleichen Prozess wie die zweite Beschichtung.

Nach der Trocknung werden die beschichteten Kapseln in Hartgelatinekapseln der Größe 1 verbracht.

Die so beschichteten Kapseln geben ihren Inhalt frei, wenn der pH Wert nach der Magenpassage über 5,5 ansteigt, und danach wieder unter 5,5 fällt.

Erfolgt ein Anstieg des pH Wertes über 7, wird der Kapselinhalt auch dann freigesetzt, wenn der pH Wert danach nur unter einen Wert von 6,5 fällt.

### Beispiel 35:

Microspheres aus Chitosan und 5-Fluoruracil (30% 5-FU) werden hergestellt wie beschrieben in "Preparation and characterization of chitosan microspheres containing doxifluridine.", Yoshino et al., Drug Development and Industrial Pharmacy, 2003, Absatz "Preparation of Microspheres". Allerdings wird Chitosan verwendet, welches zu 85% deacetyliert ist, und ein mittleres Molekülgewicht von 11000 Dalton hat. Der mittlere Durchmesser beträgt 650µm.

Chitosan, das wie in Beispiel 25 modifiziert wurde, allerdings mit 31% Bernsteinsäureanhydrid, wird in wässriger Ammoniaklösung bei pH 8,5 gelöst (1,5%). Die Lösung wird auf eine Viskosität von 100 mPa*s eingestellt. Die Microspheres werden in einem Glatt GPCG 1.1 per Top-Spray mit der Sprühlösung beschichtet. Düse: Schlick 970/0, Düsenöffnung: 1,2mm, Sprühdruck: 1,8 Bar, Zulufttemperatur: 35°, Sprührate: 12g/min/kg, Trocknungsluftvolumen: 55m³/h, Trocknungszeit bei 40°: 2h. Die Schichtdicke wird auf 20µm eingestellt.

Sodann werden sie zusammen mit Microspheres zu Minitabletten mit einem Durchmesser von 2,5mm und einer Höhe von 1,5mm verpresst. Hierzu wird zuerst Granulat, hergestellt aus Kollicoat Smartseal 30D wie in Beispiel 34 beschrieben, in die Pressform gefüllt, anschließend eine Microsphere per Vakuumpipette mittig leicht in das Granulat eingedrückt, und dann ein Granulat eingefüllt, das mit dem gleichen Prozess hergestellt wurde, wobei allerdings statt Kollicoat Smartseal eine Mischung aus 46% Eudragit S 100,45% Eudragit L 100 und 9% Triethylcitrat verwendet wurde, so dass es oberhalb von pH 6,4 löslich ist.

Ein PHEA-g-C₁₈10-PY45 Polymer wird hergestellt, wie in "Tunable phase transition behaviors of pH-sensitive polyaspartamides having various cationic pendant groups" beschrieben, aber mit entsprechend verändertem Gehalt an Aminomethylpyridin. Per Schmelzextrusion und Granulierung wird ein Granulat mit einem mittleren Partikeldurchmesser von 90µm erzeugt.

300g der Tabletten werden in einen CF-Granulator gegeben, der auf 300 Umdrehungen pro Minute und 180 l/min Einlassluft mit 35° eingestellt wird. Eine Suspension aus 300g einer mit Wasser auf 12% Polymergehalt verdünnten und auf pH 5 eingestellten Eudragit NE 30 D Dispersion und 24g des Granulates wird mit einer Zuführungsrate von 4,5 g/min auf die Tabletten getropft.

Nach der Trocknung werden die Minitabletten mit einer enterischen Beschichtung versehen.

Hierzu werden 10g Polysorbat 80, 8,5g Glycerolmonostearat und 17g Triethylcitrat mindestens 15 Minuten in 160g Wasser homogenisiert, das zuvor auf 75°C erwärmt wurde. Danach werden 230g Wasser eingerührt, und die Suspension nach Abkühlung auf Raumtemperatur in 570g Eudragit L 30 D-55 eingerührt. Die Beschichtung von 2,5kg Tabletten erfolgt in einer O'hara LabCoat, Trommelgeschwindigkeit 20U/min, Düsendurchmesser 1,2mm, Druck 1, Bar bei 10cm Düsenabstand, Trockenluftvolumen 170m³/h bei 45°C, Sprührate 10g/min, Beschichtungsstärke 7,5mg/cm².

Die Minitabletten werden in Hartgelatinekapseln der Größe 00 abgefüllt.

### Beispiel 36

HPMC Kapseln der Größe 000 werden befüllt mit Kontrastkugeln (luftgefüllte Hohlkugeln aus PMMA (Plexiglas), Durchmesser 4mm, Wandstärke 0,2mm), welche in wässrigen Medien Ultraschall mit 3,5 MHz gut reflektieren. Die Kapseln wiegen pro Stück ca. 330mg.

Es folgt eine Beschichtung mit Hydroxypropylcellulose. Dafür werden 66g Klucel EF in 660g deionisiertem Wasser aufgelöst und in einer O'Hara Labcoat 1 aus einer 1,2mm Düse mit 1,1 bar auf 1,25kg gefüllte Kapseln gesprüht. Die Einlassluft wird auf 150m³ pro Stunde und 35°C eingestellt, die Sprührate beträgt 5g/min. Je nach Feuchtigkeitsgehalt der Einlassluft kann die Sprührate erhöht werden, oder muss weiter verringert werden, um ein Erweichen der Kapseln zu vermeiden. Die Kapseln werden bei Raumtemperatur für 12 Stunden getrocknet.

Danach erfolgt eine Beschichtung mit einer Poly(MMA-DEAEMA) Copolymer Dispersion, die entsprechend Kollicoat Smartseal 30 D hergestellt wird (siehe WO 2009/016258 A1, Beispiel 1). Allerdings ist der Anteil an DEAEMA so eingestellt, dass der Schwellwert für die Löslichkeit von pH 5,5 auf pH 6,0 angehoben wird.

820g der so hergestellten Dispersion mit 30% Polymeranteil werden mit 25g Triethylcitrat, 125g Talkum und 1000g Wasser zu einer Sprühlösung vermischt. Die Beschichtung erfolgt mit dem gleichen Prozess wie die Beschichtung mit Hydroxypropylcellulose. Allerdings kann aufgrund der Vorbeschichtung eine Sprührate von 11g/min eingesetzt werden (in den ersten 10 Minuten auf 6g/min reduziert).

250g der so beschichteten Hartgelatinekapseln werden in einen CF-Granulator gegeben, der auf 180 Umdrehungen pro Minute und 150 l/min Einlassluft mit 30° eingestellt wird. Eine Suspension aus 300g einer mit Wasser auf 12% Polymergehalt verdünnten und auf pH 6,75 eingestellten Eudragit NE 30 D Dispersion und 24g SALM-CS-Pulver, hergestellt wie beschrieben in "Zwitterionic Chitosan Derivatives for pH-Sensitive Stealth Coating", Peisheng Xu et al., Biomacromolecules, Vol. 11, No. 9, 2010, mit einem An/Am Verhältnis von 0,65 und einer mittleren Partikelgröße von 80µm, wird mit einer Zuführungsrate von 3,5 g/min auf das Kapselbett getropft.

Nach Trocknung der Schicht werden 1,25kg beschichtete Kapseln mit einen Enteric Coating beschichtet. Die Sprühlösung wird folgendermaßen zusammengesetzt:
820g Eudragit L 30 D-55, 120g Talkum, 25g Triethylcitrat, 1000g Wasser.

Die Beschichtung erfolgt mit dem gleichen Prozess wie die zweite Beschichtung.

Nach der Trocknung erfolgt nochmals eine Beschichtung mit dem Poly(MMA-DEAEMA) Copolymer wie bei der zweiten aufgebrachten Schicht.

Die beschriebenen Kapseln wurden an sechs aufeinander folgenden Tagen einem Freiwilligen (männlich, 40 Jahre) verabreicht. An Tag 1 und 2 jeweils 30 Minuten vor dem Frühstück, Tag 3 und 4 jeweils 15 Minuten nach dem Mittagessen und Tag 5 und 6 zwei Stunden nach einem leichten Frühstück (Joghurt mit Reisflocken). In Abständen von jeweils 60 Minuten wurde das Abdomen des Freiwilligen mittels Ultraschall (Hitachi/Picker LC7000A mit 3,5MHz Konvex-Sensor) untersucht, um die verabreichte Kapsel zu lokalisieren und ihren Zustand zu beurteilen. Als Referenzobjekte wurden eine Woche davor entsprechende Ultraschalluntersuchungen an dem Freiwilligen mit Kapseln durchgeführt, die mit einem unlöslichen Polymer (PMMA) beschichtet waren, und eine weitere Woche davor mit Kapseln, die nur magensaftresistent beschichtet waren (Eudragit L 30 D-55). Die unlöslich beschichtete Kapsel mit den darin enthaltenen Kontrastkugeln konnte im Ultraschallbild deutlich von den aus der nur magensaftresistent beschichteten Kapsel ausgetretenen Kontrastkugeln unterschieden werden. Bei vorhergehenden Untersuchungen war die Lokalisierung der luftgefüllten Kontrastkugeln teilweise durch Darmgase behindert, weshalb der Freiwillige jeweils zwei Tage vor den Referenzuntersuchungen und zwei Tage vor, sowie während der Testdauer eine blähungsarme Diät erhielt, wodurch die Lokalisation der Kontrastkugeln deutlich erleichtert wurde.

Bei der Untersuchung konnten die Kapseln trotzdem nicht bei jeder Sonographie sicher lokalisiert werden, solange sie sich noch im oberen und mittleren Dünndarm befanden, sondern nur bei ca. 40% der Sonographien. Das terminale Ileum, und die Kapseln, wenn sie sich dort befanden, waren jedoch immer gut erkennbar. Wurden die Kapseln innerhalb des Dünndarms lokalisiert, waren sie immer intakt. 5 der 6 verabreichten Kapseln konnten im Bereich der Ileozäkalklappe intakt lokalisiert werden, zwei davon kurz vor dem Durchtritt, drei davon kurz danach. Diese letztgenannten 3 Kapseln konnten bei der nächsten Messung nicht mehr intakt lokalisiert werden. Die Verteilung der Kontrastkugeln ließ darauf schließen, dass die Kapselhülle noch teilweise intakt war, aber bereits ein Teil der Kontrastkugeln begonnen hatte, sich im Dickdarminhalt zu verteilen. Die anderen 2 Kapseln konnten bei dieser Messung intakt im Blinddarm lokalisiert werden. Bei der nächsten Messung waren auch aus diesen Kapseln Kontrastkugeln ausgetreten. Die Kapsel von Tag 2, die nicht in der Nähe der Ileozäkalklappe lokalisiert werden konnte, konnte bei der nachfolgenden Messung im aufsteigenden Dickdarm lokalisiert werden, wo auch bereits ausgetretene Kontrastkugeln lokalisiert werden konnten. Die Kapsel von Tag 5 konnte bereits 3 Stunden nach der Einnahme vor der Ileozäkalklappe lokalisiert werden und war dort 5 Stunden nach der Einnahme noch als intakt zu beurteilen. 6 Stunden nach der Einnahme (ca. 45 Minuten nach dem Mittagessen) waren dann die Kontrastkugeln im Blinddarm verteilt lokalisierbar. Es konnte also keine Freisetzung im Dünndarm festgestellt werden. Im Dickdarm wurden die Kontrastkugeln in allen Fällen vor der hepatischen Flexur freigesetzt.

### Beispiel 37

Pellets oder Kapseln werden hergestellt wie in US7604820 in den Beispielen 1 oder 2 oder der Kombination der Beispiele 6 und 7 beschrieben, wobei statt des Chitosan-Pulvers ein Pulver gleicher Korngrößenverteilung benutzt wird, das aber entweder aus SALM-CS mit einem An/Am Verhältnis von 0,65 wie in Beispiel 36 verwendet, oder einem PHEA-g-C₁₈10-PY70 Copolymer mit entsprechender Partikelgröße besteht. Die Pellets und Kapseln zeigen ähnliche Freisetzungseigenschaften wie in US7604820 für die dort beschriebenen Beispiele gezeigt, wobei die zweite Testflüssigkeit (2nd Fluid) statt auf pH 6,8 auf pH 6,2 eingestellt wird.. Zusätzlich wird der Wirkstoff aber auch dann freigesetzt, wenn statt dem dritten Testmedium mit einem pH Wert von 4,0 eines mit pH 7,4 verwendet wird. Der Wirkstoff wird also sowohl dann freigesetzt, wenn nach einem Anstieg auf über pH 6 ein Abfall auf pH 4 erfolgt, als auch dann, wenn der pH Wert auf 7,4 ansteigt.

### Beispiel 38

Paracetamol (Acetaminophen) E-CDS Pellets werden hergestellt wie in Beispiel 25 beschrieben. Allerdings wird an Stelle der Schicht, die unter pH 6 löslich, darüber aber unlöslich ist, eine Schicht aufgebracht, die wie die darauf folgende Schicht hergestellt wird, mit dem Unterschied, dass bei der Modifikation des Chitosans nur 30,8% Bernsteinsäureanhydrid verwendet wird, so dass es unterhalb von pH 6 und oberhalb von pH 7,1 löslich ist.

Es sind also zwischen dem wirkstoffhaltigen Kern und der enterischen Beschichtung zwei weitere Schichten enthalten, von denen die weiter innen gelegene zwischen pH 6 und pH 7,1 unlöslich ist, und die andere zwischen pH 5,2 und pH 6,3.

Die Freisetzung in Abhängigkeit vom pH Wert erfolgt ähnlich wie bei Beispiel 25, wobei allerdings die Freisetzungsgeschwindigkeit geringer ist. Zusätzlich erfolgt bei dieser Ausführungsform aber auch dann eine Freisetzung, wenn die Pellets in einen künstlichen Darmsaft mit pH 7,4 verbracht werden. Die Breite des Arbeitsbereichs konnte also nach oben hin vergrößert werden.

Erklärung zu den Zeichnungen:
In den Zeichnungen 1 bis 6 sind folgende Kennbuchstaben verwendet:
W für den Wirkstoffkern, beispielsweise wirkstoffhaltige oder wirkstoffbeschichtete Pellets, mit Wirkstoff gefüllte Kapseln,
wirkstoffhaltige Tabletten, Mirkotabletten etc.
P für Protective Coating (äußere Schicht)
E für Enteric Coating (innere Schicht)
C für Caecal Coating (innerste Schicht)
C1 für Caecal Coating (innerste Schicht), oder bei Ausführungsformen ohne innerste Schicht die am weitesten innen liegende weitere Schicht.
C2, C3, Cn für "Weitere Schichten"
C1a für Teilschicht 1 einer "weiteren Schicht", z.B. über einem bestimmten oberen pH Wert löslich
C1b für Teilschicht 2 einer "weiteren Schicht", z.B. unter einem bestimmten unteren pH Wert löslich
S für Sprengschichten oder Schichten, welche die Auflösung der darüber liegenden Schichten beschleunigen

Die Zeichnungen 14 bis 22 zeigen die Bereiche in denen die in den Beispielen verwendeten pH-abhängig löslichen oder quellbaren Schichten beständig sind. Die Nummerierung der Schichten erfolgt von außen, also in der Reihenfolge ihres Kontaktes mit der umgebenden wässrigen Lösung, wenn davon ausgegangen wird, dass sich im Laufe der Darmpassage alle Schichten lösen, bzw. permeabel werden.

Nicht wesentlich ph-abhängige Schichten, wie Quellschichten, Schichten mit Sprengmitteln und Schichten mit pH-Wert modulierenden Eigenschaften sind in diesen Diagrammen nicht aufgeführt und bei der Nummerierung der Schichten übersprungen.

Wurde im jeweiligen Beispiel eine unbeschichtete Kapsel zur Aufnahme beschichteter Pellets, Mikrotabletten, Matrixpresslinge etc. verwendet, so ist diese als Schicht 1 aufgeführt. Die Kapsel ist in keinem der im Gastrointestinaltrakt vorkommenden pH-Werte beständig, so dass in den Zeichnungen keine entsprechende Linie zu sehen ist. Allerdings ist sie in Speichelflüssigkeit so lange beständig, bis sie den Magen erreicht hat, so dass die als zweite Schicht bezeichnete Schicht nicht mit dem neutralen Speichel sondern erst mit dem sauren Magensaft in Kontakt kommt.

Ausgehend von links nach rechts ist aus den Zeichnungen ersichtlich, welchen Verlauf der pH-Wert der umgebenden Lösung nehmen muss, bzw. kann, damit die wässrige Lösung nacheinander die verschiedenen Schichten auflösen, bzw. durchdringen kann.

## Patentansprüche

1. Eine Formulierung zur kontrollierten Freisetzung eines oder mehrerer Wirkstoffe oder eines oder mehrerer wirkstoffhaltiger Kerne im Dickdarm eines Säugetieres, die dazu geeignet ist, den oder die Wirkstoffe oder den oder die wirkstoffhaltigen Kerne abhängig vom Verlauf des pH Wertes freizusetzen, umfassend eine Folge von Schichten (Schichtfolge), die den oder die Wirkstoffe oder den oder die wirkstoffhaltigen Kerne umhüllt, wobei die Schichtfolge eine Schicht umfasst, die in wässrigen Lösungen nur oberhalb eines bestimmten pH Wertes löslich oder permeabel ist, und, von der besagten Schicht umhüllt oder darunter angeordnet, eine Schicht umfasst, deren Löslichkeit oder Permeabilität in wässrigen Lösungen abhängig vom pH Wert ist,
**gekennzeichnet dadurch, dass** sie
eine
oder
mehrere effektiv parallel angeordnete
Schichtfolgen umfasst, die den oder die Wirkstoffe oder den oder die wirkstoffhaltigen Kerne umhüllen, wobei
jede Schichtfolge
eine "innere" Schicht umfasst,
die
in wässrigen Lösungen
nur oberhalb eines bestimmten letzten PH Wertes, der auch ihren bestimmten oberen pH Wert darstellt,
und der vorzugsweise zwischen 2 und 9 liegt, mehr bevorzugt zwischen 4 und 8,
löslich oder permeabel ist,
und mindestens eine Schichtfolge,
von der "inneren" Schicht umhüllt oder darunter angeordnet, eine oder mehrere einander umhüllende oder untereinander angeordnete "weitere" Schichten umfasst,
die jeweils
in wässrigen Lösungen
sowohl unterhalb eines individuellen bestimmten unteren PH Wertes,
der niedriger ist als der bestimmte obere pH Wert der sie umhüllenden bzw. über ihr angeordneten Schicht, vorzugsweise um 0,1 bis 2,5 pH Stufen niedriger,
als auch oberhalb eines individuellen bestimmten oberen PH Wertes,
der höher ist als ihr eigener bestimmter unterer pH Wert, vorzugsweise um 0,1 bis 2,5 pH Stufen höher,
und der höher ist als der bestimmte obere pH Wert der sie umhüllenden bzw. über ihr angeordneten Schicht, vorzugsweise um 0,1 bis 2,5 pH Stufen höher,
löslich oder permeabel sind,
jedoch nicht dazwischen,
und/oder
eine von der "inneren" Schicht oder, falls die Schichtfolge eine oder mehrere "weitere" Schichten umfasst, eine von der oder den "weiteren" Schichten umhüllte oder darunter angeordnete "innerste" Schicht umfasst,
die
in wässrigen Lösungen
nur unterhalb eines bestimmten ersten PH Wertes,
der vorzugsweise zwischen 2 und 9 liegt, mehr bevorzugt zwischen 3,5 und 8,
und der niedriger ist als der bestimmte obere pH Wert der sie umhüllenden bzw. über ihr angeordneten Schicht,
löslich oder permeabel ist,
und dass die Formulierung mindestens eine der oben als "weitere" Schichten definierte Schicht oder mindestens zwei effektiv parallel angeordnete Schichtfolgen umfasst,
wobei die effektiv parallele Anordnung dadurch sichergestellt wird, dass jeweils nicht die gesamte Oberfläche beschichtet wird, so dass die Oberfläche pro verwendeter Schichtfolge mindestens einen Bereich aufweist, an dem nur diese löslich oder permeabel werden muss, damit der Darminhalt in Kontakt mit dem oder den Wirkstoffen oder dem oder den wirkstoffhaltigen Kernen kommt, und der oder die Wirkstoffe freigesetzt werden können,
wobei Schichten Beschichtungen oder sonstige Umhüllungen darstellen.

2. Eine Formulierung nach Anspruch 1, **gekennzeichnet dadurch, dass**
eine Freisetzung im Wesentlichen nach einem Abfall des PH-Wertes innerhalb des Verdauungstraktes erfolgt,
und dass
der Wert, unter den der pH Wert zur Auslösung der pH-abhängigen Freisetzung fallen muss, und/oder ob eine Freisetzung auch ohne einen Abfall des pH Wertes ausgelöst wird, abhängig vom maximal erreichten pH Wert ist.

3. Eine Formulierung nach einem der vorhergehenden Ansprüche, **gekennzeichnet dadurch, dass**
sie mindestens zwei effektiv parallel angeordnete Schichtfolgen umfasst.

4. Eine Formulierung nach einem der vorhergehenden Ansprüche, **gekennzeichnet dadurch, dass**
jede Schichtfolge
eine von der "inneren" Schicht oder, falls die Schichtfolge eine oder mehrere "weitere" Schichten umfasst, eine von der oder den "weiteren" Schichten umhüllte oder darunter angeordnete "innerste" Schicht umfasst,
die
in wässrigen Lösungen
nur unterhalb eines bestimmten ersten PH Wertes,
der vorzugsweise zwischen 2 und 9 liegt, mehr bevorzugt zwischen 3,5 und 8,
und der niedriger ist als der bestimmte obere pH Wert der sie umhüllenden bzw. über ihr angeordneten Schicht,
löslich oder permeabel ist.

5. Eine Formulierung nach einem der vorhergehenden Ansprüche, **gekennzeichnet dadurch, dass** mindestens eine Schichtfolge,
von der "inneren" Schicht umhüllt oder darunter angeordnet, eine oder mehrere einander umhüllende oder untereinander angeordnete "weitere" Schichten umfasst,
die jeweils
in wässrigen Lösungen
sowohl unterhalb eines individuellen bestimmten unteren PH Wertes,
der niedriger ist als der bestimmte obere pH Wert der sie umhüllenden bzw. über ihr angeordneten Schicht, vorzugsweise um 0,1 bis 2,5 pH Stufen niedriger,
als auch oberhalb eines individuellen bestimmten oberen PH Wertes,
der höher ist als ihr eigener bestimmter unterer pH Wert, vorzugsweise um 0,1 bis 2,5 pH Stufen höher,
und der höher ist als der bestimmte obere pH Wert der sie umhüllenden Schicht, vorzugsweise um 0,1 bis 2,5 pH Stufen höher,
löslich oder permeabel sind,
jedoch nicht dazwischen.

6. Eine Formulierung nach Anspruch 5, **gekennzeichnet dadurch, dass**
ein oder mehrere Wirkstoffe
oder
ein oder mehrere den oder die Wirkstoffe umfassende Partikel, Tabletten, Pellets, Kapseln oder sonstige feste oder halbfeste Erzeugnisse
vorzugsweise
emulgiert, dispergiert, verpresst, beschichtet oder auf andere Art und Weise vor Kontakt mit dem umgebenden Medium geschützt sind
mit einem Material oder Materialgemisch,
das
in wässrigen Lösungen
mit einem PH Wert unterhalb eines bestimmten ersten PH Wertes
weitgehend oder komplett löslich oder permeabel ist,
und das
in wässrigen Lösungen
mit einem PH Wert in dem Bereich oberhalb dieses bestimmten ersten PH Wertes,
der sich mindestens bis zu einem bestimmten zweiten PH Wert erstreckt,
weitgehend oder komplett unlöslich oder nicht permeabel ist,
wobei dieser bestimmte zweite PH Wert vorzugsweise höher ist, als der maximal zu erwartende PH Wert innerhalb des Verdauungstraktes vor Erreichen der Ileozäkalklappe in der Gruppe von Individuen, für welche der freizusetzende Stoff, bzw. die freizusetzenden Stoffe bestimmt sind,
mehr bevorzugt mindestens so hoch, wie der maximal im Dünndarm zu erwartende pH Wert,
und dass der oder die vorzugsweise so emulgierten, dispergierten, verpressten, beschichteten oder auf andere Art und Weise vor Kontakt mit dem umgebenden Medium geschützten
Wirkstoffe
oder
den oder die Wirkstoffe umfassende Partikel, Tabletten, Pellets, Kapseln oder sonstige feste oder halbfeste Erzeugnisse
einmal oder mehrmals nacheinander emulgiert, dispergiert, verpresst, beschichtet oder auf andere Art und Weise vor Kontakt mit dem umgebenden Medium geschützt sind
mit einem
für jede dieser Beschichtungen, Dispergierungen, Emulgierungen, Verpressungen oder auf andere Art und Weise realisierten Schutzmaßnahmen vor Kontakt mit dem umgebenden Medium
individuellen
Material oder Materialgemisch,
das
sowohl in wässrigen Lösungen
mit einem PH Wert unterhalb eines individuellen bestimmten unteren PH Wertes
als auch in wässrigen Lösungen
mit einem PH Wert oberhalb eines individuellen bestimmten oberen PH Wertes
weitgehend oder komplett löslich oder permeabel ist,
und das
in wässrigen Lösungen
mit einem PH Wert in dem Bereich zwischen dem individuellen bestimmten unteren und dem individuellen bestimmten oberen PH Wert
weitgehend oder komplett unlöslich oder nicht permeabel ist,
und der oder die so emulgierten, dispergierten, verpressten, beschichteten oder auf andere Art und Weise vor Kontakt mit dem umgebenden Medium geschützten
Wirkstoffe
oder
den oder die Wirkstoffe umfassende Partikel, Tabletten, Pellets, Kapseln oder sonstige feste oder halbfeste Erzeugnisse
wiederum emulgiert, dispergiert, verpresst, beschichtet oder auf andere Art und Weise vor Kontakt mit dem umgebenden Medium geschützt sind mit einem Material oder Materialgemisch,
das
in wässrigen Lösungen
oberhalb eines bestimmten letzten PH Wertes
weitgehend oder komplett löslich oder permeabel ist,
und das
in wässrigen Lösungen
unterhalb dieses letzten PH Wertes, der auch ihren bestimmten oberen pH Wert darstellt,
weitgehend oder komplett unlöslich oder nicht permeabel ist,
wobei die Beschichtungen, Dispergierungen, Emulgierungen, Verpressungen oder auf andere Art und Weise realisierten Schutzmaßnahmen vor Kontakt mit dem umgebenden Medium nicht zwingend direkt nacheinander ausgeführt sein müssen.

7. Eine Formulierung nach einem der Ansprüche 5 oder 6, **gekennzeichnet dadurch, dass**
wenigstens eine "weitere Schicht",
durch Anwendung eines Trockenbefilmungsverfahrens realisiert ist.

8. Eine Formulierung nach einem der Ansprüche 5 bis 7, **gekennzeichnet dadurch, dass**
wenigstens eine "weitere Schicht"
zwei verschiedene Materialien umfasst,
von welchen mindestens eines
in wässrigen Lösungen
mit einem pH Wert unterhalb eines bestimmten Bereiches
weitgehend oder komplett löslich oder permeabel ist,
und mindestens ein anderes
in wässrigen Lösungen
mit einem pH Wert oberhalb jenes bestimmten Bereiches
weitgehend oder komplett löslich oder permeabel ist,
wobei beide Materialien
in wässrigen Lösungen
mit einem pH Wert innerhalb jenes bestimmten Bereiches
weitgehend oder komplett unlöslich oder nicht permeabel sind,
und dass die verschiedenen Materialien vorzugsweise effektiv parallel angeordnet sind.

9. Eine Formulierung nach einem der Ansprüche 5 bis 8, **gekennzeichnet dadurch, dass**
wenigstens eine "weitere Schicht"
ein Polymer umfasst, welches sowohl kationische als auch anionische Eigenschaften aufweist.

10. Eine Formulierung nach einem der Ansprüche 5 bis 9, **gekennzeichnet dadurch, dass**
wenigstens eine "weitere Schicht"
ein Material umfasst,
welches
sowohl in wässrigen Lösungen mit einem pH Wert unterhalb eines bestimmten Bereiches,
als auch in wässrigen Lösungen mit einem pH Wert oberhalb jenes bestimmten Bereiches,
weitgehend oder komplett löslich oder permeabel ist,
aber nicht innerhalb jenes bestimmten Bereiches.

11. Eine Formulierung nach einem der Ansprüche 5 bis 10, **gekennzeichnet dadurch, dass**
wenigstens eine "weitere Schicht"
ein Chitosan umfasst,
welches Bernsteinsäuregruppen enthält,
wobei die Bernsteinsäuregruppen bevorzugt durch Modifikation des Chitosans mit Bernsteinsäureanhydrid eingefügt wurden.

12. Eine Formulierung nach einem der Ansprüche 5 bis 11, **gekennzeichnet dadurch, dass**
wenigstens eine "weitere Schicht"
ein Chitosan umfasst,
welches Chlorogensäuregruppen enthält,
wobei die Chlorogensäuregruppen bevorzugt durch Modifikation des Chitosans mit Chlorogensäure in Anwesenheit eines enzymatischen Katalysators eingefügt wurden.

13. Eine Formulierung nach einem der vorhergehenden Ansprüche, **gekennzeichnet dadurch, dass** sie eine "äußere" Beschichtung umfasst, die im Magen löslich ist
und dass, falls die Formulierung wenigstens eine "weitere" Schicht umfasst,
der bestimmte obere pH Wert der am weitesten innen liegenden "weiteren" Schicht einer Schichtenfolge
vorzugsweise niedriger ist, als der maximal im Dünndarm zu erwartende pH Wert,
und zwar mehr bevorzugt um 0,1 bis 1,2 pH Stufen, noch mehr bevorzugt um 0,4 bis 0,8 pH Stufen, besonders bevorzugt um 0,5 bis 0,65 pH Stufen, und speziell bevorzugt um 0,55 pH Stufen.

14. Eine Formulierung nach einem der vorhergehenden Ansprüche, **gekennzeichnet dadurch, dass** die Auflösung oder das permeabel werden einzelner oder mehrerer verwendeter Materialien nicht darauf angewiesen ist, dass der oder die wirkstoffhaltige Kerne Säuren oder Säure freisetzende Stoffe enthält.

15. Eine Formulierung nach einem der vorhergehenden Ansprüche, **gekennzeichnet dadurch, dass** eine Schicht eine von ihr umhüllte Schicht oder den oder die von ihr umhüllten Wirkstoffe oder wirkstoffhaltigen Kerne so lange vor der umgebenden Lösung schützt, wie es im Verlauf der Darmpassage notwendig ist.

16. Ein Verfahren zur Herstellung einer Formulierung zur kontrollierten Freisetzung nach Anspruch 1, **gekennzeichnet dadurch, dass** ein oder mehrere Wirkstoffe, die vorzugsweise vermischt, verpresst oder anderweitig mit oder ohne weitere Zusatzstoffen verarbeitet sind,
vorzugsweise
beschichtet, verpresst, granuliert oder auf andere Art und Weise umhüllt werden mit einem Material oder Materialgemisch, das in wässrigen Lösungen
nur bei einem PH Wert unterhalb eines bestimmten ersten PH Wertes
weitgehend oder komplett löslich oder permeabel ist,
und dass der oder die vorzugsweise so umhüllten Wirkstoffe einmal oder mehrmals nacheinander beschichtet, verpresst, granuliert oder auf andere Art und Weise umhüllt werden mit einem für jeden dieser Verfahrensschritte individuellen Material oder Materialgemisch,
das jeweils
in wässrigen Lösungen
sowohl bei einem PH Wert unterhalb eines individuellen bestimmten unteren PH Wertes
als auch bei einem PH Wert oberhalb eines individuellen bestimmten oberen PH Wertes
weitgehend oder komplett löslich oder permeabel ist,
jedoch nicht dazwischen,
und der oder die so umhüllten Wirkstoffe wiederum beschichtet, verpresst, granuliert oder auf andere Art und Weise umhüllt werden mit einem Material oder Materialgemisch,
das
in wässrigen Lösungen
nur bei einem PH Wert oberhalb eines bestimmten letzten PH Wertes
löslich oder permeabel ist,
wobei diese Beschichtungs-, Verpressungs-, Umpressungs, Granulierungs- oder auf andere Art und Weise ausgeführten Umhüllungsschritte nicht zwingend direkt nacheinander erfolgen müssen.

## Claims

1. A formulation for the controlled release of one or more active ingredients or one or more active ingredient containing cores in the colon of a mammal, which is adapted to release the one or more active ingredients or the one or more active ingredient containing cores depending on the course of the pH value, comprising a sequence of layers (layer sequence), which envelops the one or more active ingredients or the one or more active ingredient containing cores, wherein said layer sequence comprises a layer, which is soluble or permeable in aqueous solutions only above a defined pH value and, enveloped by said layer or arranged below it, comprises a layer, the solubility or permeability of which in aqueous solutions is dependent on the pH value,
**characterized in that** it comprises
one
or
more than one effectively parallel arranged
layer sequences, which envelop the one or more active ingredients or the one or more active ingredient containing cores, wherein
each layer sequence
comprises an "inner" layer,
which
is soluble or permeable in aqueous solutions
only above a defined last pH value, which also constitutes its defined upper pH value,
which preferably lies between 2 and 9, more preferably between 4 and 8,
and at least one layer sequence
comprises, enveloped by or arranged below said "inner" layer, one or more "further" layers, which envelop or are arranged below each other,
which each
are soluble or permeable in aqueous solutions
both below an individual defined lower pH value,
which is lower than the defined upper pH value of the layer enveloping it or being arranged above it, preferably lower by 0.1 to 2.5 pH levels,
and above an individual defined upper pH value,
which is higher than its own defined lower pH value, preferably by 0.1 to 2.5 pH levels,
and which is higher than the defined upper pH value of the layer enveloping it or being arranged above it, preferably lower by 0.1 to 2.5 pH levels,
but not in between,
and/or
comprises, enveloped by or arranged below said "inner" layer or, in case the layer sequence comprises one or more "further" layers, enveloped by or arranged below said one or more "further" layers, a "most inner" layer,
which
is soluble or permeable in aqueous solutions
only below a defined first pH value,
which preferably lies between 2 and 9, more preferably between 3.8 and 8,
and which is lower than the defined upper pH value of the layer enveloping it or being arranged above it,
wherein the formulation comprises at least one of the layers defined above as "further" layers, or at least two effectively parallel arranged layer sequences,
wherein the effective parallel arrangement is ensured by the fact that in each case not the whole surface is covered so that the surface has at least one area per layer sequence, at which only that one has to become soluble or permeable in order for the intestinal content coming into contact with the one or more active ingredients or the one or more active ingredient containing cores and the one or more active ingredients can be released,
wherein layers represent coatings or other types of envelopments.

2. A formulation according to claim 1, **characterized in that**
a release occurs substantially after a decrease of the pH value within the digestive tract,
and that
the value, below which the pH value has to fall, in order that the pH dependent release is triggered, and/or if a release is triggered without a decrease of the pH value, is dependent on the maximally reached pH value.

3. A formulation according to one of the preceding claims, **characterized in that** it comprises at least two effectively parallel arranged layer sequences.

4. A formulation according to one of the preceding claims, **characterized in that** every layer sequence
comprises, enveloped by or arranged below said "inner" layer or, in case the layer sequence comprises one or more "further" layers, enveloped by or arranged below said one or more "further" layers, a "most inner" layer,
which
is soluble or permeable in aqueous solutions
only below a defined first pH value,
which preferably lies between 2 and 9, more preferably between 3.8 and 8,
and which is lower than the defined upper pH value of the layer enveloping it or being arranged above it.

5. A formulation according to one of the preceding claims, **characterized in that** at least one layer sequence
comprises, enveloped by or arranged below said "inner" layer, one or more "further" layers, which envelop or are arranged below each other,
which each
are soluble or permeable in aqueous solutions
both below an individual defined lower pH value,
which is lower than the defined upper pH value of the layer enveloping it or being arranged above it, preferably lower by 0.1 to 2.5 pH levels,
and above an individual defined upper pH value,
which is higher than its own defined lower pH value, preferably by 0.1 to 2.5 pH levels,
and which is higher than the defined upper pH value of the layer enveloping it or being arranged above it, preferably lower by 0.1 to 2.5 pH levels,
but not in between.

6. A formulation according to claim 5, **characterized in that**
one or more active ingredients
or
one or more particles, tablet, pellets, capsules or other solid or half-solid products comprising the one or more active ingredients
preferably
are emulsified, dispersed, compressed, coated or protected in another way against contact with the surrounding medium
with a material or material mixture,
which
is substantially or completely dissolvable or permeable
in aqueous solutions
with a pH value below a defined first pH value,
and which
is substantially or completely indissoluble or not permeable
in aqueous solutions
with a pH value in the range above this defined first pH value
which extends at least up to a defined second pH value
and this defined second pH value is preferably higher than the pH values maximally to be expected within the digestive tract before reaching of the ileocecal valve in the group of individuals for which the substance to be released or the substances to be released are determined,
more preferred at least as high as the pH value maximally to be expected in the small intestine,
and that the one or more preferably in such a way emulsified, dispersed, compressed, coated or in another way against contact with the surrounding medium protected
active ingredients
or
particles, tablet, pellets, capsules or other solid or half-solid products comprising the one or more active ingredients
are emulsified, dispersed, compressed, coated or protected in another way against contact with the surrounding medium once or several times successively
with a
material or material mixture
individual
for each of these coatings, dispersifications, emulsifications, compressions or in another way realized preventive measures against contact with the surrounding medium,
which in each case
is substantially or completely dissolvable or permeable
both in aqueous solutions
with a pH value below an individual defined lower pH value
and in aqueous solutions
with a pH value above an individual defined upper pH value,
and which
is substantially or completely indissoluble or not permeable
in aqueous solutions
with a pH value in the range between the individual defined lower pH value and the individual defined upper one,
and the one or more in such a way emulsified, dispersed, compressed, coated or in another way against contact with the surrounding medium protected
active ingredients
or
particles, tablet, pellets, capsules or other solid or half-solid products comprising the one or more active ingredients
are once again emulsified, dispersed, compressed, coated or protected in another way against contact with the surrounding medium with a material or material mixture
which
is substantially or completely dissolvable or permeable
in aqueous solutions
above a defined last pH value,
and which
is substantially or completely indissoluble or not permeable
in aqueous solutions
below this last pH value, which also constitutes its defined upper pH value,
wherein said coatings, dispersifications, emulsifications, compressions or in another way realized preventive measures against contact with the surrounding medium do not necessarily have to be carried out directly successively.

7. A formulation according to one of the claims 5 or 6, **characterized in that** at least one "further" layer is realized by using a dry coating process.

8. A formulation according to one of the claims 5 to 7, **characterized in that**
at least one "further" layer
comprises two different materials,
of which at least one
is substantially or completely dissolvable or permeable
in aqueous solutions
with a pH value below a defined range
and at least another one
is substantially or completely dissolvable or permeable
in aqueous solutions
with a pH value above said defined range,
wherein both materials
are substantially or completely indissoluble or not permeable in aqueous solutions
with a pH value within said defined range
and that the different materials preferably are arranged effectively in parallel.

9. A formulation according to one of the claims 5 to 8, **characterized in that**
at least one "further" layer
comprises a polymer that has both, cationic and anionic characteristics.

10. A formulation according to one of the claims 5 to 9, **characterized in that**
at least one "further" layer
comprises a polymer
which
is substantially or completely dissolvable or permeable
both in aqueous solutions with a pH value below a defined range
and in aqueous solutions with a pH value above a defined range
but not in within said defined range.

11. A formulation according to one of the claims 5 to 10, **characterized in that**
at least one "further" layer
comprises a Chitosan
which comprises succinic acid groups
wherein the succinic acid groups preferably have been inserted by modification of the Chitosan with succinic acid anhydride.

12. A formulation according to one of the claims 5 to 11, **characterized in that**
at least one "further" layer
comprises a Chitosan
which comprises chlorogenic acid groups
wherein the chlorogenic acid groups preferably have been inserted by modification of the Chitosan with chlorogenic acid in presence of an enzymatic catalyst.

13. A formulation according to one of the preceding claims, **characterized in that** it comprises an "outer" coating that is dissolvable in the stomach and
that, in case the formulation comprises at least one "further" layer,
the defined upper pH value of the most inner lying "further" layer of a layer sequence
preferably is lower than the pH value maximally to be expected in the small intestine,
in fact more preferable by 0.1 to 1.2 pH units, even more preferable by 0.4 to 0,8 pH units, particularly preferable by 0.5 to 0.65 pH units, and especially preferable by 0,55 pH units.

14. A formulation according to one of the preceding claims, **characterized in that** the dissolution of individual or several used materials or them becoming permeable is not reliant on the active-ingredient containing core containing acids or acid releasing substances.

15. A formulation according to one of the preceding claims, **characterized in that** a layer protects a layer enveloped by it or the one or more active ingredients enveloped by it against the surrounding solution as long as it is necessary in the course of the bowel passage

16. A process for the preparation of a formulation for the controlled release according to claim 1, **characterized in that**
one or more active ingredients, which preferably are compounded, compressed or processed in another way with or without additional excipients,
preferably are
coated, compressed, granulated or enveloped in another way with a material or material mixture
which
is substantially or completely dissolvable or permeable
in aqueous solutions
only with a pH value below a defined first pH value,
and that the one or more preferably in such a way coated active ingredients are coated, compressed, granulated or enveloped in another way once or several times successively with a material or material mixture individual for each of these process steps
which in each case
is substantially or completely dissolvable or permeable
in aqueous solutions
both with a pH value below an individual defined lower pH value
and with a pH value above individual defined upper pH value,
but not in between,
and the one or more in such a way enveloped active ingredients are once again coated, compressed, granulated or enveloped in another way with material or material mixture
which
is dissolvable or permeable
in aqueous solutions
only with a pH value above a defined last pH value,
wherein said coating-, compression-, mold-coating-, granulation- or in another way executed enveloping-steps do not necessarily have to be carried out directly successively.

## Revendications

1. Formulation pour la libération contrôlée d'une ou de plusieurs substances actives ou d'un ou plusieurs noyaux contenant des substances actives dans le gros intestin d'un mammifère et cette formulation est capable de libérer une ou plusieurs substances actives ou des noyaux contenant des substances actives en fonction de la variation de la valeur du pH, comprenant une séquence de couches (séquence de couches) ; qui enveloppe la ou les substances actives ou le ou les noyaux contenant la substance active, y comprenant que la séquence de couches comprend une couche qui dans des solutions aqueuses est seulement soluble ou perméable au-dessus d'une certaine valeur de pH, et, enveloppée par cette couche ou disposée en dessous de celle-ci, comprend une couche dont la solubilité ou perméabilité en solutions aqueuses dépend de la valeur du pH,
qui se **caractérise par le fait qu'**
une
ou
plusieurs couches disposées efficacement en parallèle
y sont comprises et qui enveloppent une ou plusieurs substances actives ou plusieurs noyaux contenant des substances actives, à condition que
chaque séquence de couches
comprend une couche « interne »,
qui
dans des solutions aqueuses
seulement au-dessus d'une certaine dernière valeur de pH, qui représente également sa valeur de pH supérieure particulière,
qui de préférence entre 2 et 9, plus préféré entre 4 et 8,
est soluble ou perméable,
et qui enveloppe au minimum une séquence de couches
de la couche « intérieure » ou que comprend une ou plusieurs couches « supplémentaires » enveloppées ou disposées entre elles,
qui sont chacune
dans des solutions aqueuses
à la fois en dessous d'une valeur spécifique individuelle de pH inférieure,
qui est inférieur à la valeur de pH supérieure spécifiée de la couche enveloppant ou placée au-dessus de celle-ci, de préférence de 0,1 à 2,5 niveaux de pH inférieurs,
et à la fois au-dessous d'une valeur spécifique individuelle de pH supérieure,
qui est supérieur à son pH inférieur spécifique, de préférence de 0,1 à 2,5 niveaux de pH supérieurs,
et qui est supérieur à la valeur de pH supérieure spécifiée de la couche enveloppant ou placée au-dessus de celle-ci, de préférence de 0,1 à 2,5 niveaux de pH inférieurs,
soient solubles ou perméables,
mais pas entre les deux,
et/ou
comprend une couche « interne », si la séquence de couches comprend un ou plusieurs couches « supplémentaires », une couche enveloppé par une ou plusieurs des couches « supplémentaires » ou une couche ordonné « interne »,
qui
dans des solutions aqueuses
seulement en dessous d'une certaine première valeur de pH,
qui est de préférence entre 2 et 9, plus préféré entre 3,5 et 8,
inférieur à la valeur de pH supérieure spécifiée de la couche enveloppant ou placée au-dessus de celle-ci,
est soluble ou perméable,
et que la formulation comprend au moins une des couches définies comme « couches supplémentaires », ou au moins deux couches effectivement disposées en parallèle,
selon lequel la disposition parallèle efficace est assurer par qu'en ne recouvrant pas toute la surface respectivement, de sorte que la surface ait au moins une surface pour chaque séquence de couches utilisées où seule elle doit être soluble ou perméable,
afin que le contenu intestinal entre en contact avec une ou plusieurs substances actives ou plusieurs noyaux contenant des substances actives et que la ou les substances actives puissent être libérées
les couches représentent des revêtements ou d'autres revêtements.

2. Formulation selon la revendication 1, **caractérisé par le fait que**
une libération survient essentiellement après une diminution du pH dans le tube digestif,
et que
la valeur au-dessous de laquelle la valeur du pH pour déclencher la libération dépendante du pH doit tomber, et/ou si une libération est déclenché même sans baisse de la valeur du pH dépend de la valeur maximale du pH obtenue.

3. Une formulation selon l'une des revendications précédentes, **caractérisée par** qu'elle comprend au moins deux couches effectivement disposées en parallèle.

4. Une formulation selon l'une des revendications précédentes, **caractérisée par** chaque séquence de couches
comprend une couche « interne », ou si la séquence de couches comprend un ou plusieurs couches « supplémentaires », une couche enveloppé par une ou plusieurs des couches « supplémentaires » ou une couche ordonné « interne »,
qui
dans des solutions aqueuses
seulement en dessous d'une certaine première valeur de pH,
qui est de préférence entre 2 et 9, plus préféré entre 3,5 et 8,
inférieur à la valeur de pH supérieure spécifiée de la couche enveloppant ou placée au-dessus de celle-ci,
est soluble ou perméable,

5. Une formulation selon l'une des revendications précédentes, **caractérisée par**
au minimum une séquence de couches,
enveloppé par la couche « intérieure » ou la couche y ordonné que comprend une ou plusieurs couches « supplémentaires » enveloppées ou disposées entre elles,
qui sont chacune
dans des solutions aqueuses
à la fois en dessous d'une valeur spécifique individuelle de pH inférieure,
qui est inférieur à la valeur de pH supérieure spécifiée de la couche enveloppant ou placée au-dessus de celle-ci, de préférence de 0,1 à 2,5 niveaux de pH inférieurs,
et à la fois au-dessous d'une valeur spécifique individuelle de pH supérieure,
qui est supérieur à son pH inférieur spécifique, de préférence de 0,1 à 2,5 niveaux de pH supérieurs,
et qui est supérieur à la valeur de pH supérieure spécifiée de la couche enveloppant, de préférence de 0,1 à 2,5 niveaux de pH supérieurs,
soient solubles ou perméables,
mais pas entre les deux.

6. Formulation selon la revendication 5, **caractérisé par le fait que** une ou plusieurs substances actives
ou
une ou plusieurs particules, comprimés, pastilles, gélules ou autres produits solides ou semi-solides contenant la ou les substances actives
de manière préférentielle
émulsionnent, dispersent, pressent, enduisent ou autrement protègent du contact avec le milieu environnant
avec un matériau ou un mélange de matériaux,
qui
dans des solutions aqueuses
avec une valeur de PH inférieure à une certaine première valeur de PH
est largement ou complètement soluble ou perméable,
et que
dans des solutions aqueuses
avec une valeur de pH comprise dans le domaine supérieure à cette première valeur de pH particulière,
qui s'étend jusqu'à au moins une certaine seconde valeur de pH,
est largement ou complètement insoluble ou non perméable,
lorsque cette seconde valeur particulière de pH est de préférence supérieure à la valeur maximale prévue de pH dans le tube digestif avant d'atteindre la valvule iléo-cæcale du groupe de personnes pour lesquelles la ou les substances à libérer est destinée ;
plus préféré au moins aussi élevé que le pH maximal attendu dans l'intestin grêle,
et que les substances actives soient de préférence émulsionnées, dispersées, pressées, enduites ou autrement protégées du contact avec le milieu environnant
ou
une ou plusieurs particules, comprimés, pastilles, gélules ou autres produits solides ou semi-solides contenant la ou les substances actives
émulsionnent, dispersent, pressent, enduisent une ou plusieurs fois ou sont autrement protéger du contact avec le milieu environnant
avec un
pour chacun de ces revêtements, dispersions, émulsions, compressions ou mesures de protection contre le contact avec le milieu environnant
individuel
matériau ou mélange de matériaux,
que
dans des solutions aqueuses
avec une valeur de pH en dessous d'une valeur spécifique individuelle de pH inférieure,
ainsi que dans les solutions aqueuses
avec une valeur de pH au-dessous d'une valeur spécifique individuelle de pH inférieure,
est largement ou complètement soluble ou perméable,
et que
dans des solutions aqueuses
avec une valeur de pH comprise entre la valeur de pH spécifiée inférieur et la valeur de pH supérieure spécifiée individuellement
est largement ou complètement insoluble ou non perméable,
et que les substances actives soient de préférence émulsionnées, dispersées, pressées, enduites ou autrement protégées du contact avec le milieu environnant
ou
une ou plusieurs particules, comprimés, pastilles, gélules ou autres produits solides ou semi-solides contenant la ou les substances actives
à leur tour sont émulsionnés, dispersés, pressés, enduits ou autrement protégés du contact avec le milieu environnant avec un matériau ou un mélange de matériaux
que
dans des solutions aqueuses
au-dessus d'une certaine dernière valeur de pH
est largement ou complètement soluble ou perméable,
et que
dans des solutions aqueuses
en dessous de cette dernière valeur de pH, qui représente également sa valeur de pH supérieure particulière,
est largement ou complètement insoluble ou non perméable,
lorsque les revêtements, dispersions, émulsions, compressions ou autres mesures de protection mises en œuvre contre le contact avec le milieu environnant ne doivent pas nécessairement être effectués successivement.

7. Une formulation selon l'une des revendications 5 ou 6, **caractérisée par** au moins une couche « supplémentaire »
est réalisé par l'application d'un processus de tournage à sec.

8. Une formulation selon les revendications 5 à 7, **caractérisée par**
au moins une couche « supplémentaire »
qui comprend deux matériaux différents,
dont au moins un
dans des solutions aqueuses
avec une valeur de pH inférieure à un certain niveau
est largement ou complètement soluble ou perméable,
et au moins un autre
dans des solutions aqueuses
avec une valeur de pH supérieure au niveau mentionné
est largement ou complètement soluble ou perméable,
où les deux matériaux
dans des solutions aqueuses
avec une valeur de pH dans le niveau mentionné
est largement ou complètement insoluble ou non perméable,
et que les différents matériaux sont de préférence disposés de manière efficace en parallèle.

9. Une formulation selon les revendications 5 à 8, **caractérisée par**
au moins une couche « supplémentaire »
comprend un polymère qui a des propriétés cationiques et anioniques.

10. Une formulation selon les revendications 5 à 9, **caractérisée par**
au moins une couche « supplémentaire »
comprend un matériau ;
lequel
à la fois dans des solutions aqueuses avec une valeur de pH inférieure à un certain niveau
ainsi que dans des solutions aqueuses avec une valeur de pH supérieur à un certain niveau
est largement ou complètement soluble ou perméable,
mais pas dans le niveau mentionné.

11. Une formulation selon les revendications 5 à 10, **caractérisée par**
au moins une couche « supplémentaire »
comprend un chitosane,
qui contient des groupes d'acide succinique
où des groupes d'acide succinique ont été insérés de préférence en modifiant le chitosane avec de l'anhydride succinique.

12. Une formulation selon les revendications 5 à 11, **caractérisée par**
au moins une couche « supplémentaire »
comprend un chitosane,
qui contient des groupes d'acides chlorogéniques,
où les groupes d'acides chlorogéniques ont été insérés de préférence en modifiant le chitosane par de l'acide chlorogénique en présence d'un catalyseur enzymatique.

13. Une formulation selon l'une des revendications précédentes, **caractérisée par le fait qu'**elle comprend un revêtement « externe » soluble dans l'estomac
et que, si la formulation comporte au moins une couche « supplémentaire »,
la valeur de pH supérieure spécifiée de la couche « supplémentaire » la plus intérieure d'une séquence de couches
est de préférence inférieure au pH maximal attendu dans l'intestin grêle,
plus préféré par des niveaux de pH de 0,1 à 1,2, encore plus préféré par des niveaux de pH de 0,4 à 0,8, surtout préféré par des niveaux de pH de 0,5 à 0,65, et surtout par des niveaux de pH de 0,55.

14. Une formulation selon l'une des revendications précédentes, **caractérisée par le fait que** la dissolution ou la perméabilité d'un ou de plusieurs matériaux utilisés ne dépend pas du ou des noyaux contenant des acides ou des substances libératrices d'acide.

15. Une formulation selon l'une des revendications précédentes, **caractérisée par le fait qu'**une couche protège une couche couverte par elle ou les substances actives ou noyaux contenant des substances actives qu'elle couvre de la solution environnante aussi longtemps que nécessaire au cours du transit intestinal.

16. Un processus de préparation d'une formulation à libération contrôlée selon la revendication 1, **caractérisé par le fait qu'**une ou plusieurs substances actives de préférence mélangées, pressées ou autrement traitées avec ou sans additifs supplémentaires,
de manière préférentielle
peuvent être enduits, pressés, granulés ou revêtus d'un matériau ou d'un mélange de matériaux utilisés dans des solutions aqueuses
avec une valeur de pH inférieure à une certaine première valeur de pH
est largement ou complètement soluble ou perméable,
et que la ou les substances actives de préférence enveloppées de cette manière soient revêtues, pressées, granulées ou autrement revêtues une ou plusieurs fois successivement d'un matériau ou d'un mélange de matériaux qui est individuel pour chacune de ces étapes,
qui respectivement
dans des solutions aqueuses
avec une valeur de pH en dessous d'une valeur spécifique individuelle de pH inférieure,
ainsi qu'avec une valeur de pH au-dessous d'une valeur spécifique individuelle de pH supérieure
est largement ou complètement soluble ou perméable,
mais pas entre les deux,
et que la ou les substances actives ainsi enveloppées soient revêtues, pressées, granulées ou autrement revêtues d'un matériau ou d'un mélange de matières
que
dans des solutions aqueuses
seulement pour une valeur de pH supérieure à une certaine dernière valeur de pH
est soluble ou perméable,
où ces étapes de revêtement, de pressage, de compression, de granulation ou d'autres étapes d'emballage ne doivent pas nécessairement être effectuées successivement.
